# EUROPEAN PATENT SPECIFICATION

(11) **EP 0 797 662 B1**
(45) Date of publication and mention of the grant of the patent: **13.02.2008**
(21) Application number: 95914759.6
(22) Date of filing: 13.03.1995
(51) Int. Cl.: C12N 15/12, C07K 14/71, C07K 14/475, C07K 14/485, C07K 14/47, A61K 38/17

(54) **METHODS FOR TREATMENT OR DIAGNOSIS OF DISEASES OR CONDITIONS ASSOCIATED WITH ABNORMAL SIGNAL TRANSDUCTION**
METHODEN ZUR BEHANDLUNG ODER DIAGNOSE VON KRANKHEITEN ODER ZUSTÄNDEN, DIE MIT EINER ABNORMALEN SIGNALÜBERTRAGUNG ASSOZIIERT SIND
METHODES DE TRAITEMENT OU DE DIAGNOSTIC DE MALADIES OU D'ETATS ASSOCIES A UNE TRANSDUCTION ANORMALE DES SIGNAUX

(30) Priority: 14.03.1994 US 212234; 08.06.1994 US 255785
(43) Date of publication of application: 01.10.1997
(73) Proprietor: NEW YORK UNIVERSITY, New York, NY 10012 (US)
(72) Inventor: MARGOLIS, Ben, Lewis, New York, NY 10016 (US); SCHLESSINGER, Joseph, New York, NY 10011 (US); LAX, Irit, Fair Lawn, NJ 07410 (US); LADBURY, John, E., New York, NY 10003 (US); LEMMON, Mark, A., New York, NY 10010 (US)
(74) Representative: Viering, Hans-Martin
(86) International application number: PCT/US1995/003452
(87) International publication number: WO 1995/025166

(56) References cited:
- WO-A-92/03476
- WO-A-93/22339
- PROC. NATL. ACAD. SCI. U. S. A. (1992), 89(19), 8894-8 CODEN: PNASA6;ISSN: 0027-8424, 1992 MARGOLIS, B. ET AL 'High-efficiency expression/cloning of epidermal growth factor-receptor-binding proteins with Src homology 2 domains' cited in the application
- JOURNAL OF CELLULAR BIOCHEMISTRY SUPPLEMENT 0 (18B). 1994. 241. ISSN: 0733-1959, MARGOLIS B ET AL 'The SH2 domain protein, GRB -7, is amplified, overexpressed, and in a tight complex with HER2 in breast cancer.'
- TRENDS IN BIOCHEMICAL SCIENCES, ELSEVIER, AMSTERDAM, NL, cited in the application
- EMBO (EUROPEAN MOLECULAR BIOLOGY ORGANIZATION) JOURNAL 13 (6). 1994. 1331-1340. ISSN: 0261-4189, STEIN D ET AL 'The SH2 domain protein GRB -7 is co-amplified, overexpressed and in a tight complex with HER2 in breast cancer.'

## Description

### Field of the Invention

The present invention relates generally to the field of celluar signal transduction and more specifically to the diagnosis and treatment of various diseases and conditions associated with abnormal cell proliferation.

### Background of the Invention

The following is a discussion of relevant art, none of which is admitted to be prior art to the invention.

Cellular signal transduction is a fundamental mechanism whereby external stimuli that regulate diverse cellular processes are relayed to the interior of cells. One of the key biochemical mechanisms of signal transduction involves the reversible phosphorylation of tyrosine residues on proteins. The phosphorylation state of a protein is modified through the reciprocal actions of tyrosine kinases (TKs) and tyrosine phosphatases (TPs).

Receptor tyrosine kinases belong to a family of transmembrane proteins and have been implicated in cellular signaling pathways. The predominant biological activity of some receptor tyrosine kinases is the stimulation of cell growth and proliferation, while other receptor tyrosine kinases are involved in arresting growth and promoting differentiation. In some instances, a single tyrosine kinase can inhibit, or stimulate, cell proliferation depending on the cellular environment in which it is expressed. (Schlessinger, J. and Ullrich, A., Neuron, 9(3):383-391, 1992.)

Receptor tyrosine kinases are composed of at least three domains: an extracellular ligand binding domain, a transmembrane domain and a cytoplasmic catalytic domain that can phosphorylate tyrosine residues. Ligand binding to membrane-bound receptors induces the formation of receptor dimers and allosteric changes that activate the intracellular kinase domains and result in the self-phosphorylation (autophosphorylation and/or transphosphorylation) of the receptor on tyrosine residues. Individual phosphotyrosine residues of the cytoplasmic domains of receptors may serve as specific binding sites that interact with a host of cytoplasmic signalling molecules, thereby activating various signal transduction pathways (Ullrich, A. and Schlessinger, J., 1990, Cell 61:203-212).

The intracellular, cytoplasmic, non-receptor protein tyrosine kinases do not contain a hydrophobic transmembrane domain and share non-catalytic domains in addition to sharing their catalytic kinase domains. Such non-catalytic domains include the SH2 domains (SRC homology domain 2; Sadowski, I. et al., Mol. Cell. Biol. 6:4396-4408; Koch, C.A. et al., 1991, Science 252:668-674) and SH3 domains (SRC homology domain 3; Mayer, B.J. et al., 1988, Nature 332:269-272). Such non-catalytic domaims are also thought to include the PH domain (Musacchio et al., 1993, TIBS 18:342-348). The noncatalytic domains are thought to be important in the regulation of protein-protein interactions during signal transduction (Pawson, T. and Gish, G., 1992, Cell 71:359-362).

A central feature of signal transduction (for reviews, see Posada, J. and Cooper, J.A., 1992, Mol. Biol. Cell 3:583-392; Hardie, D.G., 1990, Symp. Soc. Exp. Biol. 44:241-255), is the reversible phosphorylation of certain proteins. Receptor phosphorylation stimulates a physical association of the activated receptor with target molecules. Some of the target molecules are in turn phosphorylated. Such phosphorylation transmits a signal to the cytoplasm. Other target molecules are not phosphorylated, but assist in signal transmission by acting as adapter molecules for secondary signal transducer proteins. For example, receptor phosphorylation and the subsequent allosteric changes in the receptor recruit the *Grb*-2/SOS complex to the catalytic domain of the receptor where its proximity to the membrane allows it to activate *ras* Pawson, T. and Schlessinger, J., Current Biol. 13:434, 1993.

The secondary signal transducer molecules generated by activated receptors result in a signal cascade that regulates cell functions such as cell division or differentiation. Reviews describing intracellular signal transduction include Aaronson, S.A., Science, 254:1146-1153, 1991; Schlessinger, J. Trends Biochem. Sci., 13:443-447, 1988; and Ullrich, A., and Schlessinger, J., Cell, 61:203-212, 1990.

Dimerization of cell-surface receptors upon binding of their cognate ligand appears to be a primary event in transmembrane signaling (Ullrich and Schlessinger, Cell 61:203-212, 1990; Canals, Biochemistry 31:4493-4501, 1992). Studies indicate that dimerization is sufficient for receptor activation (Schreiber et al., Proc. Natl. Acad. Sci. USA 78:7535-7539, 1981; Sorokin et al., J. Biol. Chem. 269:9752-9759, 1994; Watowich et al., Proc. Natl. Acad. Sci. USA 89:2140-2144, 1992), and propose that ligand-induced receptor dimerization constitutes a general mechanism for transmembrane signaling by these receptors (Schlessinger and Ullrich, Neuron 9:383-391, 1992).

Ligand-induced receptor dimerization is thought to occur via two possible mechanisms, or a combination of them. In the first, binding of the ligand to its cognate receptor may be bivalent, as is seen in the case of dimeric ligands such as PDGF, CSF1 and SCF. Binding of each protomer of the ligand dimer to a separate receptor molecule results in cross-linking and subsequent activation of the receptor (Heldin et al., J. Biol. Chem. 264:8905-8912, 1989; Lev et al., J. Biol. Chem. 267:15970-15977, 1992). Human growth hormone (hGH) also binds bivalently to its receptor, despite being monomeric in solution. A number of biophysical methods (Cunningham et al., Science 254:821-825, 1991), including X-ray crystallography (de Vos et al., Science 255:306-312, 1992), have shown that a single molecule of hGH binds simultaneously to two receptor molecules, each of which has a single site for hGH binding. This results in dimerization of the receptor, augmented by extensive interactions between the receptor molecules themselves, resulting in its activation.

The other proposed mechanism involves ligand-induced conformational changes in the receptor molecule that result in stabilization of its dimeric form. This mechanism is most likely when a single monomeric ligand molecule binds to each receptor. Studies of EGF binding to the extracellular domain of its receptor, using circular dichroism (CD) and fluorescence spectroscopy, have shown that conformational changes occur upon ligand binding (Greenfield et al., EMBO J. 8:4115-4123, 1989), and it has been proposed that these may be responsible for EGF-induced dimerization and activation of its receptor.

In the case of receptors that pre-exist in a dimeric form, such as the insulin receptor and bacterial chemotactic receptors, signal transduction is also proposed to occur through ligand-induced conformational changes. The aspartate receptor (Tar) of *S. typhimurium* has two binding sites for aspartate per dimer, which show negative cooperativity (Biemann and Koshland, Biochemistry 33:629-634, 1994). Binding of aspartate to one of these sites results in an altered juxtaposition of the two protomers of the dimer (Milburn et al., Science 254:1342-1347, 1991; Yeh et al., J. Biol. Chem. 268:9787-9792, 1993). Ligand-binding thus induces a transition between two particular dimeric states of the receptor, one of which is active. This can be considered as formally equivalent to a ligand-induced transition from a monomeric to a dimeric state, and shows many similarities with the activation of hGH-R.

The insulin receptor pre-exists as a dimer, with each protomer consisting of an α- and a β-chain. CD and fluorescence spectroscopic studies have shown that insulin binding is associated with substantial conformational changes in the extracellular domain of the receptor (Schaefer et al., J. Biol. Chem. 267:23393-23402, 1992).

It has previously been shown that EGF binds to the soluble extracellular domain of its receptor (sEGFR), and stimulates its dimerization (Lax et al., J. Biol. Chem. 266:13828-13833, 1991; Hurwitz et al., J. Biol. Chem. 266:22035-22043, 1991). The cross-linked dimeric form of sEGFR has been reported to have a higher affinity for EGF than the monomeric form (Zhou et al., Biochemistry 32:8193-8198, 1993). EGF does not appear to bind to accessory molecules. It must induce dimerization of its receptor by inducing conformational changes (Greenfield et al., EMBO J. 8:4115-4123, 1989) that stabilize the dimeric form, and/or by binding to the receptor in a multivalent manner, as observed for hGH (Cunningham et al., Science 254:821-825, 1991). Numerous attempts to crystallize the EGF/sEGFR complex for X-ray crystallographic analysis have so far been unsuccessful (Günther et al., J. Biol. Chem. 265:22082-22085, 1990), so a detailed view of ligand-induced conformational changes and dimerization is not available.

Abnormalities in signal transduction pathways can lead to various diseases in at least three different ways: (1) underactivity (2) mutation, and (3) overexpression. An example of underactivity is observed in some forms of diabetes. Examples of mutation include the role of BCR-ABL in chronic myelogenous leukemia and acute lymphocytic leukemia. Pendergrast et al., 1993, Cell 75:175-185.

Overexpression of certain protein tyrosine kinases has been shown to subvert normal growth control pathways and lead to oncogenesis (reviewed in Hunter, T., 1991, Cell 64:249-270). An example of a protein that may participate in the aberrant growth of breast cancer cells is HER2, also known as c-erbB-2 (Coussens et al., 1985 Science 230:1132-1139; Yamamoto et al., 1986, Nature, 319:521-527). This receptor was also isolated as the rat oncogene neu, an oncogene responsible for chemically induced rat glioblastomas (Padhy et al., 1982 Cell, 28:865-871; Schechter et al., 1984 Nature 312:513-516; Bargmann et al., 1986, Nature, 319:226-230). HER2/erbB-2 is known to be amplified and overexpressed in about 25% of human breast cancers (Slamon et al., 1987 Science 235:177-182; Slamon et al., 1989 Science 244:707-712).

Various cell proliferative disorders have been associated with defects in different signaling pathways mediated by receptor tyrosine kinases. According to Aaronson, Science, 254:1146-1153, 1991:
Signaling pathways that mediate the normal functions of growth factors are commonly subverted in cancer.

Examples of specific receptor tyrosine kinases associated with cell proliferative disorders include, platelet derived growth factor receptor (PDGFR), epidermal growth factor receptor (EGFR), and HER2. The gene encoding HER2 (*her*-2) is also referred to as neu, and *c-erb*B-2 (Slamon, D.J., et al., Science, 235:177-182, 1987). HER2/neu gene amplification has been linked by some investigators to neoplastic transformation.

Publications discussing EGFR and cancer include Zeillinger et al., Clin. Biochem. 26:221-227, 1993; where it is asserted:
Increased expression of this receptor [EGFR] has been found in various malignancies. In carcinomas of the cervix, ovaries, esophagus, and stomach, positive EGF-R status is definitely associated with the aggressiveness of the tumor.
With regard to breast cancer the importance attached to the determination of EGF-R has been confirmed by reports by several groups on the positive correlation between EGF-R and relapse-free interval, as well as overall survival. [Citations omitted.]

Other references discussing cancer and EGFR include Karameris et al., Path. Res. Pract. 189:133-137, 1993; Hale et al., J. Clin. Pathol. 46:149-153, 1993; Caraglia et al., Cancer Immunol. Immunother. 37:150-156, 1993; and Koenders et al., Breast Cancer Research and Treatment 25:21-27, 1993).

Some small molecules and antibodies have been studied for their ability to affect biological activities associated with receptors of the EGF family. Some compounds that have been studied for their ability to block EGF-dependant cell proliferation have been said to block EGF-receptor autophosphorylation less effectively than exogenous-substrate phosphorylation (Yaish et al., Science 242:933-935, 1988). The drug leflunomide has been reported to inhibit cellular proliferation by inhibiting tyrosine-specific kinase activity (Mattar et al., FEBS, 334 (2) :161-164, 1993). Other small molecules, termed tyrphostins, have been studied for their ability to selectively inhibit either HER1 or HER2 in vitro (Osberov et al., J. Biol. Chemistry 268:11134-11142, 1993). One study that was performed using antibodies has reported that blockade of the EGF receptor inhibits TGF-alpha induced, but not estrogen induced, hormone-dependant human breast cancer. Arteaga et al., Mol. Endocrinology, 2:1064-1070, 1988.

Compounds such as tryphostins and leflunomide act intracellularly to block the interaction between the kinase and its substrate rather than by blocking the dimerization or activation of the receptor, and thus must be able to traverse the cell membrane. The cyano group of the tryphostins may be toxic, especially if two cyano groups are present.

### Summary of the Invention

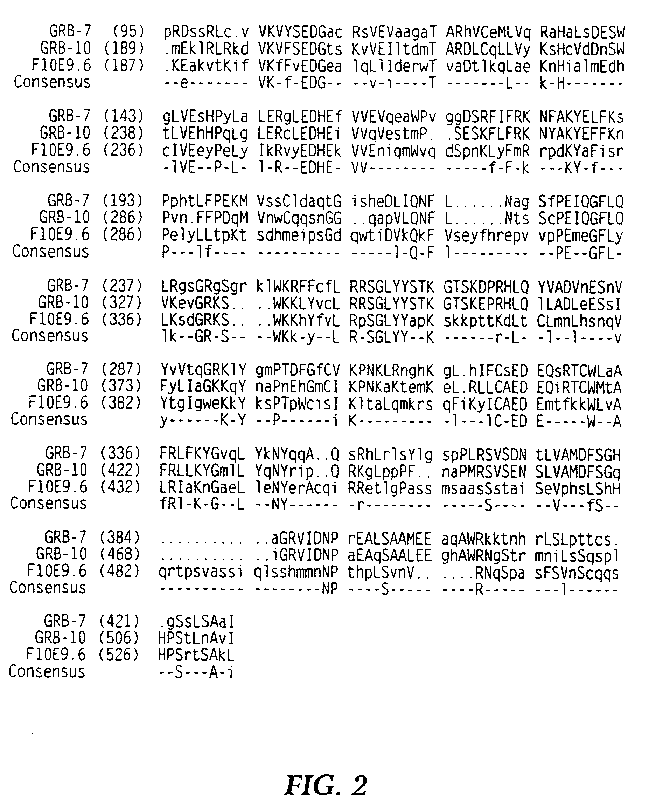

This invention relates to an isolated, purified, or enriched peptide consisting of a BLM domain wherein the BLM domain consists of amino acids 95 to 231 of GRB-7 or of amino acids 95 to 428 of GRB-7 shown in Figure 2. In a further aspect, this peptide is produced recombinantly and in another aspect this peptide is purified. In still another aspect, the invention relates to a composition comprising a peptide of the present invention and a pharmaceutically acceptable carrier or diluent.

This invention relates to peptides and compositions of the present invention which can be used for the treatment of a disease or condition in organisms, or for diagnosis of such diseases or conditions involved in abnormal protein-protein interactions.

These interactions are associated with the basic signalling function of proteins associated with various diseases or conditions. Such diseases or conditions include cell adhesion diseases, neuronal diseases, oncogenic disorders, diseases associated with defects in cellular movement or in developmental processes. For example, specific diseases or conditions include breast cancer, atherosclerosis, inflammation, and diseases associated with inappropriate activities of platelets, mononuclear, or neutrophil cells.

Measurement of the level of interaction has been determined to be a useful measure of the existence of certain diseases or conditions. The specific interactions encompassed by the present invention include the interaction between a BLM domain and its natural binding partner. Further described is the interaction between domain 1 and/or 3 of the EGF receptor and an EGF ligand. Such diseases or conditions are thus characterized by inappropriate interaction. Diseases or conditions characterized by abnormal levels of interaction can be alleviated to some extent by disrupting or promoting that interaction in vivo. In addition, useful agents for treatment of such diseases can be identified by standard screening protocols in which measurement of such interaction is determined. For example, such an agent may be a peptide which either comprises, consists of, or consists essentially of the BLM domain, domain 1 or 3 of thr EGF receptor, or a ligand that binds any of the above domains.

This invention relates to peptides and compositions of the present invention which can be used for the diagnosis and treatment of a disorder, most preferably a disorder characterized by an abnormality in a signal transduction pathway, wherein the signal transduction pathway involves one of the interactions mentioned above. The present invention is based in part on the discovery of a new domain, herein termed the BLM domain. Also described is that the simultaneous binding of a single molecule of EGF to two different regions on two receptor molecules causes ligand-induced stabilization of the active dimer of the receptors.

Thus, in a first aspect the invention features a peptide or a composition which can be used for the treatment of a disease or condition in an organism characterized by an abnormal level of interaction between: (a) a BLM domain and its natural binding partner. The use of an abnormal level of interaction between (b) domain 1 and/or 3 of an EGF receptor and an EGF ligand for the treatment of a disease or condition in an organism is also described. The disease or condition may also be characterized by an abnormality in a signal transduction pathway, wherein the pathway contains a protein with a domain listed above. The method includes disrupting or promoting that interaction (or signal) in vivo. The method also involves inhibiting the activity of the complex formed between the components listed above.

By "disease or condition" is meant a state in an organism, e.g., a human, which is recognized as abnormal by members of the medical community. The disease or condition may be characterized by an abnormality in one or more signal transduction pathways in a cell, preferably an epidermal or cancer cell, wherein one of the components of the signal transduction pathway is an EGF receptor or a protein having a BLM domain. The disease or condition may also be characterized by abnormal cell proliferation. Examples of diseases or conditions to be treated or diagnosed by using the peptides and compositions of the present invention include various cancers and cell proliferative disorders. Particular cancers that can be diagnosed and treated by using the peptides and compositions of the present invention include leukemia, epithelial carcinomas, cancers of the breast, bladder, lung, stomach and ovaries, and a variety of adenocarcinomas. These and other diseases or conditions are often characterized by one or more symptoms such as tumors.

"Cell proliferative disorders" refer to disorders wherein unwanted cell proliferation of one or more subset(s) of cells in a multicellular organism occurs, resulting in harm (e.g., discomfort or decreased life expectancy) to the multicellular organism. Cell proliferative disorders can occur in different types of animals and in humans. Cell proliferative disorders include cancers and psoriasis. A particular disorder is considered to be "driven" or caused by a particular receptor tyrosine kinase if the disorder is characterized by over-activity, or inappropriate activity, of the kinase and a compound which can inhibit the kinase activity exerts a therapeutic effect when administered to a patient having the disorder.

By "organism" is meant any living creature. The term includes mammals, and specifically humans. Preferred organisms include mice, as the ability to treat or diagnose mice is often predictive of the ability to function in other organisms such as humans.

By "abnormality" is meant an a level which is statistically different from the level observed in organisms not suffering from such a disease or condition and may be characterized as either an excess amount, intensity or duration of signal, or a deficient amount, intensity or duration of signal. The abnormality in signal transduction may be realized as an abnormality in cell function, viability or differentiation state. We have determined that such abnormal interaction in a pathway can be alleviated by action at the EGF receptor-EGF ligand interaction site in the pathway or at the BLM domain-natural binding partner site. An abnormal interaction level may also either be greater or less than the normal level and may impair the normal performance or function of the organism. Thus, it is also possible to screen for agents that will be useful for treating a disease or condition, characterized by an abnormality in the signal transduction pathway, by testing compounds for their ability to affect the interaction of interest, since the complex formed by such interaction is part of the signal transduction pathway. However, the disease or condition may be characterized by an abnormality in the signal transduction pathway even if the level of interaction between the components of interest is normal.

By "signal transduction pathway" is meant the sequence of events that involves the transmission of a message from an extracellular protein to the cytoplasm through a cell membrane. The signal ultimately will cause the cell to perform a particular function, for example, to proliferate and therefore cause cancer. Various mechanisms for the signal transduction pathway (Fry et al., 1993, Protein Science, 2:1785-1797) provide possible methods for measuring the amount or intensity of a given signal. Depending upon the particular disease associated with the abnormality in a signal transduction pathway, various symptoms may be detected. For example, if the disease is breast cancer, one may detect cell proliferation or tumor size, among other symptoms. Those skilled in the art recognize those symptoms that are associated with the various other diseases described herein. Furthermore, since some adaptor molecules recruit secondary signal transducer proteins towards the membrane, one measure of signal transduction is the concentration and localization of various proteins and complexes. In addition, conformational changes that are involved in the transmission of a signal may be observed using circular dichroism and fluorescence studies. One signal transduction pathway that has been studied is the Ras signalling pathway. (Pendergrast et al., 1993, Cell 75:175-185) The role of the GRB2 protein in the Ras signalling pathway (and the interaction with BCR-ABL, an oncoprotein implicated in certain human leukemias) was elucidated using a combination of biochemical and genetic studies. Similar studies are expected to elucidate the specific signalling pathway(s) affected by BLM domain containing proteins such as Grb7 or EGF receptor domain 1 or 3 containing proteins.

By "interact" is meant any physical association between proteins, whether covalent or non-covalent. Examples of non-covalent bonds include electrostatic bonds, hydrogen bonds, and Van der Waals bonds. Stryer, Biochemistry, 1988, pages 7-8. Furthermore, the interactions between proteins may either be direct or indirect. Thus, the association between two given proteins may be achieved with an intermediary agent, or several such agents, that connects the two proteins of interest. Another example of an indirect interaction is the independent production, stimulation, or inhibition of both an EGF receptor domain (i.e., domain 1 and/or domain 3) or BLM domain and EGF ligand or BLM domain natural binding partner by a regulatory agent (i.e., a gene transfer vehicle containing DNA encoding domain 1 and/or domain 3 of the EGF receptor or the BLM domain) . Depending upon the type of interaction present, various methods may be used to measure the level of interaction. For example, the strengths of covalent bonds are often measured in terms of the energy required to break a certain number of bonds (i.e., kcal/mol) Non-covalent interactions are often described as above, and also in terms of the distance between the interacting molecules. Indirect interactions may be described in a number of ways, including the number of intermediary agents involved, or the degree of regulatory control exercised over one domain relative to the regulatory control exercised over another domain to which the first domain binds.

The term "BLM domain" is defined below.

By "natural binding partner" is meant a protein that interacts with a BLM domain. Given that the PH domain is a subdomain of the present BLM domain, it is possible that some or all of the natural binding partners for the PH domain may also serve as natural binding partners for the BLM domain. In addition, a natural binding partner for a BLM domain may only interact with the BLM domain and not the PH domain. The structure (primary, secondary, or tertiary) of the particular natural binding partner will influence the particular type of interaction between the BLM domain and the natural binding partner. For example, if the natural binding partner comprises a sequence of amino acids complementary to the BLM domain, covalent bonding may be a possible interaction. Similarly, other structural characteristics may allow for other corresponding interactions. The interaction is not limited to particular residues and specifically may involve phosphotyrosine, phosphoserine, or phosphothreonine residues. A broad range of sequences may be capable of interacting with BLM domain. Experience with another signalling domain, the SH2 domain, has demonstrated that the range of sequences that binds that given domain may be much broader than was initially expected. Fry et al, Protein Science, 1993, 2:1785-1797. Therefore, using techniques well known in the art, one may identify several natural binding partners for the BLM domain.

By "EGF receptor" is meant a receptor that interacts with an EGF ligand through domains 1 and/or 3. An EGF receptor may be any member of the family or group of RTK's that is frequently overexpressed in a variety of epithelial carcinomas, such as EGFR, HER2, HER3 and HER4. (Plowman et al., Proc. Natl. Acad. Sci. USA 90: 1746-1750, 1993, incorporated herein by reference.) An EGF receptor typically has a hydrophobic transmembrane region that divides an extracellular ligand binding domain and a C-terminal cytoplasmic domain, both of which can be further be divided into four subdomains. Those skilled in the art can recognize other EGF receptors using techniques well-known in the art such as sequence comparison (i.e., looking for sequences with a similar length to the examples listed above and at least 34% identical to the extracellular domains, especially at the approximately 50 conserved cysteine residues, and at least 19% identical to the cytoplasmic domains). See, Plowman, supra. Other references that may be relevant to describing EGF receptors include Lax et al., EMBO 8:421-427, 1989; Avivi et al., Oncogene 6:673-673, 1991; Lax et al., Cell Regulation 2:337-345, 1991; and Lax et al., Molecular and Celluar Biology 8:1970-1978, 1988. EGF receptors are found in a variety of animals, including humans, chickens and mice. See, Avivi, supra.

By "EGF ligand" is meant a molecule, preferably a polypeptide, that binds to an EGF receptor, preferably at domain 1 and/or 3. Non-limiting examples of EGF ligands include EGF, transforming growth factor α, amphiregulin, heparin-binding EGF, vaccinia virus growth factor, gp30, and heregulin. Those skilled in the art can identify other EGF ligands using procedures well-known in the art.

Domains 1 and 3 of an EGF receptor are bound simultaneously by a single EGF ligand and are involved in EGF receptor dimerization and corresponding signal transduction and cell proliferation. By "domain 1" is meant a subdomain found in EGF receptors that binds EGF ligands. Domain 1 is typically flanking rather than cysteine-rich and generally corresponds approximately to residues 25-163 of EGFR or 29-185 of HER4. By "domain 3" is meant a subdomain found in EGF receptors that binds EGF ligands. Domain 3 is typically flanking, rather than cysteine-rich, and generally corresponds approximately residues 313-474 of EGFR or 335-495 of HER4. Domains 1 and 3 may include noncontiguous regions of an EGF receptor that is capable of binding to an EGF ligand. See, Lax, 1991, supra.

By "disrupt" is meant that the interaction is reduced either by preventing expression of a protein containing a BLM or EGF domain, or by preventing expression of its natural binding partner, or by specifically preventing interaction of the naturally synthesized proteins or by interfering with the interaction of the proteins.

By "promote" is meant that the interaction is increased either by increasing expression of a protein containing a BLM or EGF domain, or by increasing expression of its natural binding partner or ligand, or by promoting interaction of the proteins.

A method is described herein, which includes providing a molecule which blocks or promotes binding of said EGF ligand with domain 1 and/or domain 3 of the EGF receptor and disrupts or promotes EGF receptor dimerization (or signal) in vivo or in vitro. It is also described that the molecule is not an antibody, has a molecular weight below 10,000 and/or is a peptide that consists essentially of domain 1 or domain 3 or an EGF ligand fragment which binds one or both of these domains.

In a related aspect the invention features peptides and compositions of the present invention which can be used in a method for diagnosis of such a disease or condition by detecting the level of such interaction as an indication of that disease or condition. The diagnosis may also involve the detection of an abnormal amount or intensity of a signal generated by a signal transduction pathway, wherein the signal transduction pathway contains a protein having a BLM domain or a domain 1 and/or domain 3 of an EGF receptor.

By "diagnosis" is meant any method of identifying a symptom normally associated with a given disease or condition. Thus, an initial diagnosis may be conclusively established as correct by the use of additional confirmatory evidence such as the presence of other symptoms. Current classification of various diseases and conditions is constantly changing as more is learned about the mechanisms causing the diseases or conditions. Thus, the detection of an important symptom, such as the detection of an abnormal level of interaction (or the detection of an abnormal level of signal transduction in a pathway containing the interaction of interest) may form the basis to define and diagnose a newly named disease or condition. For example, conventional cancers are classified according to the location of the tumor, i.e, breast cancer, ovarian cancer, etc. However, a subset of these cancers are both associated with the overexpression of Her2, and as the role of Her2 becomes more fully understood, these cancers may be reclassified as Her2 cancers regardless of the location of the tumor.

Also described herein is a method for screening for an agent useful for treatment of such a disease or condition by assaying potential agents for the ability to disrupt or promote that interaction. The screening may also involve assaying potential agents for the ability to remove or reduce an abnormality in a signal transduction pathway, wherein the signal transduction pathway contains a protein with a BLM domain or domain 1 and/or domain 3 of an EGF receptor.

By "screening" is meant investigating organisms for the presence or absence of a property. The process includes measuring and detecting various properties, including the level of signal transduction and the level of interaction between the proteins and/or domains of interest that are listed above. Depending upon the type of interaction present, various methods may be used to measure the level of interaction. For example, the strengths of covalent bonds are often measured in terms of the energy required to break a certain number of bonds (i.e., kcal/mol) Non-covalent interactions are often described as above and also in terms of the distance between the interacting molecules. Indirect interactions may be described in a number of ways, including the number of intermediary agents involved, or the degree of control exercised over one domain relative to the control exercised over a different domain that binds the first domain.

The invention also features a peptide consisting of a BLM domain according to claim 1. Also described are peptides comprising, consisting or consisting essentially of domain 1 or domain 3 of an EGF receptor or a domain capable of inding any of the above domains. By "comprising" is meant including, but not limited to, whatever follows the word "comprising". Thus, use of the term "comprising" indicates that the listed elements are required or mandatory, but that other elements are optional and may or may not be present. By "consisting of" is meant including, and limited to, whatever follows the phrase "consisting of". Thus, the phrase "consisting of" indicates that the listed elements are required or mandatory, and that no other elements may be present. By "consisting essentially of" is meant including any elements listed after the phrase, and limited to other elements that do not interfere with or contribute to the activity or action specified in the disclosure for the listed elements. Thus, the phrase "consisting essentially of" indicates that the listed elements are required or mandatory, but that other elements are optional and may or may not be present depending upon whether or not they affect the activity or action of the listed elements.

According to claim 1 the peptide, protein, or polypeptide is isolated, purified, or enriched.

By "isolated" in reference to a polypeptide is meant a polymer of 2 (preferably 7, more preferably 13, most prefereably 25) or more amino acids conjugated to each other, including polypeptides that are isolated from a natural source or that are synthesized. The isolated polypeptides of the present invention are unique in the sense that they are not found in a pure or separated state in nature. Use of the term "isolated" indicates that a naturally occurring sequence has been removed from its normal cellular environment. Thus, the sequence may be in a cell-free solution or placed in a different cellular environment. The term does not imply that the sequence is the only amino acid chain present, but that it is essentially free (about 90 - 95% pure at least) of non-amino acid material naturally associated with it.

By the use of the term "enriched" in reference to a polypeptide is meant that the specific amino acid sequence constitutes a significantly higher fraction (2 - 5 fold) of the total of amino acids present in the cells or solution of interest than in normal or diseased cells or in the cells from which the sequence was taken. This could be caused by a person by preferential reduction in the amount of other amino acids present, or by a preferential increase in the amount of the specific amino acid sequence of interest, or by a combination of the two. However, it should be noted that enriched does not imply that there are no other amino acid sequences present, just that the relative amount of the sequence of interest has been significantly increased. The term significant here is used to indicate that the level of increase is useful to the person making such an increase, and generally means an increase relative to other amino acids of about at least 2 fold, more preferably at least 5 to 10 fold or even more. The term also does not imply that there is no amino acid from other sources. The other source amino acid may, for example, comprise amino acid encoded by a yeast or bacterial genome, or a cloning vector such as pUC19. The term is meant to cover only those situations in which man has intervened to elevate the proportion of the desired nucleic acid.

It is also advantageous for some purposes that an amino acid sequence be in purified form. The term "purified" in reference to a polypeptide does not require absolute purity (such as a homogeneous preparation); instead, it represents an indication that the sequence is relatively purer than in the natural environment (compared to the natural level this level should be at least 2-5 fold greater, *e.g.*, in terms of mg/ml). Purification of at least one order of magnitude, preferably two or three orders, and more preferably four or five orders of magnitude is expressly contemplated. The substance is preferably free of contamination at a functionally significant level, for example 90%, 95%, or 99% pure.

In preferred embodiments, the disease or condition which is diagnosed or treated using the peptide or composition of the present invention are those described herein, and the agent is a dominant negative mutant protein provided by gene therapy or other equivalent methods as described below. That is, the agent is a peptide which blocks interaction of the BLM domain with its natural binding partner. Also described is a peptide which blocks the interaction of the EGF domain with the EGF ligand. The peptide of the present invention may be recombinant, purified, or placed in a pharmaceutically acceptable carrier or diluent. The peptide may be used to produce antibodies and may either contain or be complementary to at least 20 amino acids from the BLM or EGF domain. In the screening method, transformed animals, e.g., mice containing proteins having a BLM or EGF domain, for example, the GRB-7 or GRB-10 protein, or the F10E9.6 protein (see below) may be used.

By "dominant negative mutant protein" is meant a mutant protein that interferes with the normal signal transduction pathway. The dominant negative mutant protein contains the domain of interest (e.g., the BLM domain), but has a mutation preventing proper signalling, for example by preventing binding of a second domain from the same protein. One example of a dominant negative protein is described in Millaur, B. et al., Nature February 10, 1994. In the present invention, the specific region is that corresponding to the BLM domain in GRB-7 (as shown in the figures) between bases encoding amino acids 95 to 428 or those bases 5' of the pleckstrin domain, i.e., between bases encoding amino acids 95 and 231.

Useful agents for treatment of such diseases can be identified by standard screening protocols in which measurement of such interaction is determined. For example, such an agent may be a peptide according to the present invention or a peptide which either comprises, consists of, or consists essentially of EGF receptor domains (i.e., domain 1 and domain 3) or, alternatively, a region of a natural binding partner or a region of an EGF ligand that binds one or more of these domains, or a fragment of any of these.

In the other preferred embodiments, the products produce a therapeutic effect. A "therapeutic effect" generally refers to either the inhibition, to some extent, of growth of cells causing or contributing to a cell proliferative disorder. A therapeutic effect relieves to some extent one or more of the symptoms of a cell proliferative disorder.

In reference to the treatment of a cancer, a therapeutic effect refers to one or more or the following: 1) reduction in tumor size; 2) inhibition (i.e., slowing to some extent, preferably stopping) of tumor metastasis; 3) inhibition, to some extent, of tumor growth; and/or 4) relieving to some extent one more or the symptoms associated with the disorder.

In reference to the treatment of a cell proliferative disorder other than a cancer, a therapeutic effect refers to either: 1) the inhibition, to some extent, of the growth of cells causing the disorder; 2) the inhibition, to some extent, of the production of factors (e.g., growth factors) causing the disorder; and/or 3) relieving to some extent one more or the symptoms associated with the disorder.

Other features and advantages of the invention will be apparent from the following description of the preferred embodiments thereof, and from the claims.

### Description of the Preferred Embodiments

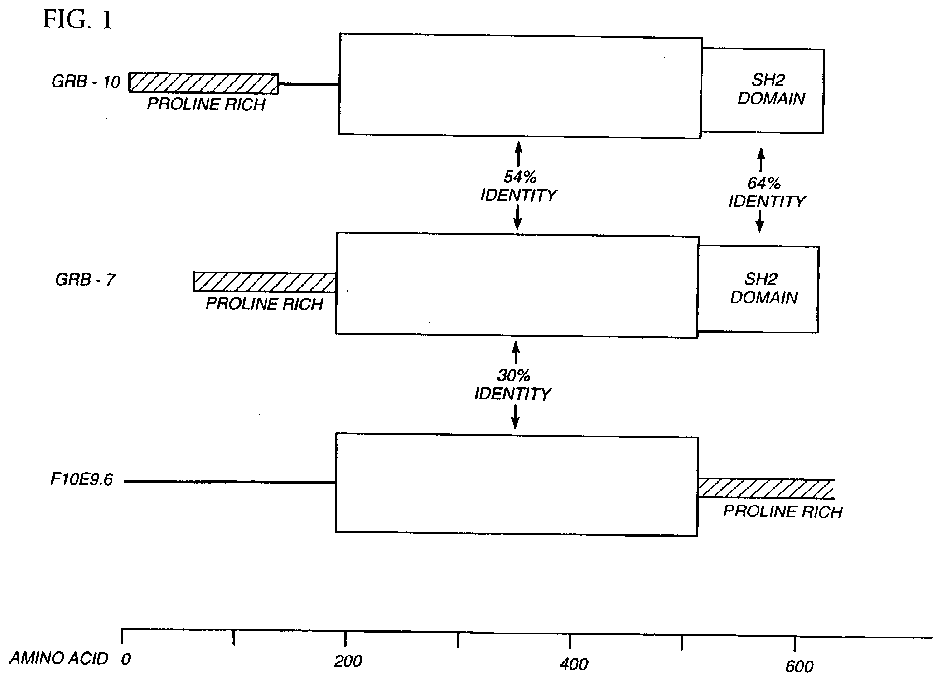

Fig. 1 is a diagrammatic representation of the BLM domain shown as regions of identity in the GRB-10, GRB-7, and F10E9.6 proteins at the amino acid level;
Fig. 2 is an alignment of the central domain of GRB-7, GRB-10, F10E9.6 including the BLM domain and within that the pleckstrin domain;

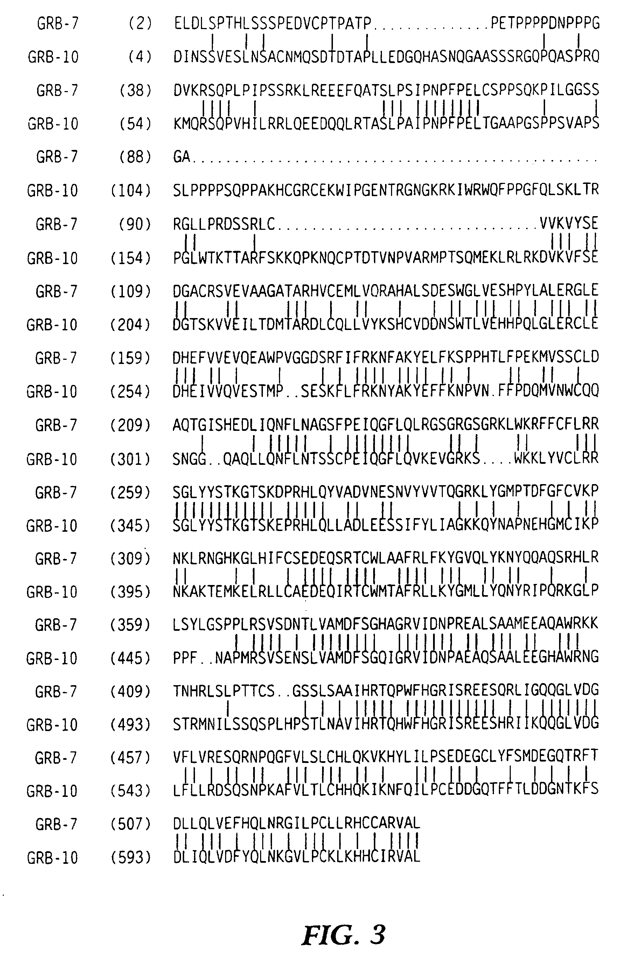

Fig. 3 is the amino acid sequence of GRB-7 compared to GRB-10 showing the BLM domain;

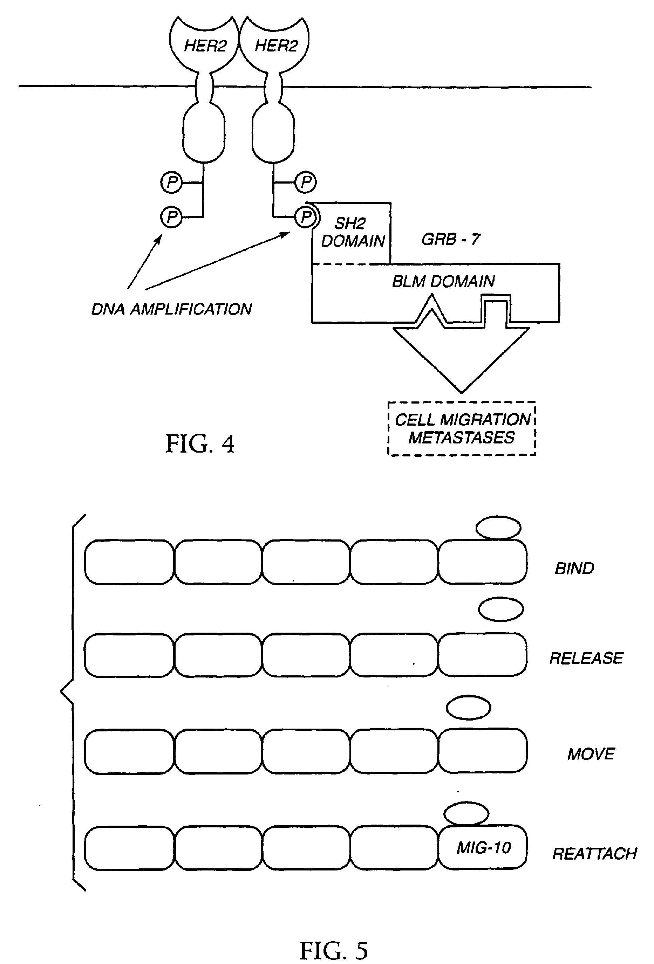

Fig. 4 is a diagrammatic representation of a proposed Her-2; GRB-7; and natural binding partner signalling process in breast cancer;
Fig. 5 is a diagrammatic representation of the association between a stationary cell and a cell which is moving.

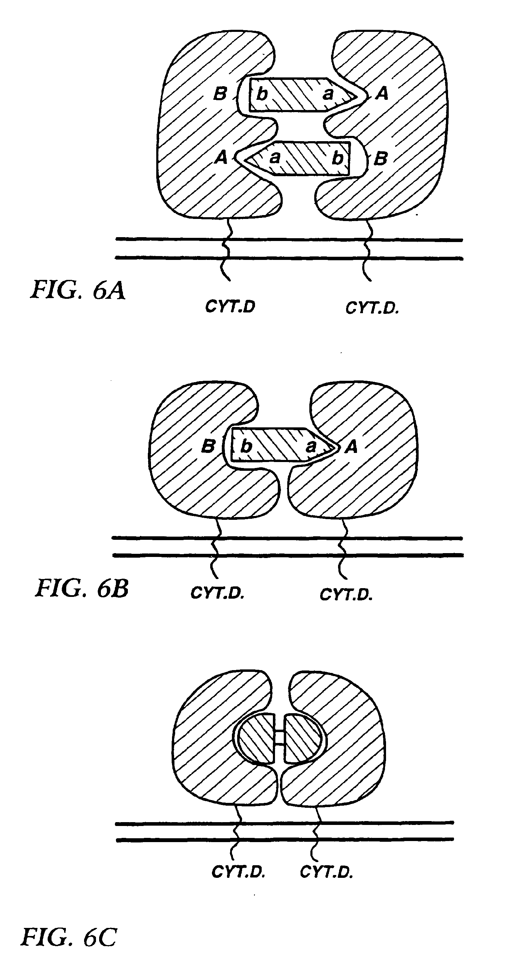

Figure 6 shows a model for the interaction between EGF and the extracellular domain of EGF receptor. Figure 6A shows that EGF has two binding sites for EGFR and that sEGFR has two binding sites for EGF, as indicated by isothermal titration calorimetry analysis. The high-affinity form of the ligand/receptor complex has a single molecule of EGF bound simultaneously to two receptor molecules. This occurs via the binding of different portions of an EGF molecule (a and b) to different half-sites on each of the two receptor molecules (sites A and B). One of these sites (site A) corresponds to subdomain 3 of the receptor, while site B corresponds to subdomain 1. Thus, the 1:2 EGF:EGFR (high-affinity) complex has a single EGF molecule bound between site A of one receptor and site B of its partner in a dimer (upper ligand molecule in Figure 1). The second (low affinity) event would involve a second EGF molecule interacting with the unoccupied site on each receptor molecule. A slightly unfavorable juxtaposition of the free sites or conformational changes could be responsible for the observed negative cooperativity. The result of the second event would be the arrangement depicted in Figure 1. Additional EGF-EGF interactions, or interactions between the two receptor molecules in the occupied EGFR dimer may also occur in this complex.
Figure 6B shows the similar mode of interaction between hGH and hGH-receptor. However, while hGH, like EGF, contains two binding sites for its receptor, the hGH receptor has only a single binding site for hGH (de Vos et al., Science 255:306-312, 1992). Binding of a second hGH molecule to the complex depicted in Figure 1B must therefore lead to its dissociation, as is indeed observed (Cunningham et al., Science 254:821-825, 1991; Fuh et al., Science 256:1677-1680, 1992). Additional receptor-receptor interactions, not depicted here, were also observed in the x-ray crystal structure of the 1:2 hGH:hGH-R complex (de Vos et al., Science 255:306-312, 1992).
Figure 6C contrasts these situations with one in which the ligand is monovalent, but dimeric, as in PDGF, CSF-1 or SCF. Independent interaction of the two ligand protomers with a single binding site in its receptor results in receptor dimerization and activation by virtue of the ligand's dimeric nature. Cyt.D. refers to the cytoplasmic domain.

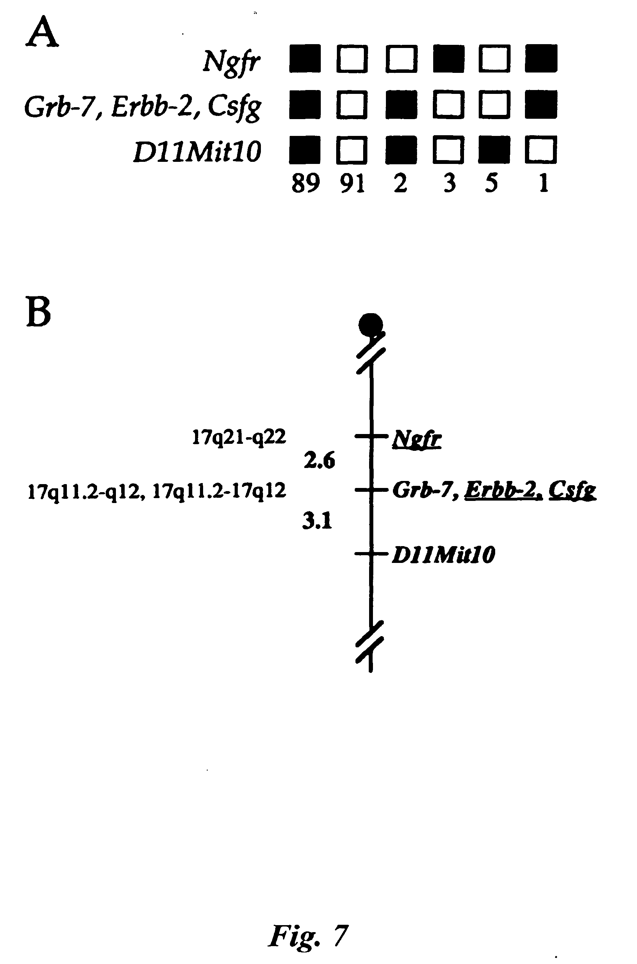

Fig. 7 GRB-7 maps on chromosome 11 near to Erbb-2/HER2. A. Summary of the results of the interspecific backcross analysis. Genes mapped in the analysis are listed on the left. Each column represents the chromosome identified in the N2 progeny inherited from the (AEj/GN X M. spretus) F1 parent. The closed boxes represent the AEj/GN allele and the open boxes represent the M. Spretus allele. The number of each type of chromosome identified in the backcross progeny are listed at the bottom. B. Genetic localization of GRB-7. The figure represents the region of mouse chromosome 11 analyzed in the interspedfic backcross. The genes mapped are listed on the right and the genetic map distance (in cMs) between adjacent loci, are listed on the left of the chromosome. The location of the human homologues of the genes are listed on the extreme left.

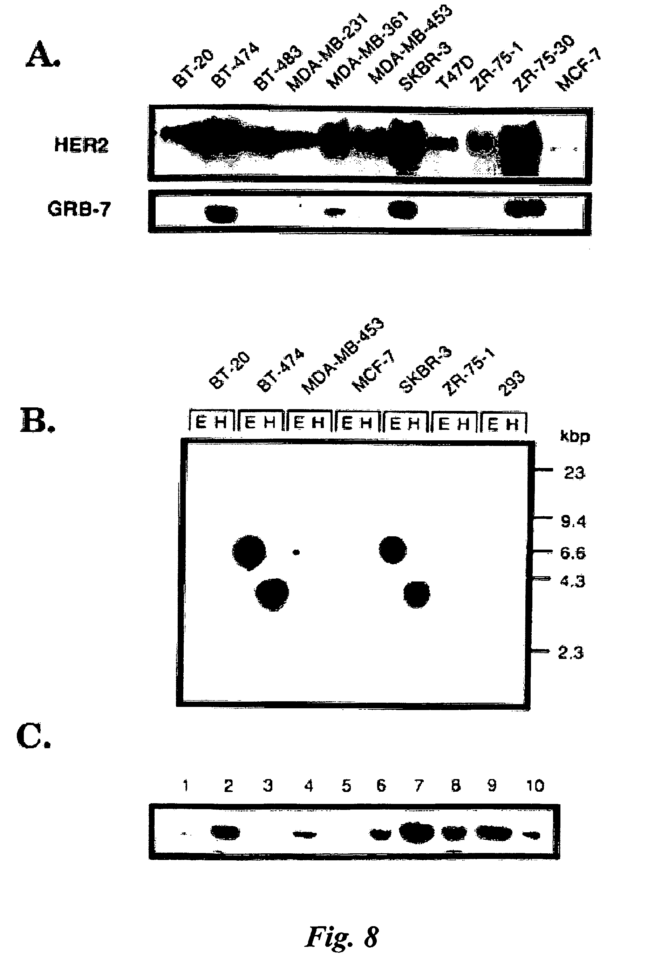

Fig. 8 GRB-7 is amplified and overexpressed in breast cancer cell lines. A Immunoblotting of GRB-7 in breast cancer cell lines. Two hundred micrograms of protein from cell lysates were run on an SDS gel and transferred to nitrocellulose. The nitrocellulose was then blocked and probed with either anti-GRB-7 (#188) antibody or anti-HER2 antibody. After detection with ¹²⁵I-protein A, blots were exposed to film. The HER2 blot was exposed for 4 hours and the GRB-7 blot for 12 hours. B. Southern blotting of GRB-7 in breast cancer cell lines. Ten micrograms of genomic DNA was digested with either EcoR1 (E) or Hind 111(H) and separated on a 0.7% agarose gel. All lanes represent breast cancer cell lines except 293 cells (human embryonic kidney) which were used as a control. Blotting and hybridization was carried out with a GRB-7 probe as described in the Materials and methods. C. Immunoblotting of GRB-7 in breast tumors. Frozen pulverized tumor powder was lysed in 5% SDS as described (Tandon et al., 1989) and 100 g of total solubilized protein was run on SDS-PAGE, transferred to nitrocellulose and immunoblotted with anti-GRB-7 (#188) antibody. After detection with ¹²⁵I-anti-rabbit sera (Amersham) the nitrocellulose blots were exposed to film. A representative blot of ten tumors is shown. All tumors overexpressed HER2 except #5. With the exception of #3 and #5, all tumors were considered positive for GRB-7 expression.

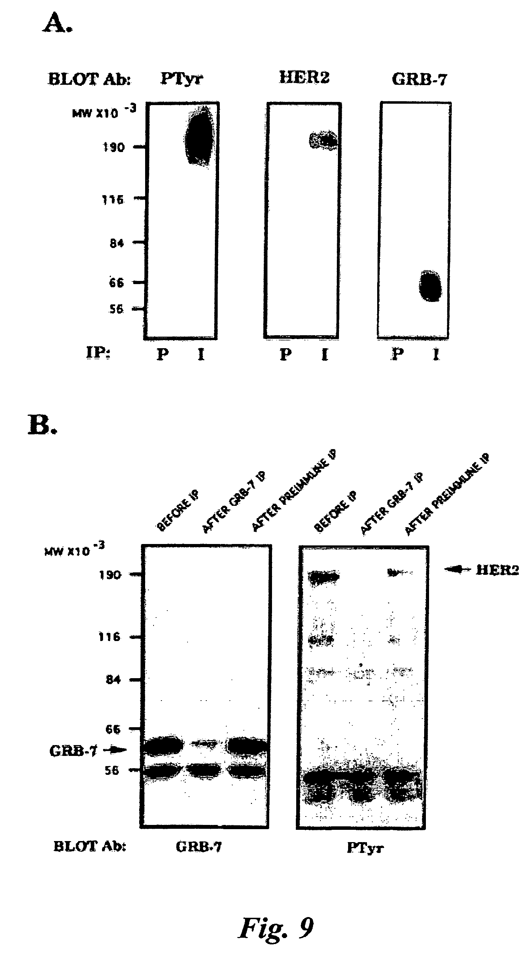

Fig. 9. GRB-7 is tightly bound to HER2. A Coimmunoprecipitation of HER2 and GRB-7. SKBR-3 cells were starved overnight in serum free medium and then lysed in 1% Triton X-100 lysis buffer with phosphatase and protease inhibitors. Lysates were then immunoprecipitated with antiGRB-7 (I) or preimmune (P) serum and separated by SDS-PAGE. After transfer to nitrocellulose, blots were probed with phosphotyrosine (PTyr), HER2 or GRB-7 (#188) antibodies. Blots were detected as in Figure 2. HER2 and PTyr blots represent immunoprecipitations from 2.5 mg of cellular protein while the GRB-7 blot is an immunoprecipitation from 1 mg of protein. GRB-7 and PTyr blots were exposed for 14 hours while the HER2 blot was exposed for 4 hours. B. GRB-7 iin:munoprecipitation clears tyoosine phosphorylated HER2 from SKBR-3 cell lysates. Five hundred micrograms of cell lysate from starved SKBR-3 cells was immunoprecipitated with 20 of preimmune or antiGRB-7 (#222) serum. Lysates (130 g) from before and after the immunoprecipitations were then immunoblotted with anti-Ptyr and anti-GRB-7 (#188) to determine what fraction of GRB-7 and tyrosine phosphorylated HER2 was cleared from the lysate by immunoprecipitation. The band at 56 kDa in the GRB-7 blot is an unidentified protein recognized by affinity purified antibody #188. It is not recognized by any of the other GRB-7 antibodies in immunoprecipitation or immunoblotting. Exposure time was 14 hours.

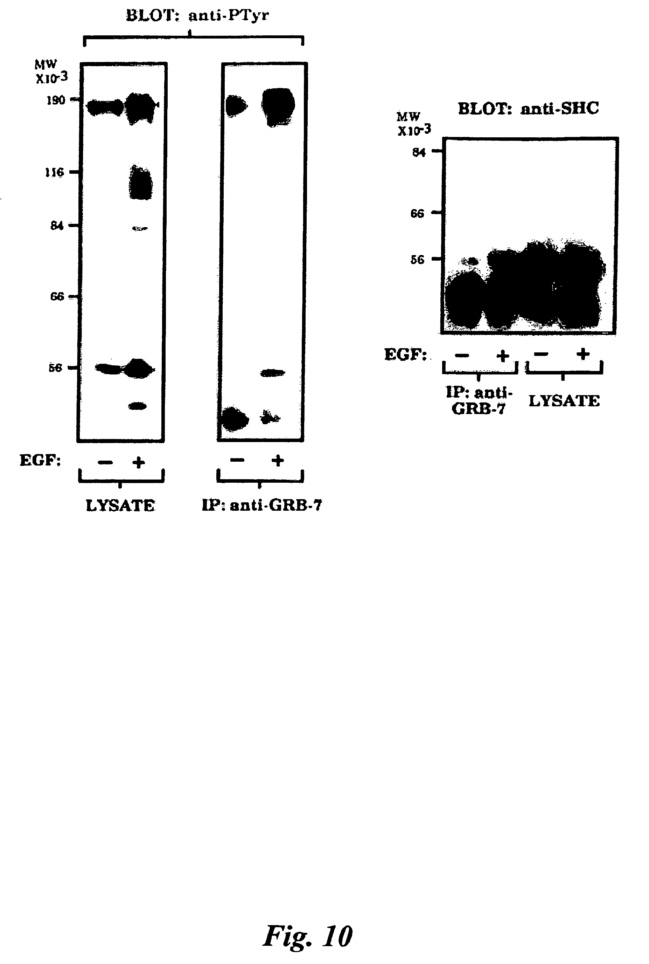

Fig. 10 GRB-7 antisera coimmunoprecipitates SHC from EGF stimulated SKBR-3 cell lysates. SKBR-3 cells were starved in serum free media overnight and then treated with or without 200 nM EGF for 3 minutes. After cell lysis, immunoprecipitation was performed with anti-GRB-7 (#222) or anti-SHC antibodies on 1.5 mg of cell lysate. Immunoprecipitates and cell lysate (130 mg) were then run on SDS-PAGE and transferred to nitrocellulose and immunoblotted with anti-phosphotyrosine (PTyr), or anti-SHC antibodies. In the SHC immunoblot, the band at approximately 50 kDa represents the IgG heavy chain in the GRB-7 immunoprecipitate while in the cell lysate it represents the 46 kDa form of SHC.

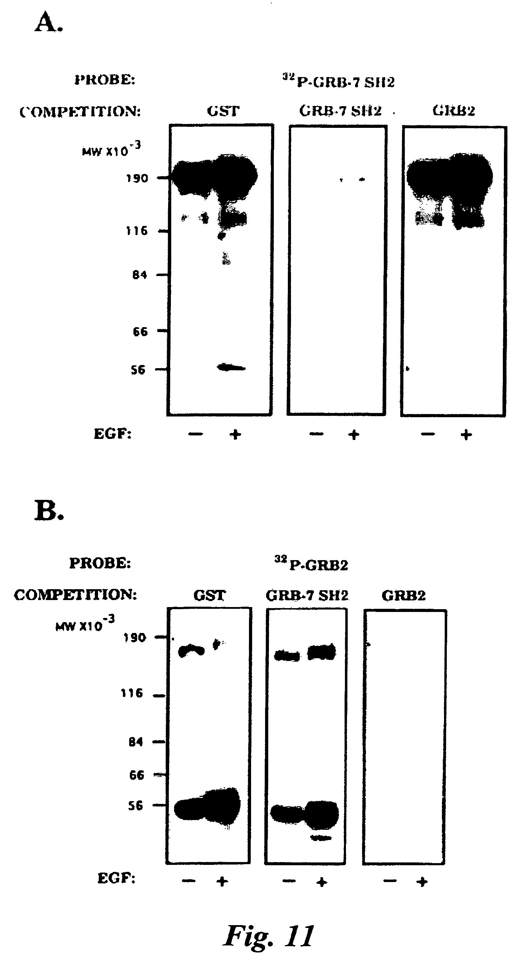

Fig. 11 Binding of GRB-7 SH2 domain and GRB2 to SKBR-3 lysates. GST fusion proteins of GRB-7 SH2 domain and full length GRB2 were prepared in a GST vector that incorporates a protein kinase A phosphorylation site. The fusion proteins were labelled using protein kinase A and g32P-ATP as described in materials and methods. SKBR-3 lysates, treated with or without EGF were then separated by SD S-PAGE and transferred to nitrocellulose. These blots were blocked for 2 hours at room temperature in 5% nonfat milk and incubated with 12.5 ng/ml (specific activity 2x10⁷ cpm/ug) of probe. Included with the probe was a 100 fold molar excess of either GST, GRB-7 SH2 or GRB2 protein. A GRB-7 SH2 domain probe, exposure time 12 hours. B. GRB2 probe, exposure time 2 hours.

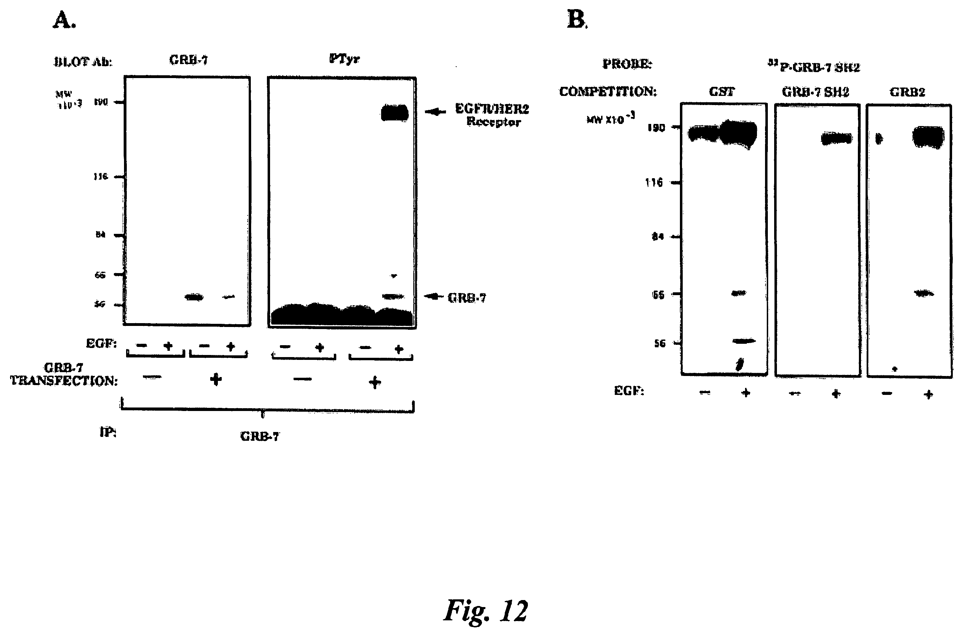

Fig. 12 GRB-7 phosphorylation and binding in NTH 3T3 cells expressing the EGFR-HER2 chimera. (A) Tyrosine phosphorylation of GRB-7. Cells expressing the EGFR-HER2 chimera with and without GRB-7 were starved in serum free media for 48 h and then treated with EGF for 3 min. Lysates (1.5 mg protein) were immunoprecipitated with GRB-7 antibodies and separated by SDS[EN]PAGE. After transfer to nitrocellulose, blots were probed with anti-PTyr or anti-GRB-7 antibodies. Eighty percent of the immunoprecipitate was run for anti-PTyr blot and 20% for GRB-7 blot. (B) GRB-7 SH2 domain associates with three proteins in NTH 3T3 cells expressing the EGFR-HER2 chimera. Lysates (50 µg) from cells expressing the chimeric receptor were run out on SDS[EN]PAGE and transferred to nitrocellulose. Blots were then probed with [³²P]GRB-7 SH2 domain as in Figure 11.

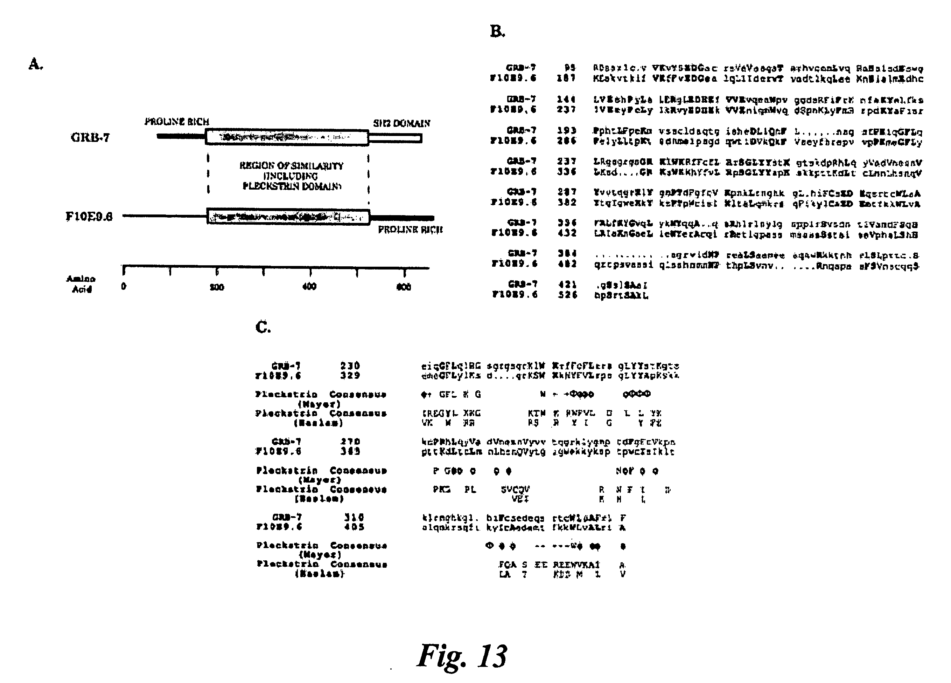

Fig. 13 Comparison of GRB-7 to the putative *C. Elegans* gene F10E9.6 and the consensus sequence for the pleckstrin domain. A. Schematic representation of GRB-7 and F10E9.6. F10E9.6 represents a putative gene derived from genomic sequence of *C. Elegans* using the program Genefinder. The sequences were deposited by the *C. Elegans* Sequencing Consortium, Genbank accession number L10986 (Sulston et al., 1992). B. Alignment of the region of similarity between GRB-7 and F1OE9.6 Alignment was performed using the GCG Bestflt program with bold capital letters indicating identity and plain capital letters indicating conservative substitution as defined by a score greater than 0.8 on the PAM 250 scoring table (Schwartz and Dayhoff, 1979). C. Alignment of GRB-7 and FlOE9.6 with the consensus sequences for pleckstrin domain. Bold capital letters indicate agreement with the consensus sequence as defined by both Mayer (Mayer et al., 1993) and Haslam (Haslam et al., 1993) while plain capital letters indicate agreement with only one of the consensus sequences. represents hydrophobic residue, and Ö represents aromatic residue.

The present invention relates to peptides and compositions which can be used in methods for the prevention, prognostic evaluation, and treatment of oncogenic and other disorders, especially breast cancer, wherein a protein having a BLM domain is capable of complexing with its natural binding partner or ligand and is involved in such a disorder.

Specifically, the present invention relates to peptides and compositions which can be used in methods for decreasing or inhibiting the interaction between the components of the two proteins and/or decreasing or inhibiting the activity of such complexes, and to methods for identifying useful agents for such compositions. The present invention also relates to peptides and compositions which can be used for removing or reducing an abnormality in a signal transduction pathway, wherein the signal transduction pathway contains a protein with a BLM domain. Further, the present invention relates to peptides and compositions which can be used for such methods for the treatment of the oncogenic disorders of interest, especially breast cancer. The present invention also relates to compositions which can be used in methods for the treatment of disorders which involve modulating the activity and/or level of individual components of the proteins. Also described herein are methods for the identification of agents for such treatments. Additionally, the present invention relates to compositions which can be used for prognostic evaluation of such disorders. Several abbreviations, defined below, are used to describe the present invention.

### ABBREVIATIONS

| **Amino Acid** | **One Letter code** | **Three Letter Code** |
|---|---|---|
| Alanine | A | Ala |
| Arginine | R | Arg |
| Asparagine | N | Asn |
| Aspartic Acid | D | Asp |
| Cysteine | C | Cys |
| Glutamic Acid | E | Glu |
| Glutamine | Q | Gln |
| Glycine | G | Gly |
| Histidine | H | His |
| Isoleucine | I | Ile |
| Leucine | L | Leu |
| Lysine | K | Lys |
| Methionine | M | Met |
| Phenylalanine | F | Phe |
| Proline | P | Pro |
| Serine | S | Ser |
| Threonine | T | Thr |
| Tryptophan | W | Trp |
| Tyrosine | Y | Tyr |
| Valine | V | Val |
| Not specified | X | |

### I. The BLM Domain

Referring to Fig. 1, examples of the BLM domain are shown diagrammatically in three newly characterized proteins. The region shown corresponds to amino acids between about 200 and 500 in the noted proteins, and includes the plextrin domain defined by Musacchio et al., 18 TIBS September 1993 (and references cited therein). The term "pleckstrin domain or PH domain" is used in this application as defined by Musacchio et al.) Those in the art will recognize that BLM equivalent domains are readily identified as those having at least 20% identity in the amino acid sequence in the noted regions or perform the same function despite a lower percentage of identity. The percentage of identity between two domains is calculated by dividing the number of amino acids that are the same in a given region by the total number of amino acids in the given region. Proteins including such domains are readily identified using standard protocols. Peptides containing a BLM domain may be used, for example, in binding experiments to identify natural binding partners, in the design of probes for particular proteins, in affinity columns, or other applications familar to those in the art. For example administration of the peptide of the present invention may disrupt or promote the interaction between a BLM domain and its natural binding partner.

Other proteins with BLM equivalent domains may be identified as those having at least 30% similarity or perform the same function despite having an even lower percentage of similarity. The percentage of similarity is calculated using a computer program (such as the GCG Bestfit program) that scores a protein based upon the number of gaps that must be induced to achieve similarity.

Regions that have at least 20% (preferably 30%, most preferably 60%) identity or 30% (preferably 40%, most preferably 70%) similarity are recognized as containing the same domain independent of any knowledge of the function of the proteins or domains. However, when knowledge regarding the function of the proteins or domain is known, then equivalent domains may be identified with much lower identity or similarity (e.g., 5-10% and 10-20% respectively). For example, the pleckstrin domain contains two proteins that only share approximately 5% identity to each other. Musacchio, id.

Those skilled in the art will be able to identify other proteins with a BLM domain using routine procedures in the art, such as sequence comparisions, three-dimensional structure comparisons, analysis of functional activities or properties, etc. For example, many PH domains have been identified by those in the art in a short period of time despite the widely divergent sequences of PH domains and the lack of consensus regarding the possible function of the PH domain. See, Gibson et al., TIBS, 19:349-353 (1994). This analogy is particularly apt, given that the BLM domain contains a PH domain within it.

Referring to Fig. 2, the amino acid sequence of the three proteins, shown in Fig. 1, are provided with the consensus sequence. The standard single letter amino acid code is used in the figure. The BLM domain is that shown in GRB-7 between amino acids 95 and 428, and the 5' region of the BLM domain is that between amino acids 95 and 231. Thus, the 5' region is 5' of the pleckstrin domain.

Referring to Fig. 3, the protein sequence of GRB-7 and GRB-10 is shown, demonstrating the high degree of conservation in the BLM domain and pleckstrin domain.

### II. BLM Domain Interaction

Referring to Fig. 4, the present invention is based upon the interaction of the BLM domain of proteins such as GRB-7 with a natural binding partner (shown as a shaded triangle in the lower portion of the Figure).

Specifically, Fig. 4 shows the interaction of Her-2 with GRB-7 in the SH2 domain. See, Stein et al., 13 EMBO Journal, 1994 (Appendix A, including figures, hereby incorporated by reference herein). This invention is designed to measure and/or to affect this interaction between GRB-7, or an equivalent BLM domain from this or another protein, and the natural binding partner of that domain. By such control of the interaction of the BLM domain with its natural binding partner, diseases associated with problems in cell migration and metastases can be diagnosed and treated.

For example, referring to Fig. 5 there is shown a diagrammatic representation of the role of the interaction between the BLM domain and its natural binding partner in movement of cells. Specifically, a mobile cell binds another cell. Without being bound to any particular theory of the invention, it is presently believed that the interaction between the BLM domain and its natural ligand may be involved in the signal transduction pathway that controls the affinity of cell receptors, cell adhesion, and cell migration.

Isolation of the GRB-7 gene is described in Margolis et al., (U.S. Patent Application Serial No. 07/906,349, filed June 30, 1992). GRB-10 was cloned from λEXlox NIH 3T3 (mouse fibroblast cell line) using the CORT technique. The probe was the EGF-Receptor carboxyterminus and the methodology the same as described for the other GRB's (Skolnik et al. 65 Cell 83-90, 1991; Lowenstein et al., 70 Cell 431, 1992; Margolis et al., PNAS, 89:8894, 1992. The randomly primed NIH 3T3 library was generated in our laboratory using standard techniques (Sambrook et al., Molecular Cloning. A Laboratory Manual, Cold Spring Harbor Laboratory Press, Cold Spring Harbor, 1989). After the initial clone was isolated, GRB-10 cDNA encoding the full length GRB-10 protein was cloned from the same library using DNA hybridization as described (Margolis et al., 3 Cell Growth & Differ., 73, 1992; Margolis et al., 89 Proc. Natl. Acad. Sci. USA 8894, 1992). The F10E9.6 gene corresponds to that described by Manser and Wood as the mig-10 gene. Manser and Wood, 11 Developmental Genetics 49, 1990.

### III. EGF Receptor Dimerization

Here we describe studies of the stoichiometry and thermodynamics of binding of the monomeric growth factor EGF to the soluble extracellular domain of the respective receptor (which is monomeric). Of the systems in which ligand:receptor interactions have been studied extensively, this is among the few cases in which the available data suggest a monovalent mode of interaction (Weber et al., J. Biol. Chem. 259:14631-14636, 1984).

We have compared the binding of epidermal growth factor (EGF) to the soluble extracellular domain of the respective receptor using isothermal titration calorimetry. EGF shows two modes of binding to its receptor, one with high affinity (K_{D} ≈ 10 nM) and one with lower affinity (K_{D} ≈ 200 nM). Surprisingly, each of these binding modes occurs with a stoichiometry corresponding to 1:2 (EGF:receptor), indicating that the high-affinity complex contains two receptor molecules and a single ligand, as seen in the case of human growth hormone bound to its receptor.

Binding of EGF to the soluble extracellular domain caused receptor dimerization as revealed by treatment with the covalent cross-linking agent DSS and SDS-PAGE. However, an isolated subdomain (domain 3) of the receptor bound EGF with a simple 1:1 stoichiometry, and did not dimerize upon binding of EGF. Thus, simultaneous binding of a single molecule of EGF to different regions on two receptor molecules is responsible for ligand-induced stabilization of the active dimeric form of its receptor.

Isothermal titration calorimetry (ITC) permits analysis of the stoichiometries of binding. The data presented here indicate that, while aFGF forms a simple 1:1 complex (K_{D} = 500 nM) with the extracellular domain of its receptor, EGF initially forms a 1:2 (EGF:sEGFR) complex (K_{D} ≈ 10 nM). A second molecule of EGF then binds to this complex with a lower affinity (K_{D} ≈ 200 nM). Thus EGF binds simultaneously to two receptor molecules, which may be important for ligand-induced stabilization of the active receptor dimer. In efforts to understand the mechanism via which binding of a monomeric growth factor leads to the dimerization of its cognate receptor, we have compared the stoichiometry and thermodynamic parameters associated with binding of EGF to soluble forms of the respective receptor.

EGF can induce dimerization of sEGFR in the absence of accessory molecules (Lax et al., J. Biol. Chem. 266:13828-13833, 1991; Hurwitz et al., J. Biol. Chem. 266:22035-22043, 1991). This effect is likely to result from ligand-induced conformational changes that stabilize the receptor dimer and/or multivalent binding of EGF to its receptor, as has been described for hGH (Cunningham et al., Science 254:821-825, 1991; de Vos et al., Science 255:306-312, 1992). The data presented here indicate that multivalent binding is also significant, since a single molecule of EGF can bind simultaneously to two molecules of sEGFR. It also appears that sEGFR itself has two EGF-binding sites, represented by subdomains 1 and 3. Isolated domain 3 binds EGF in a simple monovalent fashion.

### IV. Models for EGF Binding to sEGFR

There are two possible arrangements in which a single EGF molecule could bind with high-affinity to a dimer of sEGFR, to form the 1:2 (EGF:sEGFR) complex reported here. In one scenario, a single EGF binding-site occurs per receptor molecule, and its occupancy leads to conformational changes in the receptor. These changes, in turn, lead to association of the occupied receptor with a second unoccupied receptor molecule. The result is an asymmetric dimer of the receptor, in which the unoccupied protomer has a reduced affinity for EGF. This is equivalent to the negative cooperativity observed, for example, with four successive molecules of NAD⁺ that bind with successively lower affinity to tetrameric glyceraldehyde-3-phosphate dehydrogenase (Schlessinger and Levitzki, J. Mol. Biol. 82:547-561, 1974). Wofsy et al. (Biophys. J. 63:98-110, 1992) have shown that models of this nature cannot adequately account for the concave-up curvature, characteristic of negative cooperativity, that is observed in Scatchard plots obtained in for EGF binding to its receptor. Our finding that sEGFR itself has two binding sites for EGF thus indicate an alternative model.

In the alternative scenario, a single EGF molecule would bind simultaneously to two receptor molecules by interacting with a different site on each. It thus binds across the dimer interface in a manner similar to that described for hGH binding to hGH-R (de Vos et al., Science 255:306-312, 1992), stabilizing the active dimeric form of the receptor. Titrations of sEGFR into solutions of EGF showed that two EGF molecules bind to a single receptor molecule.

One site of interaction is subdomain 3 of the receptor, since it is able to bind EGF in isolation. On the basis of sequence homology (Lax et al., Mol. Cell. Biol. 8:1970-1978, 1988) and deletion mutagenesis (Lax et al., Cell Regulation 1:173-188, 1990), we determined that subdomain 1 represents the other site. Thus, EGF binds to one receptor in the 1:2 (EGF:sEGFR) complex via interactions with subdomains 1 and 3. Interaction with the other receptor molecule could also occur via subdomain 3. However, since EGF is an asymmetric molecule, and two EGF binding sites are present in each sEGFR molecule, interaction with the second receptor is more likely to occur via subdomain 1, as depicted in Figure 6A. This suggests one contrast with the hGH/hGH-R system (Figure 6B).

While hGH is bivalent, hGH-R is effectively monovalent, since it binds to two different portions of hGH via essentially the same site on the receptor (de Vos et al., Science 255:306-312, 1992). Formation of a 2:1 (hGH:hGH-R) complex is therefore not possible, and binding of a second hGH molecule to the 1:2 (hGH:hGH-R) complex must necessarily dissociate the receptor dimer (Cunningham et al., Science 254:821-825, 1991; Fuh et al., Science 256:1677-1680, 1992). In the case of EGFR, both ligand and receptor appear to be bivalent. This being the case, it is possible that binding of a second EGF molecule to the 1:2 (EGF:sEGFR) complex could occur without dissociation of the sEGFR dimer, as depicted in Figure 6A. The second EGF molecule would bind to the unoccupied site on each receptor molecule.

The reduced affinity for this second EGF molecule could result from conformational changes in the receptor that alter the two binding sites. Alternatively, their position with respect to one another could be restricted such that simultaneous binding of a single EGF molecule to both is disfavored. The negative cooperativity that is observed for ATP binding to yeast hexokinase provides an illustration of this. Each protomer of a hexokinase dimer contributes two partial sites for ATP binding, and ATP is bound across the dimer interface via interactions with one partial site of each protomer (Anderson and Steitz, J. Mol. Biol. 92:279-287, 1975). The other partial sites are then positioned such that their simultaneous interaction with ATP is disfavored.

### V. Comparison with other Receptors

Recent studies of ligand binding to the aspartate receptor (Tar) from S. typhimurium (Biemann and Koshland, Biochemistry 33:629-634, 1994) suggest a model where each receptor dimer has two binding sites for aspartate, each of which is contributed to by side-chains from both protomers of the dimer. Two aspartate molecules bind per dimer, but marked negative cooperativity is observed. Analysis of the X-ray crystal structure of the Tar periplasmic domain (Milburn et al., Science 254:1342-1347, 1991) showed that binding of one aspartate molecule to the receptor dimer, via interactions with both protomers, leads to slight conformational changes in the second site. This is proposed to account for the observed negative cooperativity (Biemann and Koshland, Biochemistry 33:629-634, 1994), and is likely to be paralleled in the case of EGF binding to sEGFR.

A wealth of data also suggest a similar model for insulin binding to its receptor (de Meyts, "The structural basis of insulin and insulin-like growth factor-I (IGF-I) receptor binding and negative cooperativity, and its relevance to mitogenic versus metabolic signaling", Diabetologia, in press, 1994; Soos et al., Biochem. J. 48:419-426, 1993). Both insulin and the insulin receptor α-subunit appear to be bivalent. High-affinity binding of insulin to its receptor involves the bivalent interaction of a single insulin molecule with a different site on each of two α-subunits, spanning the interface of the pre-existing receptor dimer. Additional insulin molecules can then bind to this complex, although with reduced affinity as a result of conformational changes induced by binding of the first molecule (Schaefer et al., J. Biol. Chem. 267:23393-23402, 1992).

The model presented here for EGF binding to EGFR suggests novel ways in which receptor antagonists may be designed, which could be of therapeutic value. The rational design of potent antagonists to hGH-R (Fuh et al., Science 256:1677-1680, 1992) has been shown to be feasible.

### VI. Thermodynamics of EGF Binding

One difference observed between the thermodynamic parameters for EGF binding to sEGFR and the binding of other receptors to their ligands was the different sign of the enthalpy. The interaction is predominantly enthalpy-driven for binding of hGH to its receptor (ΔH = -27 kcal/mol) (Cunningham et al., Science 254:821-825, 1991), and aspartate binding to Tar of E. coli (ΔH = -17 kcal/mol).

By contrast, EGF binding to sEGFR(1234) had a positive enthalpy value (ΔH₁ = 6.8 kcal/mol ΔH₂ = 10.4 kcal/mol). EGF binding to sEGFR(1234) is therefore entropy-driven, implying that the system becomes more disordered upon binding of EGF to sEGFR. Parts of the system that may become more disordered (and negate the negative entropic effect of bringing ligand and macromolecule together) include the ligand, the macromolecule, and the solvent. One possible explanation for this would be that EGF binding or sEGFR(1234) dimerization results in the burial of significant areas of hydrophobic surface, around which water would be ordered in the unbound state. Release of this water to the bulk would provide an entropic driving force.

That EGF binding to domain 3 has a negative enthalpy (ΔH = -2 kcal/mol) indicates that this does not result directly from EGF binding. The entropic driving force could also arise from a disordering of the receptor structure itself. Indeed, there is evidence for this from studies of the effect of EGF upon the decay of fluorescence anisotropy suggesting an increase in flexibility in the receptor upon EGF binding, (Greenfield et al., EMBO J. 8:4115-4123, 1989), whereas a similar analysis of the insulin receptor suggested increased rigidity upon ligand binding (Schaefer et al., J. Biol. Chem. 267:23393-23402, 1992).

### VII. Compositions

Described herein is removing or reducing an abnormality in a signal transduction pathway, wherein the signal transduction pathway contains an EGF receptor domain (i.e., domain 1 and/or domain 3) and an EGF ligand. The present invention also relates to compositions which can be used in methods for the treatment of disorders which involve modulating the activity and/or level of individual components, and relates to methods for the identification of agents for such treatments. Additionally, the present invention relates to compositions for prognostic evaluation of such disorders.

Described herein are compositions and methods for the prevention, prognostic evaluation, and treatment of disorders listed herein. Also described are compositions and methods for the prevention, prognostic evaluation and treatment of cell proliferative disorders, especially cancer, in which an EGF ligand is involved.

First, methods and compositions for the treatment of such disorders are described. Such methods and compositions may include, but are not limited to the agents capable of decreasing or inhibiting the interaction between EGF receptor domains (i.e., domains 1 and/or 3) and EGF ligands and agents capable of inhibiting or decreasing the activity of such complexes, agents capable of modulating the activity and/or level of individual components of the proteins, and the use and administration of such agents.

Second, methods are described for the identification of such agents. These methods may include, for example, assays to identify agents capable of disrupting or inhibiting or promoting the interaction between components of the complexes (e.g., EGF ligand:EGF receptor complexes), and may also include paradigms and strategies for the rational design of drugs capable of disruption and/or inhibition and/or promotion of such complexes.

### VIII. Ligand/Receptor Complexes

The complexes include proteins with an EGF ligand domain or proteins with an EGF receptor domain or derivatives thereof, as described below. Other complexes which are involved in the invention include at least one member of the BLM domain-containing family of proteins, and/or a derivative thereof, and at least one member of a natural binding ligand containing protein or a derivative thereof, as described below. Under standard physiological conditions, the components of such complexes are capable of forming stable, non-covalent attachments with one or more of the other complex components. Methods for the purification and production of such protein complexes, and of cells that exhibit such complexes are described below.

### IX. Disruption of Protein Complexes

Disruption of BLM domain-containing associated protein complexes, for example by decreasing or inhibiting the interactions between component members such a complex may have differing modulatory effects on the event involved, depending on the individual protein complex.

"Disruption", as used here, is meant to refer not only to a physical separation of protein complex components, but also refers to a perturbation of the activity of the complexes, regardless of whether or not such complexes remain able, physically, to form.

"Activity", as used here, refers to the function of the protein complex in the signal transduction cascade of the cell in which such a complex is formed, i.e., refers to the function of the complex in effecting or inhibiting a transduction of an extracellular signal into a cell. For example, the effect of complex disruption may augment, reduce, or block a signal normally transduced into the cell. Likewise, depending on the disorder involved, either augmentation, reduction, or blockage of a signal normally transduced into the cell will be desirable for the treatment of the disorder.

A disorder involving a complex may, for example, develop because the presence of such a complex brings about the aberrant inhibition of a normal signal transduction event. In such a case, the disruption of the complex would allow the restoration of the usual signal transduction event. Further, an aberrant complex may bring about an altered subcellular adaptor protein localization, which may result in, for example, dysfunctional cellular events. An inhibition of the complex in this case would allow for restoration or maintenance of a normal cellular architecture. Still further, an agent or agents that cause(s) disruption of the complex may bring about the disruption of the interactions among other potential components of a complex.

Nucleotide sequences encoding peptide agents which are to be utilized intracellularly may be expressed in the cells of interest, using techniques which are well known to those of ordinary skill in the art. For example, expression vectors derived from viruses such as retroviruses, vaccinia virus, adenoviruses, adeno-associated virus, herpes viruses, or bovine papilloma virus, may be used for delivery and expression of such nucleotide sequences into the targeted cell population. Methods for the construction of such vectors are well known. See, for example, the techniques described in Maniatis et al., 1989, Molecular Cloning A Laboratory Manual, Cold Spring Harbor Laboratory, N.Y. and Ausubel et al., 1989, Current Protocols in Molecular Biology, Greene Publishing Associates and Wiley Interscience, N.Y. Complex-binding domains can be identified using, for example, techniques such as those described in Rotin et al. (Rotin, D. et al., EMBO J. 11:559-567), Songyang et al. (Songyang, S. et al., 1993, Cell 72:767-778), Felder, S. et al., 1993, Mol. Cell. Biol. 13:1449-1455, Fantl, W.J. et al., 1992, Cell 69:413-422, and Domchek, S.M. et al., 1992, Biochemistry 31:9865-9870.

Alternatively, antibodies capable of interfering with complex formation may be produced as described below and administered for the treatment of disorders involving a component capable of forming a complex with another protein. For example, neutralizing antibodies which are capable of interfering with ligand binding may be administered using standard techniques. Alternatively, nucleotide sequences encoding single-chain antibodies may be expressed within the target cell population by utilizing, for example, techniques such as those described in Marasco et al. (Marasco, W. et al., 1993, Proc. Natl. Acad. Sci. USA 90:7889-7893).

Agents which act intracellularly to interfere with the formation and/or activity of the protein complexes of the invention may also be small organic or inorganic compounds. A method for identifying these and other intracellular agents is described below.

### X. Antibodies to Complexes

Described herein are methods for the production of antibodies which are capable of specifically recognizing a complex or an epitope thereof, or of specifically recognizing an epitope on either of the components of the complex, especially those epitopes which would not be recognized by the antibody when the component is present separate and apart from the complex. Such antibodies may include, but are not limited to polyclonal antibodies, monoclonal antibodies (mAbs), humanized or chimeric antibodies, single chain antibodies, Fab fragments, F(ab')₂ fragments, fragments produced by a FAb expression library, anti-idiotypic (anti-Id) antibodies, and epitope-binding fragments of any of the above. Such antibodies may be used, for example, in the detection of a complex in a biological sample, or, alternatively, as a method for the inhibition of a complex formation, thus, inhibiting the development of a disorder.

Polyclonal antibodies are heterogeneous populations of antibody molecules derived from the sera of animals immunized with an antigen, such as a complex, or an antigenic functional derivative thereof. For the production of polyclonal antibodies, various host animals may be immunized by injection with the complex including but not limited to rabbits, mice, rats, etc. Various adjuvants may be used to increase the immunological response, depending on the host species, including but not limited to Freund's (complete and incomplete), mineral gels such as lysolecithin, pluronic polyols, polyanions, peptides, oil emulsions, keyhole limpet hemocyanin, dinitrophenol, and potentially useful human adjuvants such as BCG (bacille Calmette-Guerin) and Corynebacterium parvum.

A monoclonal antibody, which is a substantially homogeneous population of antibodies to a particular antigen, may be obtained by any technique which provides for the production of antibody molecules by continuous cell lines in culture. These include, but are not limited to the hybridoma technique of Kohler and Milstein, (Nature 256:495-497 (1975) and U.S. Patent No. 4,376,110), the human B-cell hybridoma technique (Kosbor et al., 1983, Immunology Today 4:72; Cole et al., 1983 Proc. Natl. Acad. Sci. USA 80:2026-2030), and the EBV-hybridoma technique (Cole et al., 1985, Monoclonal Antibodies And Cancer Therapy, Alan R. Liss, Inc., pp. 77-96). Such antibodies may be of any immunoglobulin class including IgG, IgM, IgE, IgA, IgD and any subclass thereof. The hybridoma producing the mAb may be cultivated in vitro or in vivo. Production of high titers of mAbs in vivo makes this the presently preferred method of production.

In addition, techniques developed for the production of "chimeric antibodies" (Morrison et al., 1984, Proc. Natl. Acad. Sci., 81:6851-6855; Neuberger et al., 1984, Nature, 312:604-608; Takeda et al., 1985, Nature, 314:452-454) by splicing the genes from a mouse antibody molecule of appropriate antigen specificity together with genes from a human antibody molecule of appropriate biological activity can be used. A chimeric antibody is a molecule in which different portions are derived from different animal species, such as those having a variable region derived from a murine mAb and a human immunoglobulin constant region.

Alternatively, techniques described for the production of single chain antibodies (U.S. Patent 4,946,778; Bird, 1988, Science 242:423-426; Huston et al., 1988, Proc. Natl. Acad. Sci. USA 85:5879-5883; and Ward et al., 1989, Nature 334:544-546) can be adapted to produce complex-specific single chain antibodies. Single chain antibodies are formed by linking the heavy and light chain fragment of the Fv region via an amino acid bridge, resulting in a single chain polypeptide.

Antibody fragments which contain specific binding sites of a complex may be generated by known techniques. For example, such fragments include but are not limited to: the F(ab')₂ fragments which can be produced by pepsin digestion of the antibody molecule and the Fab fragments which can be generated by reducing the disulfide bridges of the F(ab')₂ fragments. Alternatively, Fab expression libraries may be constructed (Huse et al., 1989, Science, 246:1275-1281) to allow rapid and easy identification of monoclonal Fab fragments with the desired specificity to the PTK/adaptor complex.

One or more components of a protein complex may be present at a higher than normal cellular level (i.e., higher than the concentration known to usually be present in the cell type exhibiting the protein complex of interest) and/or may exhibit an abnormally increased level of cellular activity (i.e., greater than the activity known to usually be present in the cell type exhibiting the protein complex of interest).

For example, the gene encoding a protein complex component may begin to be overexpressed, or may be amplified (i.e., its gene copy number may be increased) in certain cells, leading to an increased number of component molecules within these cells. Additionally, a gene encoding a protein complex component may begin to express a modified protein product that exhibits a greater than normal level of activity. "Activity", here, refers to the normal cellular function of the component, either enzymatic or structural whose function may include, for example, bringing two or more cellular molecules into the appropriate proximity.

Such an increase in the cellular level and/or activity of a protein complex may lead to the development of a disorder. Treatment of such disorders may, therefore, be effectuated by the administration of agents which decrease the cellular level and/or the activity of the overexpressed and/or overactive protein complex component. Techniques for decreasing the cellular level and/or the activity of one or more of the protein complex components of interest may include, but are not limited to antisense or ribozyme approaches, and/or gene therapy approaches, each of which is well known to those of skill in the art.

### XI. Antisense and Ribozyme Approaches

Also described herein are oligoribonucleotides, including antisense RNA and DNA molecules and ribozymes that function to inhibit translation of one or more components of a protein complex. Anti-sense RNA and DNA molecules act to directly block the translation of mRNA by binding to targeted mRNA and preventing protein translation. With respect to antisense DNA, oligodeoxyribonucleotides derived from the translation initiation site, e.g., between -10 and +10 regions of the relevant nucleotide sequence, are preferred.

Ribozymes are enzymatic RNA molecules capable of catalyzing the specific cleavage of RNA. The mechanism of ribozyme action involves sequence specific interaction of the ribozyme molecule to complementary target RNA, followed by a endonucleolytic cleavage. Described herein are engineered hammerhead or other motif ribozyme molecules that specifically and efficiently catalyze endonucleolytic cleavage of RNA sequences encoding protein complex components.

Specific ribozyme cleavage sites within any potential RNA target are initially identified by scanning the target molecule for ribozyme cleavage sites which include the following sequences, GUA, GUU and GUC. Once identified, short RNA sequences of between 15 and 20 ribonucleotides corresponding to the region of the target gene containing the cleavage site may be evaluated for predicted structural features, such as secondary structure, that may render the oligonucleotide sequence unsuitable. The suitability of candidate targets may also be evaluated by testing their accessibility to hybridization with complementary oligonucleotides, using ribonuclease protection assays. See, Draper PCT WO 93/23569.

Both anti-sense RNA and DNA molecules and ribozymes may be prepared by any method known in the art for the synthesis of RNA molecules. See, Draper, id. These include techniques for chemically synthesizing oligodeoxyribonucleotides well known in the art such as for example solid phase phosphoramidite chemical synthesis. Alternatively, RNA molecules may be generated by *in vitro* and *in vivo* transcription of DNA sequences encoding the antisense RNA molecule. Such DNA sequences may be incorporated into a wide variety of vectors which incorporate suitable RNA polymerase promoters such as the T7 or SP6 polymerase promoters. Alternatively, antisense cDNA constructs that synthesize antisense RNA constitutively or inducibly, depending on the promoter used, can be introduced stably into cell lines.

Various modifications to the DNA molecules may be introduced as a means of increasing intracellular stability and half-life. Possible modifications include but are not limited to the addition of flanking sequences of ribo- or deoxy- nucleotides to the 5' and/or 3' ends of the molecule or the use of phosphorothioate or 2' O-methyl rather than phosphodiesterase linkages within the oligodeoxyribonucleotide backbone.

### XII. Gene Therapy

Target cell populations may be modified by introducing altered forms of one or more components of the protein complexes in order to modulate the activity of such complexes. For example, by reducing or inhibiting a complex component activity within target cells, an abnormal signal transduction event(s) leading to a condition may be decreased, inhibited, or reversed. Deletion or missense mutants of a component, that retain the ability to interact with other components of the protein complexes but cannot function in signal transduction may be used to inhibit an abnormal, deleterious signal transduction event.

Expression vectors derived from viruses such as retroviruses, vaccinia virus, adenovirus, adeno-associated virus, herpes viruses, or bovine papilloma virus, may be used for delivery of nucleotide sequences encoding recombinant protein complex components into the targeted cell population. Methods which are well known to those skilled in the art can be used to construct recombinant viral vectors containing coding sequences. See, for example, the techniques described in Maniatis et al., 1989, Molecular Cloning A Laboratory Manual, Cold Spring Harbor Laboratory, N.Y. and Ausubel et al., 1989, Current Protocols in Molecular Biology, Greene Publishing Associates and Wiley Interscience, N.Y. Alternatively, recombinant nucleic acid molecules encoding protein sequences can be used as naked DNA or in reconstituted system e.g., liposomes or other lipid systems for delivery to target cells (See e.g., Felgner et al., 1989, Nature 337:387-8).

### XIII. Pharmaceutical Formulations and Modes of Administration

The particular compound, antibody, antisense or ribozyme molecule that affects the protein complexes and the disorder of interest can be administered to a patient either by themselves, or in pharmaceutical compositions where it is mixed with suitable carriers or excipient(s). In treating a patient exhibiting a disorder of interest, a therapeutically effective amount of a agent or agents such as these is administered. A therapeutically effective dose refers to that amount of the compound that results in amelioration of symptoms or a prolongation of survival in a patient.

Toxicity and therapeutic efficacy of such compounds can be determined by standard pharmaceutical procedures in cell cultures or experimental animals, e.g., for determining the LD₅₀ (the dose lethal to 50% of the population) and the ED₅₀ (the dose therapeutically effective in 50% of the population). The dose ratio between toxic and therapeutic effects is the therapeutic index and it can be expressed as the ratio LD₅₀/ED₅₀. Compounds which exhibit large therapeutic indices are preferred. The data obtained from these cell culture assays and animal studies can be used in formulating a range of dosage for use in human. The dosage of such compounds lies preferably within a range of circulating concentrations that include the ED₅₀ with little or no toxicity. The dosage may vary within this range depending upon the dosage form employed and the route of administration utilized.

For any compound used in the method described herein, the therapeutically effective dose can be estimated initially from cell culture assays. For example, a dose can be formulated in animal models to achieve a circulating plasma concentration range that includes the IC₅₀ as determined in cell culture (i.e., the concentration of the test compound which achieves a half-maximal disruption of the protein complex, or a half-maximal inhibition of the cellular level and/or activity of a complex component). Such information can be used to more accurately determine useful doses in humans. Levels in plasma may be measured, for example, by HPLC. The exact formulation, route of administration and dosage can be chosen by the individual physician in view of the patient's condition. (See e.g. Fingl et al., 1975, in "The Pharmacological Basis of Therapeutics", Ch. 1 p1).

It should be noted that the attending physician would know how to and when to terminate, interrupt, or adjust administration due to toxicity, or to organ dysfunctions. Conversely, the attending physician would also know to adjust treatment to higher levels if the clinical response were not adequate (precluding toxicity). The magnitude of an administrated dose in the management of the oncogenic disorder of interest will vary with the severity of the condition to be treated and to the route of administration. The severity of the condition may, for example, be evaluated, in part, by standard prognostic evaluation methods. Further, the dose and perhaps dose frequency, will also vary according to the age, body weight, and response of the individual patient. A program comparable to that discussed above may be used in veterinary medicine.

Depending on the specific conditions being treated, such agents may be formulated and administered systemically or locally. Techniques for formulation and administration may be found in "Remington's Pharmaceutical Sciences," 1990, 18th ed., Mack Publishing Co., Easton, PA. Suitable routes may include oral, rectal, transdermal, vaginal, transmucosal, or intestinal administration; parenteral delivery, including intramuscular, subcutaneous, intramedullary injections, as well as intrathecal, direct intraventricular, intravenous, intraperitoneal, intranasal, or intraocular injections, just to name a few. For injection, the agents of the invention may be formulated in aqueous solutions, preferably in physiologically compatible buffers such as Hanks's solution, Ringer's solution, or physiological saline buffer. For such transmucosal administration, penetrants appropriate to the barrier to be permeated are used in the formulation. Such penetrants are generally known in the art.

Use of pharmaceutically acceptable carriers to formulate the compounds herein disclosed for the practice of the invention into dosages suitable for systemic administration is within the scope of the invention. With proper choice of carrier and suitable manufacturing practice, the compositions of the present invention, in particular, those formulated as solutions, may be administered parenterally, such as by intravenous injection. The compounds can be formulated readily using pharmaceutically acceptable carriers well known in the art into dosages suitable for oral administration. Such carriers enable the compounds of the invention to be formulated as tablets, pills, capsules, liquids, gels, syrups, slurries, suspensions and the like, for oral ingestion by a patient to be treated.

Agents intended to be administered intracellularly may be administered using techniques well known to those of ordinary skill in the art. For example, such agents may be encapsulated into liposomes, then administered as described above. Liposomes are spherical lipid bilayers with aqueous interiors. All molecules present in an aqueous solution at the time of liposome formation are incorporated into the aqueous interior. The liposomal contents are both protected from the external microenvironment and, because liposomes fuse with cell membranes, are efficiently delivered into the cell cytoplasm. Additionally, due to their hydrophobicity, small organic molecules may be directly administered intracellularly.

Pharmaceutical compositions suitable for use in the present invention include compositions wherein the active ingredients are contained in an effective amount to achieve its intended purpose. Determination of the effective amounts is well within the capability of those skilled in the art, especially in light of the detailed disclosure provided herein. In addition to the active ingredients, these pharmaceutical compositions may contain suitable pharmaceutically acceptable carriers comprising excipients and auxiliaries which facilitate processing of the active compounds into preparations which can be used pharmaceutically. The preparations formulated for oral administration may be in the form of tablets, dragees, capsules, or solutions.

The pharmaceutical compositions of the present invention may be manufactured in a manner that is itself known, e.g., by means of conventional mixing, dissolving, granulating, dragee-making, levigating, emulsifying, encapsulating, entrapping or lyophilizing processes.

Pharmaceutical formulations for parenteral administration include aqueous solutions of the active compounds in water-soluble form. Additionally, suspensions of the active compounds may be prepared as appropriate oily injection suspensions. Suitable lipophilic solvents or vehicles include fatty oils such as sesame oil, or synthetic fatty acid esters, such as ethyl oleate or triglycerides, or liposomes. Aqueous injection suspensions may contain substances which increase the viscosity of the suspension, such as sodium carboxymethyl cellulose, sorbitol, or dextran. Optionally, the suspension may also contain suitable stabilizers or agents which increase the solubility of the compounds to allow for the preparation of highly concentrated solutions.

Pharmaceutical preparations for oral use can be obtained by combining the active compounds with solid excipient, optionally grinding a resulting mixture, and processing the mixture of granules, after adding suitable auxiliaries, if desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethyl-cellulose, sodium carboxymethylcellulose, and/or polyvinylpyrrolidone (PVP). If desired, disintegrating agents may be added, such as the cross-linked polyvinyl pyrrolidone, agar, or alginic acid or a salt thereof such as sodium alginate.

Dragee cores are provided with suitable coatings. For this purpose, concentrated sugar solutions may be used, which may optionally contain gum arabic, talc, polyvinyl pyrrolidone, carbopol gel, polyethylene glycol, and/or titanium dioxide, lacquer solutions, and suitable organic solvents or solvent mixtures. Dyestuffs or pigments may be added to the tablets or dragee coatings for identification or to characterize different combinations of active compound doses.

Pharmaceutical preparations which can be used orally include push-fit capsules made of gelatin, as well as soft, sealed capsules made of gelatin and a plasticizer, such as glycerol or sorbitol. The push-fit capsules can contain the active ingredients in admixture with filler such as lactose, binders such as starches, and/or lubricants such as talc or magnesium stearate and, optionally, stabilizers. In soft capsules, the active compounds may be dissolved or suspended in suitable liquids, such as fatty oils, liquid paraffin, or liquid polyethylene glycols. In addition, stabilizers may be added.

### XIV. Identification of Agents

The complexes, components of such complexes, functional equivalents thereof, and/or cell lines that express such components and exhibit such protein complexes may be used to screen for additional compounds, antibodies, or other molecules capable of modulating the signal transduction event such complexes are involved in. Methods for purifying and/or producing such complexes, components of the complexes, functional equivalents thereof, and/or cell lines are described herein. The compounds, antibodies, or other molecules identified may, for example, act to disrupt the protein complexes of the invention (i.e., decrease or inhibit interactions between component members of the complexes, thereby causing physical separation of the components, and/or perturbing the activity of the complexes) or may lower the cellular level and/or decrease the activity of one or more of the components of such complexes.

Such compounds may include, but are not limited to, peptides made of D- and/or L-configuration amino acids (in, for example, the form of random peptide libraries; see Lam, K.S. et al., 1991, Nature 354:82-84), phosphopeptides (in, for example, the form of random or partially degenerate, directed phosphopeptide libraries, see Songyang, Z. et al., 1993, Cell 767-778), antibodies, and small organic or inorganic molecules. Synthetic compounds, natural products, and other sources of potentially biologically active materials may be screened in a variety of ways, as described herein. The compounds, antibodies, or other molecules identified may be used as oncogenic disorder treatments, as described herein.

Compounds that bind to individual components, or functional portions of the individual components of the complexes (and may additionally be capable of disrupting complex formation) may be identified.

One such method is a method for identifying an agent to be tested for an ability to modulate a signal transduction pathway disorder comprising:
(a) exposing at least one agent to a protein comprising a functional portion of a member of the roteine complex for a time sufficient to allow binding of the agent to the functional portion of the member;
(b) removing non-bound agents; and
(c) determining the presence of the compound bound to the functional portion of the member of the protein complex, thereby identifying an agent to be tested for an ability to modulate a disorder involving a polypeptide complex.

By "signal transduction disorder" is meant any disease or condition associated with an abnormality in a signal transduction pathway. The protein complex referred to below is a physical association of a protein containing a BLM domain and its natural binding partner. The level of interaction between the two components of the complex may be abnormal and thus cause the abnormality in the signal transduction pathway. Alternatively, the level of interaction between the complex components may be normal, but affecting that interaction may effectively treat a signal transduction pathway disorder.

The terms "protein", "polypeptide". and "peptide" refer to a compound formed of 5-50 or more amino acids joined together by peptide bonds. These terms do not carry precise length connotations and as used herein the terms may refer to any amino acid sequence of five or more residues. No implication regarding the relative length of a sequence is intended by the use one term rather than the other. An "amino acid" is a subunit that is polymerized to form proteins and there are twenty amino acids that are universally found in proteins. The general formula for an amino acid is H₂N-CHR-COOH, in which the R group can be anything from a hydrogen atom (as in the amino acid glycine) to a complex ring (as in the amino acid tryptophan).

A functional portion of an individual component of the complexes may be defined here as a protein portion of an individual component of a complex still capable of forming a stable complex with another member of the complex under standard cellular and physiological conditions. For example, a functional portion of a component may include, but is not limited to, a protein portion of the BLM domain which is still capable of stably binding a corresponding natural binding domain of an associated protein, and thus is still capable of forming a complex with that protein. Further, in the case of the catalytic domains of the individual components of the invention, a functional portion of a catalytic domain may refer to a protein still capable of stably binding a substrate molecule under standard physiological conditions.

One method utilizing this approach that may be pursued in the isolation of such complex component-binding molecules would include the attachment of a component molecule, or a functional portion thereof, to a solid matrix, such as agarose or plastic beads, microtiter wells, petri dishes, or membranes composed of, for example, nylon or nitrocellulose, and the subsequent incubation of the attached component molecule in the presence of a potential component-binding compound or compounds. Attachment to said solid support may be direct or by means of a component specific antibody bound directly to the solid support. After incubation, unbound compounds are washed away, component-bound compounds are recovered. By utilizing this procedure, large numbers of types of molecules may be simultaneously screened for complex component-binding activity.

The complex components which may be utilized in the above screening method may include, but are not limited to, molecules or functional portions thereof, such as catalytic domains, phosphorylation domains, extracellular domains, or portions of extracellular domains, such as ligand-binding domains, and adaptor proteins, or functional portions thereof. The peptides used may be phosphorylated, e.g., may contain at least one phosphorylated amino acid residue, preferably a phosphorylated Tyr amino acid residue, or may be unphosphorylated. A phosphorylation domain may be defined as a peptide region that is specifically phosphorylated at certain amino acid residues. A functional portion of such a phosphorylation domain may be defined as a peptide capable of being specifically phosphorylated at certain amino acids by a specific protein.

Molecules exhibiting binding activity may be further screened for an ability to disrupt protein complexes. Alternatively, molecules may be directly screened for an ability to promote the complexes. For example, in vitro complex formation may be assayed by, first, immobilizing one component, or a functional portion thereof, of the complex of interest to a solid support. Second, the immobilized complex component may be exposed to a compound such as one identified as above, and to the second component, or a functional portion thereof, of the complex of interest. Third, it may be determined whether or not the second component is still capable of forming a complex with the immobilized component in the presence of the compound. In addition, one could look for an increase in binding.

Additionally, complex formation in a whole cell may be assayed by utilizing co-immunoprecipitation techniques well known to those of skill in the art. Briefly, a cell line capable of forming a complex of interest may be exposed to a compound such as one identified as above, and a cell lysate may be prepared from this exposed cell line. An antibody raised against one of the components of the complex of interest may be added to the cell lysate, and subjected to standard immunoprecipitation techniques. In cases where a complex is still formed, the immunoprecipitation will precipitate the complex, whereas in cases where the complex has been disrupted, only the complex component to which the antibody is raised will be precipitated.

A preferred method for assessing modulation of complex formation within a cell utilizes a method similar to that described above. Briefly, a cell line capable of forming a complex of interest is exposed to a test compound. The cells are lysed and the lysate contacted with an antibody specific to one component of the complex, said antibody having been previously bound to a solid support. Unbound material is washed away, and the bound material is exposed to a second antibody, said second antibody binding specifically to a second component of the complex. The amount of second antibody bound is easily detected by techniques well known in the art. Cells exposed to an inhibitory test compound will have formed a lesser amount of complex compared to cells not exposed to the test compound, as measured by the amount of second antibody bound. Cells exposed to a test compound that promotes complex formation will have an increased amount of second antibody bound.

The effect of an agent on the transformation capability of the complex of interest may be directly assayed. Such agents may, but are not required to, include those agents identified by utilizing the above screening technique. For example, an agent or agents may be administered to a cell such as a breast cancer cell, capable of forming a complex, for example, which, in the absence of any inhibitory agent, would lead to the cell's transformation. The transformation state of the cell may then be measured in vitro, by monitoring, for example, its ability to form colonies in soft agar. Alternatively, a cell's transformation state may be monitored in vivo by, for example, determining its ability to form tumors in immunodeficient nude or severe combined immunodeficiency (SCID) mice.

Agents capable of disrupting complex formation and capable of reducing or inhibiting disorders, such as breast cancer, which involve the formation of such complexes may be used in the treatment of patients exhibiting or at risk for such disorders. A sufficient amount of agent or agents such as those described above may be administered to a patient so that the oncogenic capability of cells which, in the absence of such agents, would contain protein complexes, is reduced or eliminated.

### XV. Purification and Production of Complexes

Described in this Section are methods for the synthesis or recombinant expression of components, or fragments thereof, of the protein complexes of the invention. Also described herein are methods by which cells exhibiting the protein complexes of the invention may be engineered.

### XVI. Purification Methods

The complexes of the invention may be substantially purified, i.e., may be purified away from at least 90% (on a weight basis), and from at least 99%, if desired, of other proteins, glycoproteins, and other macromolecules with which it is associated. Such purification can be achieved by utilizing a variety of procedures well known to those of skill in the art, such as subjecting cells, tissue or fluid containing the complex to a combination of standard methods, for example, ammonium sulfate precipitation, molecular sieve chromatography, and/or ion exchange chromatography.

Alternatively, or additionally, a complex may be purified by immunoaffinity chromatography using an immuno-adsorbent column to which an antibody is immobilized which is capable of binding to one or more components of the complex. Such an antibody may be monoclonal or polyclonal in origin. Other useful types of affinity purification for the protein complex may utilize, for example, a solid-phase substrate which binds the catalytic kinase domain of a protein, or an immobilized binding site for noncatalytic domains of the components of the complex, which bind in such a manner as to not disrupt the complex.

The complex of the present invention may be biochemically purified from a variety of cell or tissue sources. For purification of a naturally occurring complex, cellular sources may include, for example, baculovirus-infected SF9 cells, A-431, CHO, and/or 3T3 cells. Described herein is a complex which comprises the receptor GRB-7 or GRB-10 adaptor protein.

### XVII. Synthesis and Expression Methods

Methods for the synthesis of polypeptides or fragments thereof, which are capable of acting as components of the complexes of the present invention, are well-known to those of ordinary skill in the art. See, for example, Creighton, 1983, Proteins: Structures and Molecular Principles, W.H. Freeman and Co., NY.

Components of a complex which have been separately synthesized or recombinantly produced, may be reconstituted to form a complex by standard biochemical techniques well known to those skilled in the art. For example, samples containing the components of the complex may be combined in a solution buffered with greater than about 150mM NaCl, at a physiological pH in the range of 7, at room temperature. For example, a buffer comprising 20mM Tris-HCl, pH 7.4, 137mMNaCl, 10% glycerol, 1% Triton X-100, 0.1% SDS, 0.5% deoxycholate and 2mM EDTA could be used.

Methods for preparing the components of complexes of the invention by expressing nucleic acid encoding proteins are described herein. Methods which are well known to those skilled in the art can be used to construct expression vectors containing protein coding sequences and appropriate transcriptional/translational control signals. These methods include, for example, in vitro recombinant DNA techniques, synthetic techniques and in vivo recombination/genetic recombination. DNA and RNA synthesis may, additionally, be performed using an automated synthesizers. See, for example, the techniques described in Maniatis et al., 1989, Molecular Cloning A Laboratory Manual, Cold Spring Harbor Laboratory, N.Y. and Ausubel et al., 1989, Current Protocols in Molecular Biology, Greene Publishing Associates and Wiley Interscience, N.Y.

A variety of host-expression vector systems may be utilized to express the coding sequences of the components of the complexes of the invention. Such host-expression systems represent vehicles by which the coding sequences of interest may be produced, but also represent cells which may, when transformed or transfected with the appropriate nucleotide coding sequences, exhibit the protein complexes of the invention. These include but are not limited to microorganisms such as bacteria (e.g., E.coli, B. subtilis) transformed with recombinant bacteriophage DNA, plasmid DNA or cosmid DNA expression vectors containing protein coding sequences; yeast (e.g., Saccharomyces and Pichia) transformed with recombinant yeast expression vectors containing the protein coding sequences; insect cell systems infected with recombinant virus expression vectors (e.g., baculovirus) containing the protein coding sequences; plant cell systems infected with recombinant virus expression vectors (e.g., cauliflower mosaic virus, CaMV; tobacco mosaic virus, TMV) or transformed with recombinant plasmid expression vectors (e.g., Ti plasmid) containing the protein coding sequences coding sequence; or mammalian cell systems (e.g., COS, CHO, BHK, 293, 3T3) harboring recombinant expression constructs containing promoters derived from the genome of mammalian cells (e.g., metallothionein promoter) or from mammalian viruses (e.g., the adenovirus late promoter; the vaccinia virus 7.5K promoter).

In bacterial systems a number of expression vectors may be advantageously selected depending upon the use intended for the complex being expressed. For example, when large quantities of complex proteins are to be produced for the generation of antibodies or to screen peptide libraries, vectors which direct the expression of high levels of fusion protein products that are readily purified may be desirable. Such vectors include but are not limited to the E. coli expression vector pUR278 (Ruther et al., 1983, EMBO J. 2:1791), in which the protein coding sequence may be ligated individually into the vector in frame with the lac Z coding region so that a fusion protein is produced; pIN vectors (Inouye & Inouye, 1985, Nucleic acids Res. 13:3101-3109; Van Heeke & Schuster, 1989, J. Biol. Chem. 264:5503-5509); and the like. pGEX vectors may also be used to express foreign polypeptides as fusion proteins with glutathione S-transferase (GST). In general, such fusion proteins are soluble and can easily be purified from lysed cells by adsorption to glutathione-agarose beads followed by elution in the presence of free glutathione. The pGEX vectors are designed to include thrombin or factor Xa protease cleavage sites so that the cloned protein can be released from the GST moiety.

In an insect system, Autographa californica nuclear polyhedrosis virus (AcNPV) is used as a vector to express foreign genes. The virus grows in Spodoptera frugiperda cells. The complex coding sequence may be cloned individually into non-essential regions (for example the polyhedrin gene) of the virus and placed under control of an AcNPV promoter (for example the polyhedrin promoter). Successful insertion of the PTK/adaptor complex coding sequence will result in inactivation of the polyhedrin gene and production of non-occluded recombinant virus (i.e., virus lacking the proteinaceous coat coded for by the polyhedrin gene). These recombinant viruses are then used to infect Spodoptera frugiperda cells in which the inserted gene is expressed (e.g., see Smith et al., 1983, J. Viol. 46:584; Smith, U.S. Patent No. 4,215,051).

In mammalian host cells, a number of viral based expression systems may be utilized. In cases where an adenovirus is used as an expression vector, the complex coding sequence may be ligated to an adenovirus transcription/translation control complex, e.g., the late promoter and tripartite leader sequence. This chimeric gene may then be inserted in the adenovirus genome by in vitro or in vivo recombination. Insertion in a non-essential region of the viral genome (e.g., region E1 or E3) will result in a recombinant virus that is viable and capable of expressing proteins in infected hosts. (E.g., See Logan & Shenk, 1984, Proc. Natl. Acad. Sci. USA 81:3655-3659). Specific initiation signals may also be required for efficient translation of inserted coding sequences. These signals include the ATG initiation codon and adjacent sequences.

In cases where an entire protein gene, including its own initiation codon and adjacent sequences, is inserted into the appropriate expression vector, no additional translational control signals may be needed. However, in cases where only a portion of the coding sequence is inserted, exogenous translational control signals, including the ATG initiation codon, must be provided. Furthermore, the initiation codon must be in phase with the reading frame of the desired coding sequence to ensure translation of the entire insert. These exogenous translational control signals and initiation codons can be of a variety of origins, both natural and synthetic. The efficiency of expression may be enhanced by the inclusion of appropriate transcription enhancer elements, transcription terminators, etc. (see Bittner et al., 1987, Methods in Enzymol. 153:516-544).

In addition, a host cell strain may be chosen which modulates the expression of the inserted sequences, or modifies and processes the gene product in the specific fashion desired. Such modifications (e.g., glycosylation) and processing (e.g., cleavage) of protein products may be important for the function of the protein. Different host cells have characteristic and specific mechanisms for the post-translational processing and modification of proteins. Appropriate cells lines or host systems can be chosen to ensure the correct modification and processing of the foreign protein expressed. To this end, eukaryotic host cells which possess the cellular machinery for proper processing of the primary transcript, glycosylation, and phosphorylation of the gene product may be used. Such mammalian host cells include but are not limited to CHO, VERO, BHK, HeLa, COS, MDCK, 293, 3T3, WI38, etc.

For long-term, high-yield production of recombinant proteins, stable expression is preferred. For example, cell lines which stably coexpress both the proteins may be engineered. Rather than using expression vectors which contain viral origins of replication, host cells can be transformed with the protein encoding DNA independently or coordinately controlled by appropriate expression control elements (e.g., promoter, enhancer, sequences, transcription terminators, polyadenylation sites, etc.), and a selectable marker.

Following the introduction of foreign DNA, engineered cells may be allowed to grow for 1-2 days in an enriched media, and then are switched to a selective media. The selectable marker in the recombinant plasmid confers resistance to the selection and allows cells to stably integrate the plasmid into their chromosomes and grow to form foci which in turn can be cloned and expanded into cell lines. This method may advantageously be used to engineer cell lines which coexpress both the PTK and adaptor protein. Such engineered cell lines are particularly useful in screening and evaluation of compounds that affect signals mediated by the complexes.

A number of selection systems may be used, including but not limited to the herpes simplex virus thymidine kinase (Wigler, et al., 1977, Cell 11:223), hypoxanthine-guanine phosphoribosyltransferase (Szybalska & Szybalski, 1962, Proc. Natl. Acad. Sci. USA 48:2026), and adenine phosphoribosyltransferase (Lowy, et al., 1980, Cell 22:817) genes can be employed in tk⁻, hgprt⁻ or aprt⁻cells, respectively. Also, antimetabolite resistance can be used as the basis of selection for dhfr, which confers resistance to methotrexate (Wigler, et al., 1980, Natl. Acad. Sci. USA 77:3567; O'Hare, et al., 1981, Proc. Natl. Acad. Sci. USA 78:1527); gpt, which confers resistance to mycophenolic acid (Mulligan & Berg, 1981), Proc. Natl. Acad. Sci. USA 78:2072); neo, which confers resistance to the aminoglycoside G-418 (Colberre-Garapin, et al., 1981, J. Mol. Biol. 150:1); and hygro, which confers resistance to hygromycin (Santerre, et al., 1984, Gene 30:147) genes.

New members of the protein families capable of forming the complexes of the invention may be identified and isolated by molecular biological techniques well known in the art. For example, a previously unknown protein encoding gene may be isolated by performing a polymerase chain reaction (PCR) using two degenerate oligonucleotide primer pools designed on the basis of highly conserved sequences within domains common to members of the protein family.

The template for the reaction may be cDNA obtained by reverse transcription of mRNA prepared from cell lines or tissue known to express complexes. The PCR product may be subcloned and sequenced to insure that the amplified sequences represent the sequences of a member of the PTK or adaptor subfamily. The PCR fragment may then be used to isolate a full length protein cDNA clone by radioactively labeling the amplified fragment and screening a bacteriophage cDNA library. Alternatively, the labeled fragment may be used to screen a genomic library. For a review of cloning strategies which may be used, see e.g., Maniatis, 1989, Molecular Cloning, A Laboratory Manual, Cold Springs Harbor Press, N.Y.; and Ausubel et al., 1989, Current Protocols in Molecular Biology, (Green Publishing Associates and Wiley Interscience, N.Y.).

A general method for cloning previously unknown proteins has been described by Skolnik (Skolnik, E.Y., 1991, Cell 65:75) and Skolnik et al., (U.S. Patent Application Serial No. 07/643,237). Briefly, new members of the family of proteins may be identified by their ability to specifically bind to at least a portion of a BLM or EGF domain in a peptide.

### XVIII. Derivatives of Complexes

Also provided herein are functional derivatives of a complex. By "functional derivative" is meant a "chemical derivative," "fragment," "variant," "chimera," or "hybrid" of the complex, which terms are defined below. A functional derivative retains at least a portion of the function of the protein, for example reactivity with an antibody specific for the complex, enzymatic activity or binding activity mediated through noncatalytic domains, which permits its utility in accordance with the present invention.

A "chemical derivative" of the complex contains additional chemical moieties not normally a part of the protein. Covalent modifications of the protein complex or peptides are included within the scope of this invention. Such modifications may be introduced into the molecule by reacting targeted amino acid residues of the peptide with an organic derivatizing agent that is capable of reacting with selected side chains or terminal residues, as described below.

Cysteinyl residues most commonly are reacted with alpha-haloacetates (and corresponding amines), such as chloroacetic acid or chloroacetamide, to give carboxymethyl or carboxyamidomethyl derivatives. Cysteinyl residues also are derivatized by reaction with bromotrifluoroacetone, α-bromo-β(5-imidozoyl)propionic acid, chloroacetyl phosphate, N-alkylmaleimides, 3-nitro-2-pyridyl disulfide, methyl 2-pyridyl disulfide, p-chloromercuribenzoate, 2-chloromercuri-4-nitrophenol, or chloro-7-nitrobenzo-2-oxa-1,3-diazole.

Histidyl residues are derivatized by reaction with diethylprocarbonate at pH 5.5-7.0 because this agent is relatively specific for the histidyl side chain. Parabromophenacyl bromide also is useful; the reaction is preferably performed in 0.1 M sodium cacodylate at pH 6.0.

Lysinyl and amino terminal residues are reacted with succinic or other carboxylic acid anhydrides. Derivatization with these agents has the effect or reversing the charge of the lysinyl residues. Other suitable reagents for derivatizing α-amino-containing residues include imidoesters such as methyl picolinimidate; pyridoxal phosphate; pyridoxal; chloroborohydride; trinitrobenzenesulfonic acid; O-methylisourea; 2,4 pentanedione; and transaminase-catalyzed reaction with glyoxylate.

Arginyl residues are modified by reaction with one or several conventional reagents, among them phenylglyoxal, 2,3-butanedione, 1,2-cyclohexanedione, and ninhydrin. Derivatization of arginine residues requires that the reaction be performed in alkaline conditions because of the high pKₐ of the guanidine functional group. Furthermore, these reagents may react with the groups of lysine as well as the arginine ε-amino group.

Tyrosyl residues are well-known targets of modification for introduction of spectral labels by reaction with aromatic diazonium compounds or tetranitromethane. Most commonly, N-acetylimidizol and tetranitromethane are used to form O-acetyl tyrosyl species and 3-nitro derivatives, respectively.

Carboxyl side groups (aspartyl or glutamyl) are selectively modified by reaction carbodiimide (R'-N-C-N-R') such as 1-cyclohexyl-3-(2-morpholinyl(4-ethyl) carbodiimide or 1-ethyl-3-(4-azonia-4,4-dimethylpentyl) carbodiimide. Furthermore, aspartyl and glutamyl residue are converted to asparaginyl and glutaminyl residues by reaction with ammonium ions.

Glutaminyl and asparaginyl residues are frequently deamidated to the corresponding glutamyl and aspartyl residues. Alternatively, these residues are deamidated under mildly acidic conditions. Either form of these residues falls within the scope of this invention.

Derivatization with bifunctional agents is useful, for example, for cross-linking the component peptides of the complexes to each other or the complex to a water-insoluble support matrix or to other macromolecular carriers. Commonly used cross-linking agents include, for example, 1,1-bis(diazoacetyl)-2-phenylethane, glutaraldehyde, N-hydroxysuccinimide esters, for example, esters with 4-azidosalicylic acid, homobifunctional imidoesters, including disuccinimidyl esters such as 3,3'-dithiobis(succinimidylpropionate), and bifunctional maleimides such as bis-N-maleimido-1,8-octane. Derivatizing agents such as methyl-3-[p-azidophenyl) dithiolpropioimidate yield photoactivatable intermediates that are capable of forming crosslinks in the presence of light. Alternatively, reactive water-insoluble matrices such as cyanogen bromide-activated carbohydrates and the reactive substrates described in U.S. Patent Nos. 3,969,287; 3,691,016; 4,195,128; 4,247,642; 4,229,537; and 4,330,440 are employed for protein immobilization.

Other modifications include hydroxylation of proline and lysine, phosphorylation of hydroxyl groups of seryl or threonyl residues, methylation of the alpha-amino groups of lysine, arginine, and histidine side chains (Creighton, T.E., Proteins: Structure and Molecular Properties, W.H. Freeman & Co., San Francisco, pp. 79-86 (1983)), acetylation of the Nterminal amine, and, in some instances, amidation of the C-terminal carboxyl groups.

Such derivatized moieties may improve the stability, solubility, absorption, biological half life, and the like. The moieties may alternatively eliminate or attenuate any undesirable side effect of the protein complex and the like. Moieties capable of mediating such effects are disclosed, for example, in Remington's Pharmaceutical Sciences, 18th ed., Mack Publishing Co., Easton, PA (1990).

The term "fragment" is used to indicate a polypeptide derived from the amino acid sequence of the proteins, of the complexes having a length less than the full-length polypeptide from which it has been derived. Such a fragment may, for example, be produced by proteolytic cleavage of the full-length protein. Preferably, the fragment is obtained recombinantly by appropriately modifying the DNA sequence encoding the proteins to delete one or more amino acids at one or more sites of the C-terminus, N-terminus, and/or within the native sequence.

Fragments of a protein, when present in a complex resembling the naturally occurring complex, are useful for screening for compounds that act to modulate signal transduction, as described below. It is understood that such fragments, when present in a complex may retain one or more characterizing portions of the native complex. Examples of such retained characteristics include: catalytic activity; substrate specificity; interaction with other molecules in the intact cell; regulatory functions; or binding with an antibody specific for the native complex, or an epitope thereof.

Another functional derivative intended to be within the scope of the present invention is a complex comprising at least one "variant" polypeptide which either lack one or more amino acids or contain additional or substituted amino acids relative to the native polypeptide. The variant may be derived from a naturally occurring complex component by appropriately modifying the protein DNA coding sequence to add, remove, and/or to modify codons for one or more amino acids at one or more sites of the C-terminus, N-terminus, and/or within the native sequence. It is understood that such variants having added, substituted and/or additional amino acids retain one or more characterizing portions of the native complex, as described above. A functional derivative of complexes comprising proteins with deleted, inserted and/or substituted amino acid residues may be prepared using standard techniques well-known to those of ordinary skill in the art. For example, the modified components of the functional derivatives may be produced using site-directed mutagenesis techniques (as exemplified by Adelman et al., 1983, DNA 2:183) wherein nucleotides in the DNA coding the sequence are modified such that a modified coding sequence is modified, and thereafter expressing this recombinant DNA in a prokaryotic or eukaryotic host cell, using techniques such as those described above. Alternatively, components of functional derivatives of complexes with amino acid deletions, insertions and/or substitutions may be conveniently prepared by direct chemical synthesis, using methods well-known in the art. The functional derivatives of the complexes typically exhibit the same qualitative biological activity as the native complexes.

### XIX. Evaluation of Disorders

The protein complexes of the invention involved in disorders may be utilized in developing a prognostic evaluation of the condition of a patient suspected of exhibiting such a disorder. For example, biological samples obtained from patients suspected of exhibiting a disorder involving a protein complex may be assayed for the presence of such complexes. If such a protein complex is normally present, and the development of the disorder is caused by an abnormal quantity of the complex, the assay should compare complex levels in the biological sample to the range expected in normal tissue of the same cell type.

Among the assays which may be undertaken may include, but are not limited to isolation of the protein complex of interest from the biological sample, or assaying for the presence of the complex by exposing the sample to an antibody specific for the complex, but non-reactive to any single, non-complexed component, and detecting whether antibody has specifically bound.

Alternatively, one or more of the components of the protein complex may be present in an abnormal level or in a modified form, relative to the level or form expected is normal, nononcogenic tissue of the same cell type. It is possible that overexpression of both components may indicate a particularly aggressive disorder. Thus, an assessment of the individual and levels of mRNA and protein in diseased tissue cells may provide valuable clues as to the course of action to be undertaken in treatment of such a disorder. Assays of this type are well known to those of skill in the art, and may include, but are not limited to, Northern blot analysis, RNAse protection assays, and PCR for determining mRNA levels. Assays determining protein levels are also well known to those of skill in the art, and may include, but are not limited to, Western blot analysis, immunoprecipitation, and ELISA analysis. Each of these techniques may also reveal potential differences in the form (e.g., the primary, secondary, or tertiary amino acid sequence, and/or post-translational modifications of the sequence) of the component(s).

### Disorders

HER2 driven disorders are characterized by over-activity of HER2. Over-activity of HER2 refers to either an amplification of the gene encoding HER2 or the production of a level of HER2 activity which can be correlated with a cell proliferative disorder (i.e., as the level of HER2 increases the severity of one or more of the symptoms of the cell proliferative disorder increases).

Activation of HER2 activity can result from several different events including: 1) ligand binding; 2) HER2 stimulation through transphosphorylation by activated EGFR; and 3) overexpression of *her*-2, encoding HER2 protein, can create a high abundance of HER2 resulting in ligand-independent dimerization. HER2 activity can be assayed by measuring one or more of the following activities: (1) phosphorylation of HER2; (2) phosphorylation of a HER2 substrate (e.g., IP₃ kinase, and PI 4-kinase, see Scott et al., Journal of Biological 22:14300, 1991); (3) activation of an HER2 adapter molecule; and (4) increased cell division. These activities can be measured using techniques described below and known in the art. For example, IP₃ kinase, and PI 4-kinase, activities can be assayed as described by Scott *et al., supra,* cell division can be assayed by measuring ³H-thymidine incorporation into DNA, and phosphorylation of HER2 can be assayed as described by Gazit et al., J. Med. Chem. 34:1896-1907, 1991, or as described in examples detailed below.

Treatment of patients suffering from a HER2 disorder is facilitated by first determining whether the cell proliferative disorder is characterized by an over-activity of HER2. After the disorder is identified, patients suffering from such a disorder can be identified by analysis of their symptoms by procedures well known to medical doctors. Such identified patients can then be treated as described herein.

HER2 driven disorders are typically cell proliferative disorders such as cancers. HER2 driven disorders appear to be responsible for a sub-population of different types of cancers. For example, as noted above, Slamon *et al*., found about 30% of breast cancer cells to have increased HER2 gene expression. Slamon *et al.,* also found a correlation between her2 (*c-erb*B-2) amplification and poor patient prognosis.

The peptides and compositions of the present invention which can be used to treat breast cancer are preferred because of the prevalence and severity of breast cancer. Carcinoma of the breast is the most common cancer among women and their second leading cause of cancer death (Marshall, E., Science 259:618-62.1, 1993). The incidence of breast cancer has been increasing over the past several decades (Marshall, *supra*, and Harris, J.R., et al, New Engl. J . Med., 327(5):319-328, 1992).

In addition to breast cancers, increased HER2 activity or gene expression has been associated with certain types of stomach adenocarcinomas, salivary gland adenocarcinomas, endometrial cancers, ovarian adenocarcinomas, gastric cancers, colorectal cancers, non-small cell lung cancer, and glioblastomas. The methods described herein can be used to identify the subpopulations of these different cancers which are characterized by over-activity of HER2. A cancer cell refers to various types of malignant neoplasms, most of which can invade surrounding tissues, and may metastasize to different sites, as defined by Stedman's Medical Dictionary 25th edition (Hensyl ed. 1990).

Some of the featured compounds can be used to treat cell proliferative disorders characterized by inappropriate EGFR activity. "Inappropriate EGFR" activity refers to either 1) EGF-receptor (EGFR) expression in cells which normally do not express EGFR; 2) EGF expression by cells which normally do not express EGF; 3) increased EGF-receptor (EGFR) expression leading to unwanted cell proliferation; 4) increased EGF expression leading to unwanted cell proliferation; and/or 5) mutations leading to constitutive activation of EGF-receptor (EGFR). The existence of inappropriate or abnormal EGF and EGFR levels or activities is determined by procedures well known in the art.

An increase in EGF activity or expression is characterized by an increase in one or more of the activities which can occur upon EGF ligand binding such as: (1) auto-phosphorylation of EGFR, (2) phosphorylation of an EGFR substrate (e.g., PLCγ, see Fry *supra),* (3) activation of an adapter molecule, and/or (4) increased cell division. These activities can be measured using techniques described below and known in the art. For example auto-phosphorylation of EGFR can be measured as described in the examples below using an anti-phosphotyrosine antibody, and increased cell division can be performed by measuring ³H-thymidine incorporation into DNA. Preferably, the increase in EGFR activity is characterized by an increased amount of phosphorylated EGFR and/or DNA synthesis.

Unwanted cell proliferation can result from inappropriate EGFR activity occurring in different types of cells including cancer cells, cells surrounding a cancer cell, and endothelial cells. Examples of disorders characterized by inappropriate EGF activity include cancers such as glioma, head, neck, gastric, lung, breast, ovarian, colon, and prostate; and other types of cell proliferative disorders such as psoriasis.

Psoriasis is a common, chronic disease of the skin consisting of erythematous papules which coalesce to form plaques with distinct borders. As the disease progresses and if it is untreated, a silvery, yellow-white scale develops. New lesions tend to appear at sites of trauma; they may be in any location, but frequently are located on the scalp, knees, elbows, umbilicus, and genitalia. The clinical course is variable but less than one-half of the patients followed for a prolonged period will have prolonged remissions. Severity may range from a minimal cosmetic problem to a life-threatening emergency. In about 5% of cases arthritis will develop and, in most of these, joint involvement will occur after the onset of the skin lesions. The course of the arthritis is mild, affects only a few joints, and spontaneous remissions occur. Most of the skin changes, with the exception of inflammation, may be explained by the rapid turnover of the epidermis. Normal skin produces about 1250 cells a day for each square cm, and these come from 27,000 cells; psoriatic skin produces 35,000 new cells each day for each square cm, and these come from 52,000 cells. The normal duration of the cell cycle of skin is 311 hours, but is reduced to 36 hours for psoriatic skin.

### EXAMPLES

The examples below are non-limiting and are merely representative of various aspects and features referred to herein. The examples below demonstrate the mechanism of EGF receptor eimerization, using various methods, including isothermal titration calorimetry.

We describe here studies of the stoichiometry of growth factor binding to this receptor, and compare its characteristics using isothermal titration calorimetry (ITC). This technique gives information not only concerning the binding constant of the interaction, but also provides a direct measurement of the enthalpy (ΔH) and stoichiometry of binding. We have generated milligram quantities of the extracellular ligand-binding domains of the EGF receptor in CHO cells or insect cells for biophysical studies (Lax et al., J. Biol. Chem. 266:13828-13833, 1991; Hurwitz et al., J. Biol. Chem. 266:22035-22043, 1991.

### Production of soluble forms of the EGF-receptor extracellular domain

The various forms of sEGFR were expressed in CHO cells which were transfected with a CVN expression vector containing the cDNA for the desired fragment of EGFR. Generation of the construct encoding sEGFR was previously described (Lax et al., J. Biol. Chem. 266:13828-13833, 1991). The construct encoding sEGFR(123) was generated by introducing a stop codon into this construct at the position corresponding to residue 506 of EGFR.

Purification of sEGFR and sEGFR(123) was performed by immunoaffinity chromatography using the mAb108 antibody, as described (Hurwitz et al., J. Biol. Chem. 266:22035-22043, 1991). After affinity purification, sEGFR was concentrated and loaded onto a Superose 6 (Pharmacia) gel-filtration column. The purity of the resulting preparations was estimated by Coomassie Blue-staining of overloaded SDS-PAGE gels to be better than 95%. sEGFR contains residues 1-624 of the mature receptor, while sEGFR(123) contains residues 1-505.

Domain 3 of sEGFR was prepared by limited proteolysis of sEGFR with proteinase K, with subsequent purification of the fragment as previously described (Kohda et al., J. Biol. Chem. 268:1976-1981, 1993). The resulting approximately 35 kDa (glycosylated) fragment includes residues 302-505 of EGFR, including the complete subdomain 3 as originally defined by Lax et al. (Mol. Cell. Biol. 8:1831-1834, 1988), which encompasses residues 310-474.

hEGF was purchased from Intergen, and used without further purification. Analytical gel-filtration and SDS-PAGE suggested that this is also better than 95% pure. Mouse EGF (K21) was a gift from Dr. E. Nice, Ludwig Institute, Melbourne, Australia.

Determination of the concentration of EGF and the various forms of sEGFR was achieved by measurement of OD at 278 nM. For initial titrations, the extinction coefficient was estimated from the number of tryptophan and tyrosine residues in the respective proteins. Quantitative amino-acid analysis of several samples with known OD₂₇₈ was then performed to determine the correct extinction coefficient. ε₂₇₈ for hEGF was 14400 M⁻¹ cm⁻¹, ε₂₇₈ for mouse EGF (K21) was 6000 M⁻¹ cm⁻¹, ε₂₇₈ for sEGFR was 58500 M⁻¹ cm⁻¹, ε₂₇₈ for sEGFR(123) was 46000 M⁻¹ cm⁻¹, and ε₂₇₈ for sEGFR (3) was 16400 M⁻¹ cm⁻¹.

### Chemical cross-linking experiments

Studies of the ability of EGF (20 µM) to enhance covalent cross-linking of the various forms of sEGFR (10 µM) were performed using the cross-linking reagent disuccinimidyl suberate (DSS), as previously described (Lax et al., J. Biol. Chem. 266:13828-13833, 1991b). The reaction products were analyzed by SDS-PAGE and stained with Coomassie Blue.

### Binding studies employing isothermal titration calorimetry (ITC)

All experiments were performed using the OMEGA instrument from MicroCal Inc., Northampton, MA, as described in detail by Wiseman et al. (Anal. Biochem. 179:131-137, 1989). In a typical titration, EGF was added over sixteen injections (of volume 15 µl) to the portion of the EGF-receptor extracellular domain under study, which was present in the isothermal calorimeter cell at 25 °C at the concentration given in Tables 1 and 2. In every titration, the concentrations of reactants were sufficient to result in saturation of all potential binding sites, as evidenced by the tendency of the heat-change per injection to zero.

Heats of dilution for both reactants (growth-factor and receptor) with buffer solution were measured by titration of buffer into a solution of the receptor extracellular domain, as well as of growth factor into protein-free buffer solution. The total measured heats were corrected for these heats of dilution prior to data analysis. Given the unusual nature of the titrations observed in these studies, we paid particular attention to the determination of the heats of dilution of the reactants. The heats per injection were equal for any given dilution experiment.

We also established that the time interval between injections was sufficient to allow equilibrium to be reached for all the reported titrations. For each titration, both reactants were dialyzed exhaustively (in the same vessel) against the buffer used for the titration (10 mM HEPES, pH 7.4, 100 mM NaCl, 3.4 mM EDTA).

Titration curves were fit using the ORIGIN software (MicroCal Inc., Northampton, MA.), to give the parameters quoted in Table 1 fits to the titration data, yielding reliable estimates of K_{B}, can be obtained for c-values between about 1.0 and approximately 200 (Wiseman et al., Anal. Biochem. 179:131-137, 1989). This presented a problem for ITC analysis of the EGF/sEGFR interaction, since two events occur which differ in their K_{B} values by about one order of magnitude, and the associated enthalpies are relatively small.

C-values in the case of EGF/sEGFR(1234) titrations are in the range of 15 - 52 for the second binding event (K_{B2}), which is ideal, but are in the region of 300 - 1000 for the first (high-affinity) event (K_{B1}). This is reflected in the large errors associated with the K_{B1} values in Table 2, whereas the errors associated with K_{B2} are reasonable. Experiments performed under conditions where c for the high-affinity event was less than 100 gave titrations that could not be fit adequately. Therefore, values for the high-affinity binding constants reported here should be regarded as approximate K_{B} values. It should be noted that these considerations do not affect the ability to fit for ΔH and stoichiometry values.

### Example 1: Two Different Modes for EGF-Binding to sEGFR

To compare the characteristics of EGF binding to its receptor with those observed for aFGF binding to sFGFR, recombinant human EGF was titrated into a solution of sEGFR(1234), which includes the entire extracellular domain of the receptor (residues 1-624; with all four proposed subdomains present; Lax et al., Cell Regulation 2:337-345, 1991; Hurwitz et al., J. Biol. Chem. 266:22035-22043, 1991; Lax et al., EMBO J. 8:421-427, 1989). These titrations showed unexpected characteristics.

The overall stoichiometry of EGF binding was determined to be 1:1, in agreement with previous studies (Weber et al., J. Biol. Chem. 259:14631-14636, 1984; Günther et al., J. Biol. Chem. 265:22082-22085, 1990). However, the shape of the titration curves clearly shows that more than one binding event occurs during the titration. The titration curves obtained for EGF binding to sEGFR could not be fit satisfactorily by assuming a single class of binding site. The best fits to the data in this case were obtained by assuming that two types of binding site exist on the receptor, each with a different affinity for EGF. There is a great deal of evidence for the existence of two different classes of EGF-binding sites presented by the EGF-receptor on the cell-surface.

This heterogeneity gives rise to the curved Scatchard plots that have been observed in studies of EGF to cells or isolated EGFR (Bôni-Schnetzler and Pilch, Proc. Natl. Acad. Sci. USA 84:7832-7836, 1987; Yarden and Schlessinger, Biochemistry 26:1434-1442, 1987; Wofsy et al., Biophys. J. 63:98-110, 1992). In the context of the receptor-dimerization model for transmembrane signaling, it is thought that the high-affinity form of the receptor represents its active, dimeric form (Yarden and Schlessinger, Biochemistry 26:1434-1442, 1987; Yarden and Schlessinger, Biochemistry 26:1443-1451, 1987. A number of recent studies provide strong support for this suggestion (Zhou et al., Biochemistry 32:8193-8198, 1993; Sorokin et al., J. Biol. Chem. 269:9752-9759, 1994).

The nature of the two different modes of binding of EGF to sEGFR can be understood by reference to Table 2. The best fits to the binding data suggest that a high-affinity (K_{D1} ≈ 10 nM) binding event, with a moderately positive value for ΔH, (site 1) is followed in the titration by a second, lower affinity (K_{D2} ≈ 200 nM), event with a higher positive value for ΔH (site 2). The binding isotherms thus appear to be the result of two equilibrium binding events.

Titration data was obtained for EGF binding to sEGFR, and for the binding of EGF to sEGFR. In these titrations, the best fits were obtained using a model based upon two independent classes of binding site. The thermodynamic parameters in each case are given in Tables 2 and 3. Titration of 16 x 15 µl aliquots of recombinant human EGF (85 µM) into sEGFR (13 µM) at 25°C was performed and titration of 16 x 15 µl aliquots of recombinant truncated (K21) mouse EGF (394 µM) into sEGFR (28 µM) at 25°C was also performed. In addition, titration of 20 x 10 µl aliquots of sEGFR (160 µM) into human EGF (17 µM) at 25°C was performed.

Upon injection of the first aliquots of EGF into the sEGFR solution, the high-affinity reaction will predominate (binding to site 1). This has the smaller (positive) associated enthalpy value (+7 kcal/mol). During subsequent injections these high-affinity sites begin to saturate, and the second equilibrium reaction begins (binding to site 2). EGF that is injected into the calorimeter cell at this stage will partition between the two sites, since their affinities differ by only an order of magnitude. As the titration progresses, an increasing proportion of the injected EGF will bind to the lower affinity site (site 2), which has a larger (positive) enthalpy value (+10 kcal/mol). Therefore, the heat absorbed per injection will increase accordingly.

The heat per injection will reach its maximum value when the first site is completely saturated, and all EGF that is injected binds to site 2 (which has the highest positive enthalpy). Thereafter, the heat absorbed per injection will fall as site 2 begins to becomes saturated. Finally, the heat absorbed tends to zero when no further detectable ligand binding occurs. Titrations exhibiting this behavior were obtained in more than three separate experiments using hEGF. The same behavior was also observed in two experiments in which the binding of a truncated (residues 1-49) recombinant mouse EGF from a different source was studied.

### Example 2: Stoichiometry of EGF Binding to sEGFR

The occurrence of two different modes of EGF binding to sEGFR is entirely consistent with previous data regarding the occurrence of high- and low-affinity forms of EGFR. The ratio of the affinities of the two events seen in these studies is similar to that observed for EGFR on the cell surface and in detergent solution. Moreover, the K_{D} values estimated here are similar in magnitude to those previously reported in studies of EGF binding to sEGFR (Lax et al., J. Biol. Chem. 266:13828-13833, 1991b; Hurwitz et al., J. Biol. Chem. 266:22035-22043, 1991; Greenfield et al., EMBO J. 8:4115-4123, 1989) as well as to the full-length receptor solubilized in detergent (Yarden et al., J. Biol. Chem. 260:315-319, 1985; Yarden and Schlessinger, Biochemistry 26:1434-1442, 1987).

The surprising observation, however, in the present studies is that each mode of binding occurs with a stoichiometry of 1:2 (EGF:sEGFR), resulting in a final complex with overall stoichiometry of 1:1 (Table 1). Thus there appears to be one high-affinity and one low-affinity binding-site per receptor dimer. Indeed, in the titrations performed in Example 1, the inflection point at which the first'equilibrium reaction is complete (where the heat per injection is maximal) occurs at an EGF:sEGFR ratio of approximately 0.6. The high-affinity EGF-binding site therefore appears to be composed of two sEGFR molecules. A similar situation has been described for binding of the hGH to its receptor (Cunningham et al., Science 254:821-825, 1991), also employing ITC, and the simultaneous binding of two receptor molecules to a single ligand molecule has been confirmed in this case by X-ray crystallographic studies (de Vos et al., Science 255:306-312, 1992).

After saturation of the high-affinity 1:2 EGF:sEGFR complex suggested by these experiments, a second EGF molecule binds to give an overall 1:1 stoichiometry, in agreement with that measured previously under conditions of excess EGF (Weber et al., J. Biol. Chem. 259:14631-14636, 1984; Günther et al., J. Biol. Chem. 265:22082-22085, 1990). It is not possible to determine whether this second binding event leads to the formation of a 2:2 EGF:sEGFR complex, or to dissociation of the complex into two binary EGF:sEGFR complexes. It has been reported that binary (1:1) complexes of hGH with hGH-R result when hGH is added to its receptor in large excess (Cunningham et al., Science 254:821-825, 1991).

### Example 3: A Single sEGFR can Bind Two EGF Molecules

Titrations of sEGFR into solutions of EGF resulted in binding curves with similar characteristics. The best fits to the data thus suggest that two binding events also occur in these titrations. The first event occurs with a stoichiometry of 2:1 (EGF:sEGFR), suggesting that two EGF molecules can bind simultaneously to a single sEGFR molecule. The second, lower affinity, event corresponds to the formation of an EGF/sEGFR complex in which the overall stoichiometry is 1:1, as was also observed as the final stage when EGF was titrated into a solution of sEGFR. A third event, which would correspond to the formation of a 1:2 EGF:sEGFR complex, is not observed in titrations of sEGFR into EGF. Titrations of EGF into sEGFR also showed no evidence for a third event that would correspond to formation of the 2:1 (EGF:sEGFR) complex.

Similar observations have been reported by Cunningham et al. (Science 254:821-825, 1991) for hGH binding to the soluble portion of hGH-R. A fluorescence quenching study showed that addition of hGH in large excess to the 1:2 (hGH:hGH-R) complex resulted in its dissociation into two binary 1:1 complexes. This event was not detectable in the ITC experiments reported in this study (Cunningham et al., 1991), suggesting either that the enthalpy per injection associated with the event is below the limit of detection of the calorimeter, or that the excess of hGH required for its occurrence was not reached.

We suggest that similar possibilities explain our failure to detect the expected third event in ITC studies of the EGF/sEGFR interaction. In titrations of EGF into sEGFR, the ratio of the concentration of EGF to that of sEGFR in the calorimeter cell ranged from around 0.1 to 2.5. Similarly, in titrations of sEGFR into EGF, the sEGFR:EGF concentration ratio ranged from 0.1 to 1.8. Increasing the maximum ratio in either set of titrations, in order to observe the proposed third event, would not yield interpretable data. Finally, analysis of an EGF:sEGFR complex formed with an excess of EGF, and isolated by gel filtration, also suggested a state in which two EGF molecules were bound to each sEGFR.

### Example 4: Binding of EGF to Different Portions of sEGFR

The calorimetric studies of the EGF/sEGFR interaction presented above indicate the existence of complexes in which a single EGF molecule can bind to two receptor molecules. They also indicate a state in which two EGF molecules can bind to a single receptor molecule. One possible explanation for these observations would be that each receptor has two different potential EGF-binding domains, or half-sites. In the 2:1 EGF:sEGFR complex, each would be occupied by a separate ligand. In the 1:2 EGF:sEGFR complex, a different half-site on each receptor molecule could interact with the same EGF molecule.

The extracellular domain of EGFR can be divided into four subdomains: 1, 2, 3, and 4, on the basis of internal sequence homology (Lax et al., EMBO J. 8:421-427, 1989). Domains 2 and 4 are the cysteine-rich domains, and domains 1 and 3 are defined above. Studies of ligand binding to chimeric chicken/human EGF-receptor molecules, exploiting the differences in specificity of the two different receptors, indicate that domain 3 is a major determinant of ligand-binding domain in the receptor ((Lax et al., EMBO J. 8:421-427, 1989)), and that domain 1 also makes some contribution (Lax et al., Cell Regulation 2:337-345, 1991a). The role of domain 3 has been confirmed by affinity labeling studies (Lax et al., Mol. Cell. Biol. 8:1831-1834, 1988b), and by the observation that EGF binds specifically to isolated domain 3 (Kohda et al., J. Biol. Chem. 268:1976-1981, 1993). Significant homology is observed between subdomains 1 and 3 (Lax et al., Mol. Cell. Biol. 8:1970-1978, 1988), and deletion of domain 1 has been reported to reduce the affinity of EGF for the receptor by approximately one order of magnitude (Lax et al., Cell Regulation 1:173-188, 1990).

In order to investigate the possibility that subdomains 1 and 3 of sEGFR correspond to the potential half-sites for EGF binding, we generated two additional fragments of sEGFR: sEGFR(123), which lacks the second cysteine-rich domain (domain 4), and includes residues 1-505; as well as sEGFR(3), which contains only subdomain 3 (for example, residues 302-505). These definitions of the subdomains are merely meant to clarify what sequences were used in the examples . sEGFR(3) has previously been shown to bind EGF; but no ligand-induced dimerization was observed (Kohda et al., J. Biol. Chem. 268:1976-1981, 1993).

EGF induced dimerization of sEGFR(123) but not sEGFR(3). Purified sEGFR(123) or sEGFR(3) were treated with EGF in the absence or presence of the covalent cross linking agent DSS according to published procedures (Lax et al., J. Biol. Chem. 266:13828-13833, 1991; Hurwitz et al., J. Biol. Chem. 266:22035-22043, 1991). The samples were analyzed by SDS PAGE and stained with Coomasie-blue for visualization. sEGFR(3) migrated in SDS gel with an apparent molecule weight of 35,000 daltons (M-monomers). After cross-linking in the presence of EGF, a slower migrating component was detected that corresponds to sEGFR(3) covalently cross-linked with EGF. No evidence for cross-linked dimers of the sEGFR(3)/EGF complex was observed. sEGFR(123) migrates SDS-PAGE with an apparent molecular weight of 75,000 daltons (M-monomers). In the presence of both EGF and DSS an additional band of apparent molecular weight of 170,000 daltons (D-dimers) was detected, corresponding to dimers of the sEGFR(123)/EGF complex. The effect of addition of EGF to purified preparations of sEGFR(123) and sEGFR(3) upon their behavior in a covalent cross-linking experiment was observed. A small mobility shift upon treatment with the covalent cross linking agent DSS, only in the presence of EGF, shows that EGF binds to both fragments. In addition, cross-linked dimers are seen in the presence of EGF and DSS for sEGFR(123), as has been reported for sEGFR(1234) (Lax et al., J. Biol. Chem. 266:13828-13833, 1991; Hurwitz et al., J. Biol. Chem. 266:22035-22043, 1991), but not for sEGFR(3). Since EGF induces dimerization of sEGFR(123), subdomain 4 appears not to be required for receptor dimerization. sEGFR(3) binds specifically to EGF but does not undergo EGF dependent dimerization (Kohda et al., J. Biol. Chem. 268:1976-1981, 1993).

Binding studies performed with sEGFR(123) were consistent with a similar mode of EGF binding to that observed with sEGFR(1234). However, low levels of sEGFR(123) expression in CHO or insect cells precluded further detailed examination. ITC studies of EGF binding to sEGFR(3) gave K_{D} values (Table 2) that were similar to those obtained for the second EGF binding event to sEGFR(1234). However, the characteristics of the titrations were very different. The binding isotherms obtained from EGF/sEGFR(3) titrations were more similar to those seen for the aFGF/sFGFR titration. EGF binds to sEGFR(3) in a simple 1:1 fashion, with a K_{D} of 416 nM, and a negative associated enthalpy (ΔH = -2 kcal/mol). These characteristics, together with the absence of EGF-induced dimerization of sEGFR(3) are consistent with domain 3 representing one half-site for EGF binding to the EGF receptor.

The SH2 domain protein GRB-7 is co-amplified, overexpressed and in tight complex with HER2 in breast cancer

D. Stein, J. Wu, S.A.W. Fuqua¹, C. Roonprapunt, V. Yajnik, P. D'Eustachio², J. J. Moskow³, A. M. Buchberg³, C. K. Osborne' and B. Margolis⁴

New York University Medical Center, Department of Pharmacology, Kamplan Cancer Center and ²Department of Biochemistry, 550 First Avenue, New York, NY 10016, ³Department of Microbiology and Immunology, Jefferson Cancer Institute, Thomas Jefferson Medical College, Philadelphia, PA 19107 and ¹Division of Medical Oncology, at San Antonio, San Antonio, TX 78284, USA
¹Corresponding Author

### Communicated by J. Schlessinger

SH2 domain proteins are important components of the signal transduction pathways activated by growth factor receptor tyrosine kinases. We have been cloning SH2 domain proteins by bacterial expression cloning using the tyrosine phosphorylated carboxyterminus of the Epidermal Growth Factor Receptor as a probe. One of these newly cloned SH2 domain proteins, GRB-7, was mapped on mouse chromosome 11 to a region which also contains the tyrosine kinase receptor, HER2/erbB-2. The analogous chromosomal locus in man is often amplified in human breast cancer leading to overexpression of HER2. We find that GRB-7 is amplified in concert with HER2 in several breast cancer cell lines and that GRB-7 is overexpressed in both cell lines and breast tumors. GRB-7, through its SH2 domain, binds tightly to HER2 such that a large fraction of the tyrosine phosphorylated HER2 in SKBR-3 cells is bound to GRB-7. GRB-7 can also bind tyrosine phosphorylated SHC, albeit at a lower affinity than GRB2 binds SHC. We also find that GRB-7 has a strong similarity over more than 300 amino acids to a newly identified gene in *C. Elegans.* This region of similarity, which lies outside the SH2 domain, also contains a pleckstrin homology domain. The presence of evolutionarily conserved domains indicates that GRB-7 is likely to perform a basic signaling function. The fact that GRB-7 and HER2 are both overexpressed and bound tightly together suggests that this basic signaling pathway is greatly amplified in certain breast cancers.

### INTRODUCTION

Many growth factors bind to receptors with intrinsic tyrosine kinase activity (ScMessinger and uIlrich, 1992; Fantl et al., 1993). These receptors are crucial for normal development but can also act as oncogenes leading to cell transformation. The family of Epidermal Growth Factor Receptor (EGF-Receptor) related tyrosine kinases illustrates the diverse functions of these receptors. One of the EGF-Receptor related tyrosine kinases, the *C. Elegans* protein Let-23, is crucial for proper development of the nematode vulva (Aroian et al., 1990). Other members of this receptor family are more recognized for their role in oncogenesis. The avian oncogene, verbB, represents a truncated homologue of the mammalian EGF-Receptor (Downward et al., 1984). Another receptor with close similarity to EGFReceptor is HER2, also known as c-erbB-2 (Coussens et al., 1985). This receptor was also isolated as the rat oncogene neu, an oncogene responsible for chemically induced rat glioblastomas (Bargmann et al., 1986). HER2/erbB-2 is known to be amplified and overexpressed in about 25% of human breast cancers (Slamon et al., 1987; Slamon et al., 1989). In many, but not all studies, this HER2 overexpression correlates with a poor prognosis (Elledge et al., 1992).

To understand the mechanism by which these receptor tyrosine kinases are responsible for both normal development and transformation, it is important to understand the basic intracellular signaling mechanisms activated by these tyrosine kinases. Recent studies have indicated an important role for proteins with SH2 domains in growth factor receptor signal transduction (Pawson and Schlessinger, 1993; Margolis, 1992). These proteins bind to the tyrosine phosphorylated growth factor receptors due to a direct interaction between the SH2 domain and the phosphotyrosine containing peptide sequences within the receptor (Moran et al., 1990; Margolis et al., 1990a). The SH2 domain proteins then trigger several different signaling cascades. Some SH2 domain proteins, such as -PLC-g, have intrinsic enzymatic activity. The binding of PLC-g to the growth factor receptor tyrosine kinase triggers the phosphorylation of this enzyme leading to a breakdown of polyphosphoinositides (Rhee and Choi, 1992). Other SH2 domain proteins, such as Phosphatidylinositol-3 Kinase (PI-3 Kinase) associated p85, act as adaptors coupling growth factor receptors to other signaling molecules. As an example, p85 serves as a bridge binding growth factor receptors to the 110 kDa PI-3 Kinase (Hiles et al., 1992).

Several SH2 domain proteins were isolated in our laboratory by screening bacterial expression libraries with the tyrosine phosphorylated carboxyterminus of the EGF-Receptor (Skolnik et al., 1991; Lowenstein et al., 1992; Margolis et al., 1992). We called this method CORT for Cloning Factor Receptor Targets. The proteins cloned were called GRB's (Growth Factor Receptor Bound). GRB1 was identified as the PI-3 Kinase associated p85 (Skolnik et al., 1991). The second protein isolated using this method was termed GRB2 and consisted of one SH2 domain sandwiched between two SH3 domains (Lowenstein et al., 1992). GRB2 is a homologue of the C. Elegans protein sem-5 (Clark et al., 1992). Sem-5 is downstream of the receptor tyrosine kinase Let-23 and like Let-23 is crucial for vulval development. Similarly, a D. melanogaster homologue of GRB2 and sem-5, Drk, is necessary for proper eye development where it lies downstream of the sevenless tyrosine kinase receptor (Simon et al., 1993; Olivier et al., 1993). Through a combination of biochemical and genetic studies it has been determined that GRB2 serves as a crucial link between receptor tyrosine kinases and ras. GRB2 acts as an adaptor forming a complex between activated receptor tyrosine kinases and son of sevenless (SOS), a ras GTP/GDP exchange protein (Olivier et al., 1993; Simon et al., 1993; Li et al., 1993; Gale et al., 1993; Egan et al., 1993; Buday and Downward, 1993; Chardin et al., 1993).

Recently, using CORT, we identified another novel SH2 domain protein called GRB-7 (Margolis et al., 1992). GRB-7 has a single SH2 domain at its carboxyterminus, a central region with similarity to ras GAP and a proline rich aminoterminus. In this paper, we report that GRB-7 maps to a region on mouse chromosome 11 containing the gene for the HER2/erbB-2 receptor tyrosine kinase. This region of mouse chromosome 11 is syntenic to an area of human chromosome 17q that is often amplified in breast cancer (Buchberg et al., 1989; van de Vijver et al., 1987; Slamon et al., 1987). Accordingly, we examined GRB-7 expression in breast cancer. We find that GRB-7 is amplified and overexpressed in breast cancer in concert with the HER2 receptor. GRB-7 is bound tightly to HER2 via its SH2 domain such that the majority of tyrosine phosphorylated HER2 is complexed with GRB-7. Finally, we demonstrate that GRB-7 contains a region of more than 300 amino acids with similarity to a *C. Elegans* gene recently identified by the *C. Elegans* genome project (Sulston et al., 1992). This region lies outside the SH2 domain and is likely crucial in GRB-7 signal transduction.

### RESULTS

### GRB-7 Localizes to mouse chromosome 11 near HER2/Erbb-2

To determine GRB-7 chromosomal localization, liver genomic DNA from inbred and recombinant inbred (RI) strains of mice was digested with TaqI and typed by Southern blotting as described previously (Sap et al., 1990). This analysis revealed two allelic forms of GRB-7, whose inheritance in RI strains of mice defined a single locus. Comparison of the strain distribution pattern for GRB-7 to those for 1124 other markers distributed over all of the mouse chromosomes allowed GRB-7 to be localized to chromosome 11 on the basis of its tight linkage to markers such as HoxB (1 recombinant among 31 informative RI strains scored), Gfap (5 recombinants among 52 strains), and Krt-1 (1 recombinant among 31 strains).

To refine the localization of GRB-7, we determined its location on mouse chromosome 11 in an interspecific backcross, that has been typed for over 30 genetic markers distributed throughout its length. Genomic DNAs from AEJ/Gn and Mus spretus parental control animals were digested with several restriction endonucleases and analyzed by Southern blot hybridization using probes that identified the following loci; Ngfr, Grb-7, Erbb-2, and Csfg (Table 1). At least one restriction fragment length polymorphism (RFLP) was identified for each of the probes tested and the sizes of the genomic restriction fragments detected by each probe are listed in Table 1. To determine the size polymorphism for D11Mit10, parental DNAs and F1 controls were polymerase chain reaction (PCR) amplified using the oligomers defining D11Mit10. As expected, a simple sequence length polymorphism (SSLP) was detected between AEJ/Gn and M. spretus (Table 1).

The segregation pattern of the M. spretus allele in 191 backcross animals was then determined for Ngfr, Grb-7, Erbb-2, and Csfg (by Southern blot hybridization) and for D11Mit10 by PCR amplification and agarose gel electrophoresis. The results are summarized in Figure 1.

Mice were either homozygous for the AEJ/Gn allele or heterozygous for the M. spretus and AEJ/Gn alleles. The frequency of AEJ/Gn and M. spretus alleles for the loci mapped in the N2 progeny did not significantly differ from the expected 1:1 ratio. The results presented here are consistent with the previous genetic localization of Ngfr, Erbb-2, and Csfg using a [(C57BL/6J x M. spretus) x C57BL/6J interspecific backcross (Buchberg et al., 1989). Gene order was determined by minimizing the number of multiple recombinants between loci. The order of the loci and the ratio of the number of recombinants to the total number of N2 offspring examined for each locus are: Ngfr-5/191-(Grb-7, Erbb-2, Csfg)-6/191-D11Mit10. The genetic distances between the loci in centimorgans+standard error are: Ngfr -2.6±1.1-(Grb-7, Erbb-2, Csfg)-3.1±1.2-D11Mit10. No recombinants were detected between Grb-7, Erbb-2 and Csfg in 191 N2 progeny, indicating that these loci are tightly linked and must lie < 1.6 cM apart (upper 95% confidence limit).

### GRB-7 is amplified and overexpresed in breast cancer cell lines

Next, we determined if GRB-7 is amplified and overexpressed with HER2 in breast cancer. We checked the expression of GRB-7 in several breast cancer cell lines by immunblotting cell lysates for GRB-7 expression. We found a close correlation between HER2 and GRB-7 expression (Fig. 8A). To confirm that this overexpression was due to gene amplification, we performed southern blotting with a GRB-7 probe (Fig. 8B). GRB-7 is amplified in both SKBR-3 cells and BT-474; two cell lines in which HER2 is also amplified (Kury et al., 1990). Neither GRB-7 nor HER2 is amplified in the other cell lines.

### GRB-7 is overexpressed in breast cancer tissue.

Immunoblotting with the GRB-7 antibody was then performed on 72 breast cancer samples in which HER2 expression had previously been analyzed, also by an immunoblot procedure (Ciocca et al., 1992). A representative result for ten such tumors is displayed in Fig. 8C. Thirty-four of the breast cancer specimens exhibited HER2 overexpression, and 24 of these were concomitantly positive for GRB-7 expression (Table 2). In the 38 tumors negative for HER2 overexpression, only 8 were positive for GRB-7. These results were statistically significant by chi-square analysis (p<0.001), and they confirm the cell line data, suggesting that GRB-7 is often expressed in human breast tumors in concert with HER2 overexpresslon.

### GRB-7 binds tightly to HER2.

We next examined if the GRB-7 protein was physically associated with the HER2 receptor. This might be expected as GRB-7 was cloned based on its ability to bind to the EGF-Receptor, a receptor closely related to HER2 (Coussens et al., 1985). To perform these studies, GRB-7 was immunoprecipitated from serum-starved SKBR-3 cells and then blotted with phosphotyrosine or HER2 antibodies. Antibodies to GRB-7 specifically immunoprecipitated a phosphotyrosine-containing band at 190 kDa (Fig. 9A, left panel) which was identified as HER2 (Fig. 9A, middle panel). This coimmunoprecipitation was not seen with preimmune serum. No other bands were detected in the antiphosphotyrosine blot indicating that GRB-7 was not tyrosine phosphorylated (Fig. 9A, middle and right panels). This coimmunoprecipitation between GRB-7 and HER2 was demonstrated with three different GRB-7 antisera indicating it was not dependent on the antibody used. The coimmunoprecipitation of GRB-7 and HER2 was also seen in BT474 cells (results not shown).

To determine what percentage of HER2 was bound to GRB-7 in these cells, we immunoprecipitated GRB-7 from a small number of cells such that we depleted the lysate of GRB-7. Under these conditions, the lysates were almost completely cleared of tyrosine phosphorylated HER2 as well as GRB-7 (Fig. 9B). Preimmune serum which did not immunoprecipitate GRB-7 did not significantly affect the tyrosine phosphorylated HER2 in the lysates. It should be noted that GRB-7 immunoprecipitation did not measurably affect the total amount of HER2 in the cell lysate as only a small fraction of HER2 is tyrosine phosphorylated and able to bind GRB-7. These results indicate a strong association between tyrosine phosphorylated HER2 and GRB-7.

### GRB-7 binds SHC.

We next asked if GRB-7 became tyrosine phosphorylated after ligand stimulation of growth factor receptors. Because the true ligand or ligands for HER2 is still unclear (Peles et al., 1993; Plowman et al., 1993), we stimulated tyrosine phosphorylation by activation of the EGF-Receptor in SKBR-3 cells (King et al., 1988). After stimulation with EGF, several additional bands become tyrosine phosphorylated yet tyrosine phosphorylation of GRB-7 was still undetectable (Fig. 10). Phosphoamino acid analysis of GRB-7 before and after stimulation revealed the presence of phosphoserine and phosphothreonine but no phosphotyrosine (results not shown).

We did find that GRB-7 antibodies immunoprecipitated a tyrosine phosphorylated protein of 54 kDa after EGF stimulation (Fig. 10). We suspected that this 54 kDa band might be tyrosine phosphorylated SHC (Pelicci et al., 1992) as it has been found that another SH2 domain protein, GRB2, binds tightly to phosphorylated SHC (Rozakis-Adcock et al., 1992; Skolnik et al., 1993).

To confirm this suspicion, we immunoprecipitated GRB-7 from EGF stimulated SKBR-3 cells and immunoblotted with antiSHC antibodies (Fig. 10 right panel). We were able to detect EGF stimulated association of SHC and GRB-7.

We next wanted to determine if GRB-7 binds directly to HER2 and SHC through the GRB-7 SH2 domain. The association of the GRB2 SH2 domain with SHC presumably occurs due to the tyrosine phosphorylation of SHC at residue 317 (Skolnik et al., 1993; Rozakis-Adcock et al., 1992; Songyang et al., 1993). The sequence around this tyrosine, YVN, is felt to represent a high affinity binding site for the SH2 domain of GRB2 when tyrosine phosphorylated. A similar motif is present in EGF-Receptor at tyrosine 1068 (YIN) and in HER2 at tyrosine 1139 (YVN). We suspected that the GRB-7 SH2 domain might also bind to both HER2 and SHC via this motif. To study this problem, we prepared a GST fusion protein of the GRB-7 SH2 domain containing a protein kinase A phosphorylation site (Ron and Dressler, 1992). Phosphorylation by protein kinase A in the presence of ²P-ATP was then used to label the GST fusion protein. SKBR-3 lysates were separated by SDS-PAGE, transferred to nitrocellulose and probed with the labeled GRB-7 SH2 domain. During these incubations, we added a one hundred fold molar excess of unlabeled GST, GST-GRB-7-SH2 or GSTGRB2. With GST alone, the GRB-7 SH2 domain bound to two major bands at 190 kDA and 54 kDa corresponding to HER2 and SHC (Fig. 11A, left panel). This demonstrates that GRB-7 can bind directly to these proteins in cells and does not require intermediate molecules. As expected, unlabeled GRB-7 competed the binding to both proteins (Fig. 11A, middle panel). We found that GRB2 could prevent the GRB-7 SH2 domain from binding to SHC but did not affect HER2 binding (Fig. 11A, right panel).

When we probed the same lysates with labeled GRB2 at a similar concentration and specific activity as the GRB-7-SH2 domain, GRB2 showed strong binding to SHC but no binding to HER2 (Fig. 11B). We did detect binding of GRB2 to a protein at 160 kDA which is not HER2 but likely represents the association of the GRB2 SH3 domains to SOS (Li et al., 1993; Egan et al., 1993; Buday and Downward, 1993; Rozakis-Adcock et al., 1993). Unlabelled GRB-7 could not compete the binding of GRB2 to SHC in contrast to the fact that GRB2 could easily compete GRB-7 binding to SHC. Taken together these results indicate that GRB-7 binds SHC probably at the same Y(V/I)N motif as GRB2. However, the affinity of GRB2 for SHC appears much greater than that of GRB-7. In contrast, GRB-7 binds tightly to HER2, but GRB2 does not compete this binding.

### GRB-7 is phosphorylated by the EGFR/HER2 chimera

To determine if GRB-7 was tyrosine phosphorylated by activated HER2, we transfected GRB-7 into NIH 3T3 cells containing the chimeric EGFR/HER2 receptor (Lee et al., 1989). These cells contain the HER2 intracellular domain fused to the EGF-Receptor extracellular domain such that EGF can stimulate the HER2 tyrosine kinase. Mter EGF stimulation of these cells, GRB-7 becomes tyrosine phosphorylated and associates with the chimeric receptor in a ligand dependent fashion (Fig. 12A). EGF also induces association between GRB-7 and an unidentified tyrosine phosphorylated protein of 70 kDa. We examined the binding of the ³²P-GRB-7 SH2 domain to lysates from NIH 3T3 cells expressing the EGFR/HER2 chimera (Fig. 12B). We detected binding to three bands; the chimeric receptor at 190 kDa, the 54 kDa SHC band, and the unidentified band at 70 kDa. The binding of the GRB-7 SH2 domain to these protein was enhanced in lysates from EGF stimulated cells. There was some binding of the probe to the receptor even in the absence of ligand because the receptor is autophosphorylated to some extent in the untreated cells. GRB2 was able to completely compete the binding of the GRB-7 SH2 domain to SHC but did not significantly affect the binding to the 70 kDa protein. GRB2 weakly competed with GRB-7 for binding to the chimeric receptor.

Fig. 12 GRB-7 phosphorylation and binding in NIH 3T3 cells expressing the EGFR/HER2 chimera. A Tyrosine phosphorylation of GRB-7. Cells expressing the EGFR/HER2 chimera with and without GRB-7 were starved in serum free media for 48 hours and then treated with EGF for 3 minutes. Lysates (1.5 mg protein) were immunoprecipitated with GRB-7 antibodies and separated by SDS-PAGE. After transfer to nitrocellulose, blots were probed with anti-PTyr or anti-GRB-7 antibodies. Eighty percent of the immunoprecipitate was run for anti-PTyr blot and twenty percent for GRB7 blot. B. GRB-7 SH2 domain associates with three proteins in NIH 3T3 cells expressing the EGFR/HER2 chimera. Lysates (50 mg) from cells expressing the chimeric receptor were run out on SDS-PAGE and transferred to nitrocellulose. Blots were then probed with ³²P-GRB-7 SH2 domain as in Fig. 11. Primary sequence of GRB-7 reveals homology to a putative gene in *C. Elegans* and the pleckstrin domain.

Our data indicates that GRB-7 is a signaling partner for HER2 especially in breast cancer cells where both are overexpressed. However, it is not clear what signal might be sent through GRB-7 when it binds and perhaps becomes phosphorylated by HER2. We have recently detected a close similarity between GRB-7 and a putative gene identified by the *C. Elegans* genome sequencing project (Sulston et al., 1992). This putative *C. Elegans* gene F10E9.6 encodes a predicted protein of 650 amino acids with no SH2 domain (Fig. 13A). The region of similarity spans approximately 330 amino acids with an identity of 28% and similarity of 38% (Fig. 13B). Using the randomization utility of the alignment program, Bestfit (Devereux et al., 1984), we found that the optimal alignment score for GRB-7 to F10E9.6 lies more than 18 standard deviations from the mean of scores from ten scrambled alignments. This indicates a highly significant relationship between the two proteins.

This region of similarity between F10E9.6 and GRB-7 also contains a pleckstrin domain. First described by Mayer and coworkers (Mayer et al., 1993) as well as Haslam and coworkers (Haslam et al., 1993), the pleckstrin domain is found in several different signaling molecules including pleckstrin (Tyers et al., 1988), the serine kinase akt/rac (Jones et al., 1991; Bellacosa et al., 1991) ras GAP (Vogel et al., 1988; Trahey et al., 1988) and the SH3 binding protein, 3BP-2 (Ren et al., 1993). In fact, the similarity we initially detected between ras GAP and GRB-7 encompasses the pleckstrin domain (Margolis et al., 1992). It has been suggested that the pleckstrin motif may function as a protein binding domain (Mayer et al., 1993; Haslam et al., 1993; Musacckio et al., 1993). An alignment of GRB-7, F10E9.6 and the derived consensus sequences for the pleckstrin domains is shown in Fig. 13C.

A more detailed alignment of these proteins in the context of other pleckstrin domain proteins has also been recently published (Musacchio et al., 1993). While it is not known what signal is relayed by GRB-7, it is clearly a protein composed of evolutionarily conserved domains indicating that it likely performs a basic signaling function.

### DISCUSSION

In this paper we report an amplification and overexpression of GRB7 in several different breast cancer cell lines. GRB-7 overexpression is also found in tissue samples from primary human breast cancer. This overexpression correlates with HER2 expression indicating that these two genes are often coamplifled an&or coexpressed. Other genes on human chromosome 17q have also been found to be coamplified with HER2. Although c-erbA/Thyroid hormone receptor is coamplified with HER2, overexpression of this protein has not been detected (van de Vijver et al., 1987; Tavassoli et al., 1989). Topoisomerase Ila has been found to be coamplified with HER2 but in a relatively small percentage of the cases (Smith et al., 1993). Our results indicate that GRB-7 is likely present in an amplicon which also contains HER2 and which represents a region on 17q. This region on 17q lies proximal to the locus containing the breast cancer susceptibility gene, BRCA1 (Bowcock et al., 1993).

Not only are GRB-7 and HER2 coexpressed in breast cancer but they also exist in a tight complex. In SKBR-3 cells, we coimmunoprecipitate a large fraction of the tyrosine phosphorylated HER2 with GRB-7 antibodies. The fraction of total HER2 that is bound to GRB-7 is relatively low, as only a small percentage of HER2 is tyrosine phosphorylated in these unstimulated cells. GRB-7 is not only bound to HER2 in SKBR-3 cells but can also bind SHC. The binding of GRB-7 to SHC gives us some insight into the tyrosine phosphorylation sites to which the GRB-7 SH2 domain can bind. It seems likely that the GRB-7 SH2 domain binds the Y(VII)N motif on SHC but with a lower affinity than GRB2. GRB2, which competes with GRB-7 for binding to SHC does not affect the binding of GRB-7 to HER2 in the SKBR-3 cells. This suggests that GRB-7 either binds to a site other than Y(V/I)N on HER2 or binds to the Y(V/I)N site on HER2 but with a much greater affinity than GRB2. It should be noted that GRB2 does not bind HER2 from the SKBR-3 cells yet binds to the EGFR/HER2 chimeric receptor after EGF stimulation (J. W. and B.M., unpublished results) . This suggests that the intensity or sites of autophosphorylation on the EGFR/HER2 chimera may be different than on HER2 from SKBR-3 cells.

The tyrosine phosphorylation sites of HER2 and EGF-Receptor are closely related in sequence (Hazan et al., 1990; Segatto et al., 1990). Thus it is likely that GRB-7 binds to the same site on HER2 and EGFR. However, attempts to inhibit the binding of GRB-7 to HER2 using tyrosine phosphorylated peptides from the EGF-Receptor have been to date, unsuccessful. It has been notoriously difficult to map SH2 domain binding sites on EGF-receptor in the past due to the close proximity and overlapping binding of several of the autophosphorylation sites. For example, PLC-g binds with high affinity to three different autophosphorylation sites (Rotin et al., 1992). Thus further work will be necessary to determine the actual binding preference of the GRB-7 SH2 domain. Other clues to the exact binding site of GRB-7 may come from the identity of the 70 kDa protein which binds to the SH2 domain of GRB-7 in the EGF stimulated NIH 3T3 cells expressing the EGFR/HER2 chimera. Our data suggest that this protein is not the 66 kDa form of SHC because GRB2 cannot compete this binding. Additionally, we could not detect the 66 kDA form of SHC in GRB7 immunoprecipitates (J. Wu and B.M., unpublished observations). Other potential candidates for this protein are PTP-1D/Syp (Feng et al., 1993; Vogel et al., 1993) or paxillin (Birge et al., 1993).

In contrast to the results obtained in the SKBR-3 cells, GRB-7 was tyrosine phosphorylated in EGF stimulated cells expressing chimeric EGFR/HER2 receptor. The phosphorylation state of GRB-7 in breast tumors in vivo remains to be determined. Nonetheless, there is a distinct possibility that the binding of GRB-7 to the receptor may be sufficient to initiate the GRB-7 signaling pathway. Several proteins such as GRB2 and PI-3 kinase associated p85 appear to transduce their signal without tyrosine phosphorylation (Lowenstein et al., 1992; Backer et al., 1992; Carpenter et al., 1993). For these adaptor proteins, the binding of the SH2 domain to the receptor appears sufficient to initiate the signaling cascade.

One of the major questions that remains is the signal relayed by GRB7. The SH2 domain proteins are grouped into two major classes; those that have intrinsic catalytic or signaling activity and those that act as adaptors coupling secondary proteins to growth factor receptor tyrosine kinases (Pawson and Schlessinger, 1993; Margolis, 1992). The one clue that GRB-7 may act as an adaptor molecule is the fact that GRB-7 contains a sequence similar to the recently described pleckstrin domain. This motif is found in several proteins that already have catalytic activity (Mayer et al., 1993; Haslam et al., 1993; Musacchio et al., 1993). In these proteins, it is theorized that the pleckstrin domain has a role in binding other regulatory factors. One possible example of this function is the association of the pleckstrin domain on the b-adrenergic receptor kinase with bg subunits of heterotrimeric G-proteins (Musacchio et al., 1993; Koch et al., 1993). The amino acid sequence identity between different pleckstrin domains is less than 20%, so more studies will be required before a uniform function can be assigned to this domain.

The alignment between the putative C. *Elegans* gene F10E9.6 and GRB-7 is more significant and the two genes likely function in a similar fashion. The F10E9.6 gene is theorized to exist based on the analysis of the C. *Elegans* genome sequence using the program Genefinder (Sulston et al., 1992). This similarity includes the pleckstrin domain and a second region which extends toward the aminoterminus. It is also interesting to note that GRB-7 and F10E9.6 both have proline rich domains but that F10E9.6 does not appear to have a SH2 domain. Such proline rich regions are potential binding sites for SH3 domains (Ren et al., 1993; Li et al., 1993). One gene that has been mapped to the same region of the C. Elegans genome as F10E9.6 is mig-10. Mig-10 was isolated as a gene involved in longitudinal neuronal migration in the nematode (Manser and Wood, 1990). Certain cells in C. *Elegans* such as the canal associated neuron (CAN) and the hermaphrodite specific neuron (HSN) must undergo long range migrations which they do not complete in the mig-10 mutants. There are more than 20 genes known to be required for this process but the identity of these genes is not yet known (Wadsworth and Hedgecock, 1992). There is preliminary evidence that mig-10 could be encoded by F10E9.6 based on cosmid rescue data and partial cDNA sequence (J. Manser, personal communication). However, further studies on the relationship of F10E9.6, mig-10 and GRB-7 will be required.

Our data seems to suggest a basic signaling function for GRB-7, yet GRB-7 is expressed only in kidney, liver and gonads suggesting it may have a more specialized function. Recently, in a CORT screen of NIH 3T3 cells using the tyrosine phosphorylated EGF-Receptor, we have isolated a second gene with high similarity to GRB-7 (J. Ooi, V.Y., D. Immanuel, and B.M, unpublished data). This gene, which we call GRB-10, is approximately 60% identical to GRB-7 through the SH2 domain and the central F10E9.6 domain. GRB-10 appears to be more widely expressed than GRB-7 indicating that the basic signaling function of GRB-7 may be performed by a family of related genes in different tissues.

One point of future interest is the effect of GRB-7 overexpression on breast cancer biology. Currently, our results indicate that GRB-7 expression alone cannot transform NIH-3T3 cells (J.W. and B.M. unpublished results). In the NIH 3T3 cells which express GRB-7 and the chimeric EGFR/HER2 receptor, our data is inconclusive because these cell lines are already transformed due to the high expression of the chimeric receptor. Whether GRB-7 expression, like HER-2, has prognostic significance in patients with primary breast cancer remains to be seen. Although our data indicate a highly significant correlation between overexpression of HER-2 and overexpression of GRB-7 in patient samples, the relationship is imperfect; 24 of the 34 specimens overexpressing HER-2 also overexpress GRB-7 but 10 do not. Similarly, about one-third of HER-2 negative tumors still overexpress GRB-7. It is interesting to speculate that those tumors overexpressing both proteins would have particularly aggressive course and worse patient outcome, a hypothesis we are now testing. Aside from their role in cancer, GRB-7 and related genes are likely to have a basic function in signal transduction. A combination of biochemical studies in mammalian cells and genetic studies in D. Melanogaster and C. Elegans lead to the elucidation of the function of GRB2. Likewise, a similar combination of genetics and biochemical studies will lead to a better understanding of the function of GRB-7.

### MATERIALS AND METHODS

### Tissue Culture

Breast cancer cell lines were originally obtained from the ATCC and grown in DMEM, 4500 mg/dl glucose with penicillin/streptomycin and 10% Fetal Calf Serum. All NIH 3T3 cells were grown in the same media but using 10% calf serum. NIH 3T3 cells expressing the EGFR/HER2 chimera were obtained from Dr. A. Zilberstein and Dr. A. Ullrich. For experiments cells were starved as described in the figure legends.

### Immunoprecipitation and immunoblotting.

Several rabbit polyclonal antibodies were generated against GRB-7. Three antibodies were generated against GST fusion proteins of GRB-7 consisting of amino acid 419-535 (#191), amino acid 297-535 (#193) and the full length protein (#222). An antipeptide antibody was generated against amino acid sequence 264-279 (#188). Antibodies were either used as whole serum or were affinity purified. For affinity purification, the fusion protein or peptide was immobilized on Affi-gel 10 (Biorad). Serum was concentrated by 50% ammonium sulfate precipitation and dissolved in 10 mM Tris, pH 7.5. The ammonium sulfate precipitated antibodies were then purified on the antigen column as described (Harlow and Lane, 1988) eluting the antibody with 100 mM Glycine, pH 2.5. Immunoprecipitation and immunoblotting in the cell lines were performed as previously described (Margolis et al., 1989). Immunoblotting of the breast tumor samples with the GRB-7 antibody was performed using 100 mg of sodium dodecyl sulfate-solubilized protein extract as previously described (Tandon et al., 1989). SHC antibodies were purchased from Signal Transduction Labs (Lexington, Kentucky). Rabbit polyclonal antibodies directed against the carboxyterminus of HER2 were generously provided by Dr. A. Zilberstein. Rabbit polyclonal antiphosphotyrosine antibodies were prepared using standard techniques (Kamps and Sefton, 1988).

### GRB-7 constructs

GST fusion proteins were generated by polymerase chain reaction of GRB-7 incorporating BamH1 restriction sites into the priming oligonucleotides. For the GST GRB-7 SH2 domain probe, the sequence encoding amino acids 419-535 was amplified, digested with Bam Hi and ligated into Bam Hi digested pGSTag vector (Ron and Dressler, 1992). The pGSTag vector was a gift from D. Ron and the GRB2 pGSTag construct was a gift from E. Lowenstein and J. Schlessinger. For mammalian expression of GRB-7, GRB-7 was cut from the gXIox plasmid with xba1 and Mse1 and blunt ended using Kienow fragment. This blunt end fragment was ligated into EcoRV digested PMJ30 vector (Margolis et al., 1990b). NIH 3T3 cells were transfected as previously described (Margolis et al., 1990b) using GRB7 in the anti sense direction as a control. The Hygromycin-B phosphotransferase gene was cotransfected with the GRB-7 constructs as a selectable marker.

### GST Blotting

The pGSTag constructs were labelled with g32P-ATP as described using 0.2 U/ml protein kinase A (Ron and Dressler, 1992). Blots were incubated for 2 hours at room temperature in block buffer (20 mM Hepes, pH 7.5, 5 mM MgCl2, 1 mM KCl, 5 mM DTT, 5% nonfat dry milk, 0.02% sodium azide) and probed for two hours at room temperature in the same buffer using 12.5 ng ( 3 x 10⁵ dpm) per ml of probe. Blots were washed four times for fifteen minutes with Tris buffered saline (10 mM Tris, pH 7.5, 150 mM NaCl) containing 0.1% Triton X-100 before exposing.

### Southern blotting

Genomic DNA was prepared from tissue culture cells using standard techniques (Sambrook et al., 1989). Ten micrograms of DNA was digested with EcoR1 or Hind III and separated on 0.7% agarose gel. The DNA was transferred to Nytran (Schleicher and Schuell) using capillary action and crosslinked by ultraviolet light. The blot was blocked for 4 hours at 42°C in hybridization buffer (40% Formamide, 10 x Denhardt's, 1% SDS, 6x SSC and 100 mg/ml salmon sperm DNA) and then incubated with probe (2 x10⁶ dpm/ml) overnight at 42°C. The probe consisted of a PCR product from mouse GRB-7 encompassing nucleotides 1437 to 1909. The probe was labelled with ³²P-dCTP using a random priming kit (USB). The blots final wash was 0.5xSSC, 0.1% SDS, 42°C.

### DNA analysis

All DNA and protein database searches were performed using the University of Wisconsin Genetics Computer Group Sequence Analysis Software (GCG) package (Devereux et al., 1984). The GenEMBL database were searched using Fasta and Tfasta respectively (Pearson and Lipman, 1988). Protein alignments were performed with the GCG program, Bestflt. Conservative substitutions were defined as a score of> 0.8 using the scoring table of Schwartz and Dayhoff (Schwartz and Dayhoff, 1979) as modified by Gribskov and Burgess (Gribskov et al., 1984).

### Chromosome mapping

The interspecific backcross between (AEJ/Gn-a bpH/abpH x Mus spretus) F1 x AEJ/Gn-a bpH/abpH was previously described (Marini et al., 1993). Genomic DNA extractions, restriction endonuclease digestions, agarose gel electrophoresis, and Southern blot transfers, hybridizations and washes were described (Ma et al., 1993). DNA oligonucleotides used for detecting the SSLP marker was made using an Applied Biosystems Model 393 DNA synthesizer. SSLP markers were detected by amplifying genomic DNA from N2 animals using the specified DNA oligonucleotide pairs (Dietrich et al., 1992) and Taq DNA polymerase as described (Ma et al., 1993). The results of the interspecific backcrosses were analyzed by calculating the maximum likelihood estimates of linkage parameters as described. (Green, 1981)

### ACKNOWLEDGMENTS.

We thank Yossi Schlessinger for his continued support and members of his laboratory for advice and reagents. We also thank Axel Ullrich, Peter Hirth and Son Kuan (Sugen, Inc.) for breast cancer cell lines, the C. Elegans sequencing consortium for F10E9.6 sequence and chromosomal localization and especially Jim Manser for information on mig10. We are grateful to Kiki Nelson for oligonucleotide and peptide synthesis. Computing at NYU was supported by NSF under grant number DIR8908095. B.M is a Lucille P. Markey and Kaplan Cancer Center Scholar. This work was supported by grants from The Lucille P. Markey Charitable Trust, NIH grants CA58586 (AMB), P30 CA54174 (CKO), PO1 CA30195 (CKO), and P50 CA58183 (CKO).

### REFERENCES

Aroian,R.V., Koga,M., Mendel ,J.E., Ohshima,Y. and Sternberg,P.W. (1990) Nature,348, 693-699
Backer,J.M., Myers,M.G.,Jr., Shoelson,S.E., Chin,D.J., Sun, X.-J., Miralpeix,M., Hu,P., Margolis,B., Skolnik,E .Y., Schlessinger,J. and White,M.F. (1992) EMBO J.,11, 3469-3479
Bargmann,C.I., Hung,M.C. and Weinberg,R.A. (1986) Nature,319, 226-230 Bellacosa,A., Testa,J.R., Staal,S.P. and Tsichlis,P.N. (1991) Science ,2 54, 274277
Birge,R.B., Fajardo,E., Reichman,C., Shoelson,S.E., Songyang,Z., Cantley,L.C. and Hanafusa,H. (1993) Mol. Cell. Biol.,13, 4648-4656
Bowcock,A.M., Anderson,L.A., Friedman,L.S., Black,D .M., OsborneLawrence,S., Rowell,S.E., Hall,J.M., Solomon,E. and King,M.C. (1993) Am. j. Hum. Genet.,52, 718-722
Buchberg,A.M., Brownell,E., Nagata,S., Jenkins,N.A. and Copeland,N.G. (1989) Genetics,122, 153-161
Buday,L. and Downward,J. (1993) Cell,73, 611-620
Carpenter,C.L., Auger,K.R., Chanudhuri, M., Yoakim,M., Schaffhausen,B., Shoelson, S. and Cantley,L.C. (1993) j. Biol. C. hem. ,268, 9478-9483
Chardin,P., Camonis, J.H., Gale,N.W., van Aelst,L., Schlessinger,J., Wigler,M.H. and Bar-Sagi,D. (1993) Science,260, 1338-1343
Ciocca,D.R., Fujimura,F.K., Tandon,A.K., Clark,G.M., Mark,C., LeeChen,G.J., Pounds,G.W., Vendely,P., Owens,M.A., Pandian,M.R. and McGuire,W.L. (1992) JNCI,84, 1279-1282
Clark,S.G., Stern,M.J. and Horvitz,H.R. (1992) Nature,356, 340-344 (24)
Coussens,L., Yang-Feng,T.L., Liao,Y.C., Chen,E., Gray,A., McGrath,J., Seeburg,P.H., Libermann,T.A., ScMessinger,J., Francke,U.,Levinson,A. and Ullrich,A. (1985) Science,230, 1132-1139
Devereux,J., Haeberli,P. and Smithies,O. (1984) Nucleic Acids Res.,12, 387-395
Dietrich,W., Katz,H., Lincoln,S.E., Shin,H.S., Friedman,J., Dracopoli,N.C. and Lander,E.S. (1992) Genetics,131, 423-447
Downward,J., Yarden,Y., Mayes ,E., Scrace ,G., Totty,N., Stockwell,P., Ullrich,A., Schlessinger,J. and Waterfleld,M.D. (1984) Nature,307, 521-527
Egan,S .E., Giddings,E.W., Brooks ,M.W., Buday,L., Sizeland,A.M. and Weinberg,R.A. (1993) Nature,263, 45-51
Elledge,R.M., McGuire,W.L. and 0 sborne,C K. (1992) Semin. Oncol. , 19, 244-253
Fantl,W.J., Johnson,D.E. and Williams,L.T. (1993) Annu. Rev. Biochem. ,62, 453-481
Feng,G.S., Hui,C.C. and Pawson,T. (1993) Science,259, 1607-1611
Gale,N.W., Kaplan,S., Lowenstein,E.J., Schlessinger,J. and Bar-Sagi,D. (1993) Nature,363, 88-92
Green,E.L. (1981) in Foster,H.L., Smith,J.D. & Fox,J.G., (eds.), The Mouse in Biomedical Research Academic Press, New York, pp. 91-104,
Gribskov,M., Devereux,J. and Burgess,R.R. (1984) Nucleic Acid Res.,12, 53549
Harlow,E. & Lane,D. (1988) Antibodies, a laboratory manual, Cold Spring Harbor Laboratory Press, Cold Spring Harbor
Haslam,R.J., Koide,H.B. and Hemmings,B.A. (1993) Nature,363, 809-310 (25)
Hazan,R., Margolis,B., Dombalagian,M., Ullrich,A., Zilberstein,A. and Schlessinger,J. (1990) Cell Growth & Diff,1, 3-7
Hiles,I.D., Otsu,M., Volinia,S., Fry,M.J., Gout,I., Dhand,R., Panayotou,G., Ruiz-Larrea,F., Thompson,A., Totty,N.F., Hsuan,J.J., courtneidge,S.A., Parker,P.J., and Waterfleld,M.D. (1992) Cell,70, 419-429
Jones,P.F., Jakubowicz ,T., Pitossi,F.J., Maurer,F. and Hemmings ,B A. (1991) Pmc. NatL Acad. Sci. USA,88, 4171-4175
Kamps,M.P. and Sefton,B.M. (1988) Oncogene,2, 305-315
King,C.R., Borrello,I., Bellot,F., Comoglio,P. and Schlessinger,J. (1988) EMBO J,7, 1647-1651
Koch,W.J., Inglese,J., Stone,W.C. and Lefkowitz,R.J. (1993) J. Biol. Chem.,268, 8256
Kury,F.D., Schneeberger,C., Sliutz,G., Kubista,E., Salzer,H., Medl,M., Leodolter,S., Swoboda,H., Zeillinger,R. and Spona,J. (1990) Oncogene,5, 1403-1408
Lee,J., Dull,T.J., Lax,I., Schlessinger,J. and Ullrich,A. (1989) EMBO J,8, 167-173
Li,N., Batzer,A., Daly,R., Yajnik,V., Skolnik,E., Chardin,P., Bar-Sagi,D., Margolis,B. and Schlessinger,J. (1993) Nature,363, 85-88
Lowenstein,E.J., Daly,R.J., Batzer,A.G., Li,W., Margolis,B., Lammers,R., Ullrich,A., Skolnik,E .Y., Bar-Sagi,D. and Schlessinger,J. (1992) Cell,70, 431-442
Ma,Q., Alder,H., Nelson,K.K., chatterjee,D., Gu,Y., Nakamura,T., Canaani,E., Croce,C.M., Siracusa,L.D. and Buchberg,A.M. (1993) Proc. Nati. Acad. Sci. USA,90, 6350-6354
Manser,J. and Wood,W.B. (1990) Developniental Genetics,11, 49-64
Margolis,B., Rhee,S.G., Felder,S., Mervic,M., Lyall,R., Levitski,A., UIlrich,A., Zilberstein,A. and Schlessinger,J. (1989) Cell,57, 1101-1107 Margolis,B., Li,N., Koch,A., Mohammadi,M., Hurwitz,D., Zilberstein,A., UIlrich,A., Pawson,T. and Schlessinger,J. (1990a) EMBO J.,9, 4375-4380
Margolis,B., Zilberstein,A., Franks,C., Felder,S., Kremer,S., Ullrich,A., Rhee,S.G., Skorecki,K. and Schlessinger,J. (1990b) Science,248, 607-610 Margolis,B. (1992) Cell Growth & Differ. ,3, 73-80
Margolis,B., Silvennoinen,O., Comoglio,F., Roonprapunt,C., Skolnik,E., uIlrich,A. and Schlessinger,J. (1992) Proc. Natl. Acad. Sci. USA,89, 8894898
Marini,J.C., Nelson,K.K., Battey,J. and Siracusa,L.D. (1993) Genomics,15, 200-202
Mayer,B.J., Ren,R., Clark,K.L. and Baltimore,D. (1993) Ceil, 73, 629-630
Moran,M.F., Koch,C.A,, Anderson,D., Ellis,C., England,L., Martin,G.S. and Pawson,T. (1990) Proc. Natl. Acad. Sci. USA,87, 8622-8626
Musacchio,A., Gibson,T., Rice,P., Thompson, J. and Saraste,M. (1993) Trends Biochem. Sci.,18, 343-348
Olivier,J.P., Raabe,T., Henkemeyer,M., Dickson,B., Mbamalu,G., Margolis,B., Schlessinger,J., Hafen,E. and Pawson,T. (1993) Cell,73,17191
Pawson,T. and Schlessinger,J. (1993) Current Biology,2, 434-442
Pearson,W.R. and Lipman,D.J. (1988) Proc. Natl. Acad. Sci.,USA85, 2444-2448
Peles,E., Ben-Levy,R., Tzahar,E., Liu,N., Wen,D. and Yarden,Y. (1993) EMBO J,12, 961-971 (27)
Pelicci,G., Lanfrancone,L., Grignani,F., McGlade,J., Cavallo,F., Forni,G., Nicoletti,I., Pawson,T. and Pelicci,P.G. (1992) Cell,70, 93-104
Plowman,G.D., Green,J.M., Culouscou,J.-M., Carlton,G.W., Rothwell,V.M., and Buckley,S. (1993) Nature, 366,473-475.
Ren,R., Mayer,B.J., Cicchetti,P. and Baltimore,D. (1993) Science,259, 1157-1161
Rhee,S.G. and Choi,K.D. (1992) J. Biol. Chem.,267, 12393-12396
Ron,D. and Dressler,H. (1992) Biotechniques,13, 866-869
Rotin,D., Margolis,B., Mohammadi ,M., Daly,R.J., Daum,G., Li,N., Fischer,E.H., Burgess,W.H., Ullrich,A. and Schlessinger,J. (1992) EMBO J.,11, 559-567
Rozakis-Adcock,M., McGlade,J., Mbamalu,G., Pelicci,G., Daly,R., Li,W., Batzer,A., Thomas ,S., Brugge,J., Pelicci,P.G., Schlessinger,J. and Pawson,T. (1992) Nature,360, 689-692
Rozakis-Adcock,M., Fernley,R., Wade,J., Pawson,T. and Bowtell,D. (1993) Nature,363, 83-85
Sambrook,J., Fritsch,E.F. & Maniatis,T. (1989) Molecular Cloning. A Laboratory Manual. , Cold Spring Harbor Laboratory Press, Cold Spring Harbor
Sap,J., D'Eustachio,P., Givol,D. and Schlessinger,J. (1990) Proc. Natl. Acad. Sci. USA,87, 6112-6116
Schlessinger,J. and Ullrich,A. (1992) Neuron,9, 383-391
Schwartz,R.M. and Dayhoff,M.O. (1979) in Dayhoff, M.O., (ed.), Atlas of protetn sequences and structure (National Biomedical Research Foundation, Washington, D.C.pp. 353-358,
Segatto,O., Lonardo,F., Pierce,J.H., Bottaro,D.P. and Di Fiore,P.P. (1990) New Biologist,2, 187-195
Simon, M.A., Dodson,G.S. and Rubin,G.M. (1993) Cell,73, 169-177 Skolnik,E .Y., Margolis ,B., Mohammadi,M., Lowenstein,E., Fischer,R., Drepps,A., Ullrich,A. and Schlessinger,J. (1991) Cell,65, 83-90 Skolnik,E .Y., Lee ,C H., Batzer,A., Vicentini,L.M., Zhou,M., Daly,R., Myers,M.J.,Jr., Backer,J.M., Ullrich,A., White,M.F. and Schiessinger,J. (1993) EMBO J,12, 1929-1936 Slamon,D.J., Clark,G.M., Wong,S.G., Levin,W.J., Ullrich,A. and McGuire,W.L. (1987) Science,235, 177-182
Slamon,D .J., Godolphin,W., Jones,L.A., Holt,J.A., Wong,S.G., Keith,D .E., Levin,W.J., Stuart,S.G., Udove,J., Ullrich,A. and Press,M.F. (1989) Science,244, 707-712
Smith,K., Houlbrook,S., Greenall,M., Carmichael,J. and Harris,A.L.(1993) Oncogene,8, 933-938
Songyang,Z., Shoelson,S.E., Chaudhuri,M., Gish,G., Pawson,T., Haser,W.G., King,F., Roberts,T., Ratnofsky,S., Lechleider,R.J. Neel,B.G, Birge,R.B., Fajardo,J.E., Chou,M.M., Hanafusa,H. Schaffhausen,B., Cantley,L.C. (1993) Cell, 72, 767-778
Sulston,J., Du,Z., Thomas,K., Wilson,R., Hillier,L., Staden,R., Halloran,N., Green,P., Thierry-Mieg,J., Qiu,L., Dear,S., Coulson,A., Craxton,M., Durbin,R., Berks,M., Metzstein,M., Hawkins,T., Ainscough,R., and Waterston,R. (1992) Nature ,356, 37-41
Tandon,A.K., Clark,G.M., Chamness,G.C., Ullrich,A. and McGuire,W.L. (1989) J. Clin. Oncol.,7, 1120-1128
Tavassoli,M., Quirke,P., Farzaneh,F., Lock,N.J., Mayne,L.V. and Kirkham,N. (1989) British Journal of Cancer,60, 505-510
Trahey,M., Wong,G., Halenbeck,R., Rubinfield,B., Martin,G., Ladner,M., Long,C.M., Crosier,W.J., Watt,K., Koths,K. and McCormick,F. (1988) Science,242, 1697-1700
Tyers,M., Rachubinski,R.A., Stewart,M.I., Varrichio,A.M., Shorr,R.G.,Haslam,R.J. and Harley,C.B. (1988) Nature,333, 470-473
van de Vijver,M., van de Bersselaar,R., Devilee,P., Cornelisse,C., Peterse,J. and Nusse,R. (1987) Mol. Cell. Biol., 7, 2019-2023
Vogel ,U.S., Dixon,R.A.F., Schaber,M.D., Diehl,R.E, Marshall,M.S., Scolnik,E.M., Sigal,I.S and Gibbs,J.B. (1988) Nature,335, 90-93
Vogel,W., Lammers,R., Huang, J. and Ullrich,A. (1993) Science,259, 1611-1614
Wadsworth,W.G. and Hedgecock,E.M. (1992) Current Opinion in Neurobiology,2, 36-41

## Claims

1. Isolated, purified, or enriched peptide consisting of a BLM domain wherein the BLM domain consists of amino acids 95 to 231 of GRB-7 or of amino acids 95 to 428 of GRB-7 shown in Figure 2.

2. The peptide of claim 1, wherein said peptide is produced recombinantly.

3. The peptide of any of claims 1 to 2, wherein said peptide is purified.

4. A composition comprising a peptide according to any of claims 1 to 3 and a pharmaceutically acceptable carrier or diluent.

## Patentansprüche

1. Ein isoliertes, gereinigtes oder angereichertes Peptid bestehend aus einer BLM Domäne, wobei die BLM Domäne aus den Aminosäuren 95 bis 231 von GRB-7 oder aus den Aminosäuren 95 bis 428 von GRB-7, die in Figur 2 gezeigt sind, besteht.

2. Das Peptid nach Anspruch 1, wobei das Peptid rekombinant hergestellt wird.

3. Das Peptid nach einem der Ansprüche 1 bis 2, wobei das Peptid gereinigt ist.

4. Eine Zusammensetzung, die ein Peptid gemäß einem der Ansprüche 1 bis 3 und einen pharmazeutisch annehmbaren Trägerstoff oder ein pharmazeutisch annehmbares Verdünnungsmittel umfasst.

## Revendications

1. Peptide isolé, purifié ou enrichi, constitué d'un domaine BLM, dans lequel le domaine BLM est constitué des acides aminés 95 à 231 de GRB-7 ou des acides aminés 95 à 428 de GRB-7 présenté à la figure 2.

2. Peptide de la revendication 1, lequel dit peptide est produit par recombinaison.

3. Peptide de l'une quelconque des revendications 1 à 2, lequel dit peptide est purifié.

4. Composition comprenant un peptide selon l'une quelconque des revendications 1 à 3 et un véhicule ou un diluant pharmaceutiquement acceptable.


### SEQUENCE LISTING

(1) GENERAL INFORMATION:
   (i) APPLICANT: SUGEN, INC. 515 Galveston Drive Redwood City CA 94063-4720 UNITED STATES OF AMERICA
   (ii) TITLE OF INVENTION: METHODS FOR TREATMENT OR DIAGNOSIS OF DISEASES OR CONDITIONS ASSOCIATED WITH ABNORMAL SIGNAL TRANSDUCTION
   (iii) NUMBER OF SEQUENCES: 5
   (iv) CORRESPONDENCE ADDRESS:
      (A) ADDRESSEE: Lyon & Lyon
      (B) STREET: 633 West Fifth Street
      (C) CITY: Los Angeles
      (D) STATE: California
      (E) COUNTRY: USA
      (F) ZIP: 90071
   (v) COMPUTER READABLE FORM:
      (A) MEDIUM TYPE: 3.5" Diskette, 1.44 Mb
      (B) COMPUTER: IBM compatible
      (C) OPERATING SYSTEM: IBM P.C. DOS (Version 5.0)
      (D) SOFTWARE: WordPerfect (Version 5.1)
   (vi) CURRENT APPLICATION DATA:
      (A) APPLICATION NUMBER: PCT/US95/03452
      (B) FILING DATE: March 14, 1995
   (vii) PRIOR APPLICATION DATA:
      Prior applications total, including application described below: two

      (A) APPLICATION NUMBER: USSN 08/225,785
      (B) FILING DATE: June 8, 1994
      (A) APPLICATION NUMBER: USSN 08/212,234
      (B) FILING DATE: March 14, 1994
   (viii) ATTORNEY/AGENT INFORMATION:
      (A) NAME: Warburg, Richard J.
      (B) REGISTRATION NUMBER: 32,327
      (C) REFERENCE/DOCKET NUMBER: 211/011-PCT
   (ix) TELECOMMUNICATION INFORMATION:
      (A) TELEPHONE: (213) 489-1600
      (B) TELEFAX: (213) 955-0440
      (C) TELEX: 67-3510
(2) INFORMATION FOR SEQ ID NO: 1:
   (i) SEQUENCE CHARACTERISTICS:
      (A) LENGTH: 335
      (B) TYPE: amino acid
      (C) STRANDEDNESS: single
      (D) TOPOLOGY: linear
   (ii) SEQUENCE DESCRIPTION : SEQ ID NO: 1:
   Pro Arg Asp Ser Ser Arg Leu Cys Val Val Lys Val Tyr Ser Glu Asp
                   5                   10                  15
   Gly Ala Cys Arg Ser Val Glu Val Ala Ala Gly Ala Thr Ala Arg His
               20                  25                  30
   Val Cys Glu Met Leu Val Gln Arg Ala His Ala Leu Ser Asp Glu Ser
           35                  40                  45
   Trp Gly Leu Val Glu Ser His Pro Tyr Leu Ala Leu Glu Arg Gly Leu
       50                  55                  60
   Glu Asp His Glu Phe Val Val Glu Val Gln Glu Ala Trp Pro Val Gly
   65                  70                  75                  80
   Gly Asp Ser Arg Phe Ile Phe Arg Lys Asn Phe Ala Lys Tyr Glu Leu
                   85                  90                  95
   Phe Lys Ser Pro Pro His Thr Leu Phe Pro Glu Lys Met Val Ser Ser
               100                 105                 110
   Cys Leu Asp Ala Gln Thr Gly Ile Ser His Glu Asp Leu Ile Gln Asn
           115                 120                 125
   Phe Leu Asn Ala Gly Ser Phe Pro Glu Ile Gln Gly Phe Leu Gln Leu
       130                 135                 140
   Arg Gly Ser Gly Arg Gly Ser Gly Arg Lys Leu Trp Lys Arg Phe Phe
   145                 150                 155                 160
   Cys Phe Leu Arg Arg Ser Gly Leu Tyr Tyr Ser Thr Lys Gly Thr Ser
                   165                 170                 175
   Lys Asp Pro Arg His Leu Gln Tyr Val Ala Asp Val Asn Glu Ser Asn
               180                 185                 190
   Val Tyr Val Val Thr Gln Gly Arg Lys Leu Tyr Gly Met Pro Thr Asp
           195                 200                 205
   Phe Gly Phe Cys Val Lys Pro Asn Lys Leu Arg Asn Gly His Lys Gly
       210                 215                 220
   Leu His Ile Phe Cys Ser Glu Asp Glu Gln Ser Arg Thr Cys Trp Leu
   225                 230                 235                 240
   Ala Ala Phe Arg Leu Phe Lys Tyr Gly Val Gln Leu Tyr Lys Asn Tyr
                   245                 250                 255
   Gln Gln Ala Gln Ser Arg His Leu Arg Leu Ser Tyr Leu Gly Ser Pro
               260                 265                 270
   Pro Leu Arg Ser Val Ser Asp Asn Thr Leu Val Ala Met Asp Phe Ser
           275                 280                 285
   Gly His Ala Gly Arg Val Ile Asp Asn Pro Arg Glu Ala Leu Ser Ala
       290                 295                 300
   Ala Met Glu Glu Ala Gln Ala Trp Arg Lys Lys Thr Asn His Arg Leu
   305                 310                 315                 320
   Ser Leu Pro Thr Thr Cys Ser Gly Ser Ser Leu Ser Ala Ala Ile
                   325                 330                 335
(2) INFORMATION FOR SEQ ID NO: 2:
   (i) SEQUENCE CHARACTERISTICS:
      (A) LENGTH: 326
      (B) TYPE: amino acid
      (C) STRANDEDNESS: single
      (D) TOPOLOGY: linear
   (ii) SEQUENCE DESCRIPTION : SEQ ID NO: 2:
   Met Glu Lys Leu Arg Leu Arg Lys Asp Val Lys Val Phe Ser Glu Asp
                   5                   10                  15
   Gly Thr Ser Lys Val Val Glu Ile Leu Thr Asp Met Thr Ala Arg Asp
               20                  25                  30
   Leu Cys Gln Leu Leu Val Tyr Lys Ser His Cys Val Asp Asp Asn Ser
           35                  40                  45
   Trp Thr Leu Val Glu His His Pro Gln Leu Gly Leu Glu Arg Cys Leu
       50                  55                  60
   Glu Asp His Glu Ile Val Val Gln Val Glu Ser Thr Met Pro Ser Glu
   65                  70                  75                  80
   Ser Lys Phe Leu Phe Arg Lys Asn Tyr Ala Lys Tyr Glu Phe Phe Lys
                   85                  90                  95
   Asn Pro Val Asn Phe Phe Pro Asp Gln Met Val Asn Trp Cys Gln Gln
               100                 105                 110
   Ser Asn Gly Gly Gln Ala Pro Val Leu Gln Asn Phe Leu Asn Thr Ser
         115                 120                 125
   Ser Cys Pro Glu Ile Gln Gly Phe Leu Gln Val Lys Glu Val Gly Arg
       130                 135                 140
   Lys Ser Trp Lys Lys Leu Tyr Val Cys Leu Arg Arg Ser Gly Leu Tyr
   145                 150                 155                 160
   Tyr Ser Thr Lys Gly Thr Ser Lys Glu Pro Arg His Leu Gln Leu Leu
                   165                 170                 175
   Ala Asp Leu Glu Glu Ser Ser Ile Phe Tyr Leu Ile Ala Gly Lys Lys
               180                 185                 190
   Gln Tyr Asn Ala Pro Asn Glu His Gly Met Cys Ile Lys Pro Asn Lys
           195                 200                 205
   Ala Lys Thr Glu Met Lys Glu Leu Arg Leu Leu Cys Ala Glu Asp Glu
       210                 215                 220
   Gln Ile Arg Thr Cys Trp Met Thr Ala Phe Arg Leu Leu Lys Tyr Gly
   225                 230                 235                 240
   Met Leu Leu Tyr Gln Asn Tyr Arg Ile Pro Gln Arg Lys Gly Leu Pro
                   245                 250                 255
   Pro Pro Phe Asn Ala Pro Met Arg Ser Val Ser Glu Asn Ser Leu Val
               260                 265                 270
   Ala Met Asp Phe Ser Gly Gln Ile Gly Arg Val Ile Asp Asn Pro Ala
           275                 280                 285
   Glu Ala Gln Ser Ala Ala Leu Glu Glu Gly His Ala Trp Arg Asn Gly
       290                 295                 300
   Ser Thr Arg Met Asn Ile Leu Ser Ser Gln Ser Pro Leu His Pro Ser
   305                 310                 315                 320
   Thr Leu Asn Ala Val Ile
                   325
(2) INFORMATION FOR SEQ ID NO: 3:
   (i) SEQUENCE CHARACTERISTICS:
      (A) LENGTH: 348
      (B) TYPE: amino acid
      (C) STRANDEDNESS: single
      (D) TOPOLOGY: linear
   (ii) SEQUENCE DESCRIPTION : SEQ ID NO: 3:
   Lys Glu Ala Lys Val Thr Lys Ile Phe Val Lys Phe Phe Val Glu Asp
                   5                   10                  15
   Gly Glu Ala Leu Gln Leu Leu Ile Asp Glu Arg Trp Thr Val Ala Asp
   20 25 30
   Thr Leu Lys Gln Leu Ala Glu Lys Asn His Ile Ala Leu Met Glu Asp
   35 40 45
   His Cys Ile Val Glu Glu Tyr Pro Glu Leu Tyr Ile Lys Arg Val Tyr
   50 55 60
   Glu Asp His Glu Lys Val Val Glu Asn Ile Gln Met Trp Val Gln Asp
   65 70 75 80
   Ser Pro Asn Lys Leu Tyr Phe Met Arg Arg Pro Asp Lys Tyr Ala Phe
   85 90 95
   Ile Ser Arg Pro Glu Leu Tyr Leu Leu Thr Pro Lys Thr Ser Asp His
   100 105 110
   Met Glu Ile Pro Ser Gly Asp Gln Trp Thr Ile Asp Val Lys Gln Lys
   115 120 125
   Phe Val Ser Glu Tyr Phe His Arg Glu Pro Val Val Pro Pro Glu Met
   130 135 140
   Glu Gly Phe Leu Tyr Leu Lys Ser Asp Gly Arg Lys Ser Trp Lys Lys
   145 150 155 160
   His Tyr Phe Val Leu Arg Pro Ser Gly Leu Tyr Tyr Ala Pro Lys Ser
   165 170 175
   Lys Lys Pro Thr Thr Lys Asp Leu Thr Cys Leu Met Asn Leu His Ser
   180 185 190
   Asn Gln Val Tyr Thr Gly Ile Gly Trp Glu Lys Lys Tyr Lys Ser Pro
   195 200 205
   Thr Pro Trp Cys Ile Ser Ile Lys Leu Thr Ala Leu Gln Met Lys Arg
   210 215 220
   Ser Gln Phe Ile Lys Tyr Ile Cys Ala Glu Asp Glu Met Thr Phe Lys
   225 230 235 240
   Lys Trp Leu Val Ala Leu Arg Ile Ala Lys Asn Gly Ala Glu Leu Leu
   245 250 255
   Glu Asn Tyr Glu Arg Ala Cys Gln Ile Arg Arg Glu Thr Leu Gly Pro
   260 265 270
   Ala Ser Ser Met Ser Ala Ala Ser Ser Ser Thr Ala Ile Ser Glu Val
   275 280 285
   Pro His Ser Leu Ser His His Gln Arg Thr Pro Ser Val Ala Ser Ser
   290 295 300
   Ile Gln Leu Ser Ser His Met Met Asn Asn Pro Thr His Pro Leu Ser
   305 310 315 320
   Val Asn Val Arg Asn Gln Ser Pro Ala Ser Phe Ser Val Asn Ser Cys
               325                 330                 335
   Gln Gln Ser His Pro Ser Arg Thr Ser Ala Lys Leu
               340                 345
(2) INFORMATION FOR SEQ ID NO: 4:
   (i) SEQUENCE CHARACTERISTICS:
      (A) LENGTH: 534
      (B) TYPE: amino acid
      (C) STRANDEDNESS: single
      (D) TOPOLOGY: linear
   (ii) SEQUENCE DESCRIPTION : SEQ ID NO: 4:
   Glu Leu Asp Leu Ser Pro Thr His Leu Ser Ser Ser Pro Glu Asp Val
                     5                  10                  15
   Cys Pro Thr Pro Ala Thr Pro Pro Glu Thr Pro Pro Pro Pro Asp Asn
                20                  25                  30
   Pro Pro Pro Gly Asp Val Lys Arg Ser Gln Pro Leu Pro Ile Pro Ser
            35                  40                  45
   Ser Arg Lys Leu Arg Glu Glu Glu Phe Gln Ala Thr Ser Leu Pro Ser
        50                  55                  60
   Ile Pro Asn Pro Phe Pro Glu Leu Cys Ser Pro Pro Ser Gln Lys Pro
    65                  70                  75                  80
   Ile Leu Gly Gly Ser Ser Gly Ala Arg Gly Leu Leu Pro Arg Asp Ser
                    85                  90                  95
   Ser Arg Leu Cys Val Val Lys Val Tyr Ser Glu Asp Gly Ala Cys Arg
               100                 105                 110
   Ser Val Glu Val Ala Ala Gly Ala Thr Ala Arg His Val Cys Glu Met
           115                 120                 125
   Leu Val Gln Arg Ala His Ala Leu Ser Asp Glu Ser Trp Gly Leu Val
       130                 135                 140
   Glu Ser His Pro Tyr Leu Ala Leu Glu Arg Gly Leu Glu Asp His Glu
   145                 150                 155                 160
   Phe Val Val Glu Val Gln Glu Ala Trp Pro Val Gly Gly Asp Ser Arg
                   165                 170                 175
   Phe Ile Phe Arg Lys Asn Phe Ala Lys Tyr Glu Leu Phe Lys Ser Pro
               180                 185                 190
   Pro His Thr Leu Phe Pro Glu Lys Met Val Ser Ser Cys Leu Asp Ala
           195                 200                 205
   Gln Thr Gly Ile Ser His Glu Asp Leu Ile Gln Asn Phe Leu Asn Ala
       210                 215                 220
   Gly Ser Phe Pro Glu Ile Gln Gly Phe Leu Gln Leu Arg Gly Ser Gly
   225                 230                 235                 240
   Arg Gly Ser Gly Arg Lys Leu Trp Lys Arg Phe Phe Cys Phe Leu Arg
                   245                 250                 255
   Arg Ser Gly Leu Tyr Tyr Ser Thr Lys Gly Thr Ser Lys Asp Pro Arg
               260                 265                 270
   His Leu Gln Tyr Val Ala Asp Val Asn Glu Ser Asn Val Tyr Val Val
           275                 280                 285
   Thr Gln Gly Arg Lys Leu Tyr Gly Met Pro Thr Asp Phe Gly Phe Cys
       290                 295                 300
   Val Lys Pro Asn Lys Leu Arg Asn Gly His Lys Gly Leu His Ile Phe
   305                 310                 315                 320
   Cys Ser Glu Asp Glu Gln Ser Arg Thr Cys Trp Leu Ala Ala Phe Arg
                   325                 330                 335
   Leu Phe Lys Tyr Gly Val Gln Leu Tyr Lys Asn Tyr Gln Gln Ala Gln
               340                 345                 350
   Ser Arg His Leu Arg Leu Ser Tyr Leu Gly Ser Pro Pro Leu Arg Ser
           355                 360                 365
   Val Ser Asp Asn Thr Leu Val Ala Met Asp Phe Ser Gly His Ala Gly
       370                 375                 380
   Arg Val Ile Asp Asn Pro Arg Glu Ala Leu Ser Ala Ala Met Glu Glu
   385                 390                 395                 400
   Ala Gln Ala Trp Arg Lys Lys Thr Asn His Arg Leu Ser Leu Pro Thr
                   405                 410                 415
   Thr Cys Ser Gly Ser Ser Leu Ser Ala Ala Ile His Arg Thr Gln Pro
               420                 425                 430
   Trp Phe His Gly Arg Ile Ser Arg Glu Glu Ser Gln Arg Leu Ile Gly
           435                 440                 445
   Gln Gln Gly Leu Val Asp Gly Val Phe Leu Val Arg Glu Ser Gln Arg
       450                 455                 460
   Asn Pro Gln Gly Phe Val Leu Ser Leu Cys His Leu Gln Lys Val Lys
   465                 470                 475                 480
   His Tyr Leu Ile Leu Pro Ser Glu Asp Glu Gly Cys Leu Tyr Phe Ser
                   485                 490                 495
   Met Asp Glu Gly Gln Thr Arg Phe Thr Asp Leu Leu Gln Leu Val Glu
               500                 505                 510
   Phe His Gln Leu Asn Arg Gly Ile Leu Pro Cys Leu Leu Arg His Cys
             515                 520                 525
   Cys Ala Arg Val Ala Leu
        530
(2) INFORMATION FOR SEQ ID NO: 5:
   (i) SEQUENCE CHARACTERISTICS:
      (A) LENGTH: 618
      (B) TYPE: amino acid
      (C) STRANDEDNESS: single
      (D) TOPOLOGY: linear
   (ii) SEQUENCE DESCRIPTION : SEQ ID NO: 5:
   Asp Ile Asn Ser Ser Val Glu Ser Leu Asn Ser Ala Cys Asn Met Gln
                   5                  10                  15
   Ser Asp Thr Asp Thr Ala Pro Leu Leu Glu Asp Gly Gln His Ala Ser
               20                  25                  30
   Asn Gln Gly Ala Ala Ser Ser Ser Arg Gly Gln Pro Gln Ala Ser Pro
           35                  40                  45
   Arg Gln Lys Met Gln Arg Ser Gln Pro Val His Ile Leu Arg Arg Leu
       50                  55                  60
   Gln Glu Glu Asp Gln Gln Leu Arg Thr Ala Ser Leu Pro Ala Ile Pro
    65                  70                  75                  80
   Asn Pro Phe Pro Glu Leu Thr Gly Ala Ala Pro Gly Ser Pro Pro Ser
                   85                  90                  95
   Val Ala Pro Ser Ser Leu Pro Pro Pro Pro Ser Gln Pro Pro Ala Lys
               100                 105                 110
   His Cys Gly Arg Cys Glu Lys Trp Ile Pro Gly Glu Asn Thr Arg Gly
           115                 120                 125
   Asn Gly Lys Arg Lys Ile Trp Arg Trp Gln Phe Pro Pro Gly Phe Gln
       130                 135                 140
   Leu Ser Lys Leu Thr Arg Pro Gly Leu Trp Thr Lys Thr Thr Ala Arg
   145                 150                 155                 160
   Phe Ser Lys Lys Gln Pro Lys Asn Gln Cys Pro Thr Asp Thr Val Asn
                   165                 170                 175
   Pro Val Ala Arg Met Pro Thr Ser Gln Met Glu Lys Leu Arg Leu Arg
               180                 185                 190
   Lys Asp Val Lys Val Phe Ser Glu Asp Gly Thr Ser Lys Val Val Glu
           195                 200                 205
   Ile Leu Thr Asp Met Thr Ala Arg Asp Leu Cys Gln Leu Leu Val Tyr
       210                 215                 220
   Lys Ser His Cys Val Asp Asp Asn Ser Trp Thr Leu Val Glu His His
   225                 230                 235                 240
   Pro Gln Leu Gly Leu Glu Arg Cys Leu Glu Asp His Glu Ile Val Val
                   245                 250                 255
   Gln Val Glu Ser Thr Met Pro Ser Glu Ser Lys Phe Leu Phe Arg Lys
               260                 265                 270
   Asn Tyr Ala Lys Tyr Glu Phe Phe Lys Asn Pro Val Asn Phe Phe Pro
           275                 280                 285
   Asp Gln Met Val Asn Trp Cys Gln Gln Ser Asn Gly Gly Gln Ala Gln
       290                 295                 300
   Leu Leu Gln Asn Phe Leu Asn Thr Ser Ser Cys Pro Glu Ile Gln Gly
   305                 310                 315                 320
   Phe Leu Gln Val Lys Glu Val Gly Arg Lys Ser Trp Lys Lys Leu Tyr
                   325                 330                 335
   Val Cys Leu Arg Arg Ser Gly Leu Tyr Tyr Ser Thr Lys Gly Thr Ser
               340                 345                 350
   Lys Glu Pro Arg His Leu Gln Leu Leu Ala Asp Leu Glu Glu Ser Ser
           355                 360                 365
   Ile Phe Tyr Leu Ile Ala Gly Lys Lys Gln Tyr Asn Ala Pro Asn Glu
       370                 375                 380
   His Gly Met Cys Ile Lys Pro Asn Lys Ala Lys Thr Glu Met Lys Glu
   385                 390                 395                 400
   Leu Arg Leu Leu Cys Ala Glu Asp Glu Gln Ile Arg Thr Cys Trp Met
                   405                 410                 415
   Thr Ala Phe Arg Leu Leu Lys Tyr Gly Met Leu Leu Tyr Gln Asn Tyr
               420                 425                 430
   Arg Ile Pro Gln Arg Lys Gly Leu Pro Pro Pro Phe Asn Ala Pro Met
           435                 440                 445
   Arg Ser Val Ser Glu Asn Ser Leu Val Ala Met Asp Phe Ser Gly Gln
       450                 455                 460
   Ile Gly Arg Val Ile Asp Asn Pro Ala Glu Ala Gln Ser Ala Ala Leu
   465                 470                 475                 480
   Glu Glu Gly His Ala Trp Arg Asn Gly Ser Thr Arg Met Asn Ile Leu
                   485                 490                 495
   Ser Ser Gln Ser Pro Leu His Pro Ser Thr Leu Asn Ala Val Ile His
               500                 505                 510
   Arg Thr Gln His Trp Phe His Gly Arg Ile Ser Arg Glu Glu Ser His
           515                 520                 525
   Arg Ile Ile Lys Gln Gln Gly Leu Val Asp Gly Leu Phe Leu Leu Arg
       530                 535                 540
   Asp Ser Gln Ser Asn Pro Lys Ala Phe Val Leu Thr Leu Cys His His
   545                 550                 555                 560
   Gln Lys Ile Lys Asn Phe Gln Ile Leu Pro Cys Glu Asp Asp Gly Gln
                   565                 570                 575
   Thr Phe Phe Thr Leu Asp Asp Gly Asn Thr Lys Phe Ser Asp Leu Ile
               580                 585                 590
   Gln Leu Val Asp Phe Tyr Gln Leu Asn Lys Gly Val Leu Pro Cys Lys
           595                 600                 605
   Leu Lys His His Cys Ile Arg Val Ala Leu
       610                 615